(19) Europäisches Patentamt
European Patent Office
Office européen des brevets

(11) EP 4 574 818 A1

(12) EUROPEAN PATENT APPLICATION
published in accordance with Art. 153(4) EPC

(43) Date of publication:
**25.06.2025 Bulletin 2025/26**

(21) Application number: **23854894.5**

(22) Date of filing: **15.08.2023**

(51) International Patent Classification (IPC):
***C07D 401/12*** *(2006.01)* ***A01N 43/40*** *(2006.01)*
***A01N 43/50*** *(2006.01)* ***A01N 43/653*** *(2006.01)*
***A01N 47/02*** *(2006.01)* ***A01P 7/02*** *(2006.01)*
***A01P 7/04*** *(2006.01)* ***A61K 31/4439*** *(2006.01)*
***A61K 31/444*** *(2006.01)* ***A61K 31/695*** *(2006.01)*
***A61P 33/00*** *(2006.01)* ***C07F 7/12*** *(2006.01)*

(52) Cooperative Patent Classification (CPC):
**A01N 43/40; A01N 43/50; A01N 43/653; A01N 47/02; A01P 7/02; A01P 7/04; A61K 31/4439; A61K 31/444; A61K 31/695; A61P 33/00; C07D 401/12; C07F 7/12**

(86) International application number:
**PCT/JP2023/029530**

(87) International publication number:
**WO 2024/038860 (22.02.2024 Gazette 2024/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.08.2022 JP 2022131222**

(71) Applicant: **Nippon Soda Co., Ltd.**
**Tokyo 100-7010 (JP)**

(72) Inventors:
- **IZUMITANI, Maho**
  **Odawara-shi, Kanagawa 250-0280 (JP)**
- **SUZUKI, Hiroto**
  **Odawara-shi, Kanagawa 250-0280 (JP)**
- **KOBAYASHI, Hiroyuki**
  **Odawara-shi, Kanagawa 250-0280 (JP)**
- **SHIBAYAMA, Kotaro**
  **Odawara-shi, Kanagawa 250-0280 (JP)**
- **KIM, JaeHyun**
  **Odawara-shi, Kanagawa 250-0280 (JP)**
- **KAWAGUCHI, Masahiro**
  **Odawara-shi, Kanagawa 250-0280 (JP)**

(74) Representative: **Sonnenhauser, Thomas Martin**
**Wuesthoff & Wuesthoff**
**Patentanwälte und Rechtsanwalt PartG mbB**
**Schweigerstraße 2**
**81541 München (DE)**

# (54) ONIUM SALT OF NITROGEN-CONTAINING HETEROARYL COMPOUND AND PEST CONTROL AGENT

(57) An onium salt of a nitrogen-containing (hetero) aryl compound according to the present invention is represented by formula (I) (in the formula, $Q^1$ represents a pyrazole ring or the like, $G^1$ represents a nitrogen or carbon atom, $X^1$ represents a halogeno group or the like, m indicates the number of $X^1$, A represents an oxygen or sulfur atom, $Q^2$ represents a substituted or unsubstituted phenyl group, $X^2$ represents a halogeno group or the like, $X^3$ represents a halogeno group or the like, n represents an integer of 0-2, $Z^{q-}$ represents a counter ion, and q represents 1 or 2).

EP 4 574 818 A1

[Chemical Formula 1]

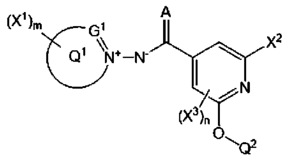

(I)

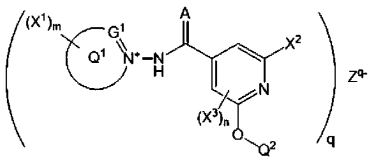

(II)

**Description**

TECHNICAL FIELD

The present invention relates to an onium salt of a nitrogen-containing heteroaryl compound and a pest control agent. In more detail, the present invention relates to an onium salt of a nitrogen-containing heteroaryl compound that has excellent insecticidal activity and/or acaricidal activity, and excellent safety, and that can be synthesized industrially advantageously, as well as a pest control agent containing the same as an active ingredient thereof.

Priority is claimed on Japanese Patent Application No. 2022-131222, filed August 19, 2022, the content of which is incorporated herein by reference.

BACKGROUND ART

Various compounds having insecticidal and/or acaricidal activity have been proposed. In the case where such compounds are used as agrochemicals, the compounds are required not only to exhibit high efficacy, but also to exhibit less possibility of causing chemical resistance, harmful effects on plants, or soil pollution, and to exhibit low toxicity to domestic animals or fish.

Patent Document 1 discloses an imidazolium salt of formula (A) which exhibits pest control effects.

[Chemical Formula 1]

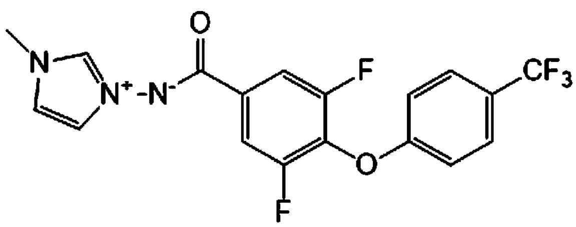

(A)

Citation List

Patent Document

Patent Document 1: International Patent Application, Publication No. WO 2021/049522

SUMMARY OF INVENTION

Technical Problem

An object of the present invention is to provide an onium salt of a nitrogen-containing heteroaryl compound (hereinafter, may be abbreviated as "aromacyclic quaternary ammonium compound") that has excellent pest control activity, particularly insecticidal activity and/or acaricidal activity, and excellent safety, and that can be synthesized industrially advantageously. Another object of the present invention is to provide a pest control agent, insecticide, acaricide, ectoparasite control agent, endoparasite control agent, or expellant, containing the aromacyclic quaternary ammonium compound as an active ingredient thereof.

Solution to Problem

The present invention encompassing the following aspects has been completed as a result of studying to solve the above-described problems.

(1) A compound of formula (I) or formula (II).

[Chemical Formula 2]

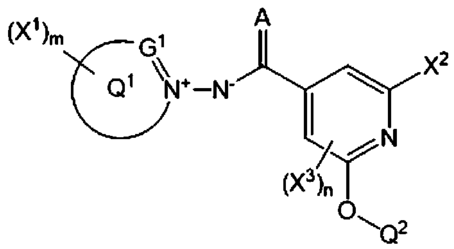

(I)

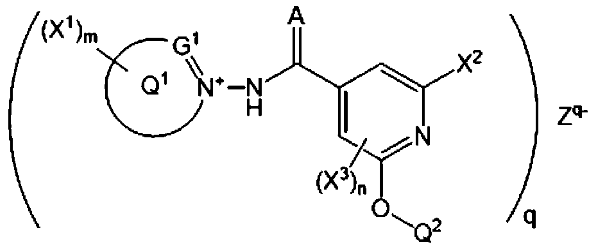

(II)

(In the formulae (I) and (II),

$Q^1$ is a pyrazole ring, an imidazole ring, a 1,2,4-triazole ring, a pyridine ring, a pyrazine ring, a pyrimidine ring, or a pyridazine ring,
$G^1$ is a nitrogen atom or a carbon atom,
$X^1$ is a halogeno group, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C1-6 alkylcarbonyl group, a substituted or unsubstituted C6-10 aryl group, a substituted or unsubstituted 5- or 6-membered heteroaryl group, a pentafluorosulfanyl group, a nitro group, or a cyano group,
m indicates the number of $X^1$, and m is an integer of 1 to 3 when $Q^1$ is a 1,2,4-triazole ring, is an integer of 1 to 4 when $Q^1$ is a pyrazole ring or an imidazole ring, is an integer of 0 to 4 when $Q^1$ is a pyrazine ring, a pyrimidine ring or a pyridazine ring, or is an integer of 0 to 5 when $Q^1$ is a pyridine ring,
A is an oxygen atom or a sulfur atom,
$Q^2$ is a substituted or unsubstituted phenyl group, a substituted or unsubstituted 5- or 6-membered heteroaryl group, or a substituted or unsubstituted 9- or 10-membered heteroaryl group,
$X^2$ is a halogeno group, a substituted or unsubstituted C1-6 alkyl group, a substituted or unsubstituted C2-6 alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C3-8 cycloalkyl group, a substituted or unsubstituted C6-10 aryl group, a substituted or unsubstituted C6-10 aryloxy group, a substituted or unsubstituted pyrazolyl group, an aldehyde group, a cyano group, or $-C=N-OCH_3$,
$X^3$ is a halogeno group, a substituted or unsubstituted C1-6 alkyl group, a substituted or unsubstituted C1-6 alkoxy group, or a cyano group,
n indicates the number of $X^3$, and is an integer of 0 to 2,
$Z^{q-}$ is a counter ion, and
q indicates a valence of the counter ion and is 1 or 2.)

(2) A pest control agent containing at least one selected from the compounds of (1) as an active ingredient thereof.
(3) An insecticide or acaricide containing at least one selected from the compounds of (1) as an active ingredient thereof.
(4) An ectoparasite control agent containing at least one selected from the compounds of (1) as an active ingredient thereof.
(5) An endoparasite control agent or expellant containing at least one selected from the compounds of (1) as an active ingredient thereof.

Advantageous Effects of Invention

The aromacyclic quaternary ammonium compound of the present invention has control activity against pests that cause problems to agricultural crops or in the health field. The control agent containing the aromacyclic quaternary ammonium compound of the present invention can effectively control pests, particularly agricultural insect pests and acarians, at lower doses, and can further effectively control ectoparasites and endoparasites that may harm humans and animals.

DESCRIPTION OF EMBODIMENTS

An aromacyclic quaternary ammonium compound of the present invention is a compound of formula (I) (inner salt) or a compound of formula (II) (intermolecular salt). The inner salt is a compound having both a cation center and an anion center in one molecule that is a zwitterion. The intermolecular salt is a compound formed by ionic association between a cation and anion that form an ionic pair.

[Chemical Formula 3]

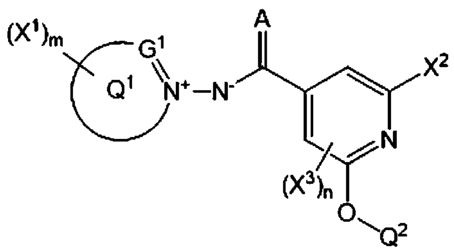

(I)

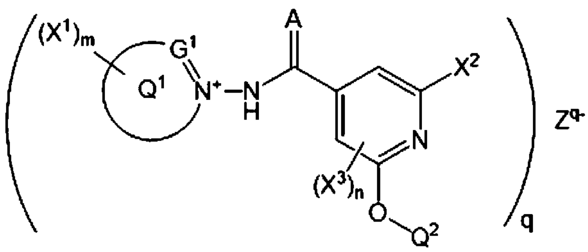

(II)

In the present specification, the term "unsubstituted" refers to a group consisting of a mother nucleus. In the case where only the name of a group serving as a mother nucleus is provided without accompanying the term "substituted", this refers to "unsubstituted" unless specifically indicated otherwise.
On the other hand, the term "substituted" means that any hydrogen atom of a group serving as a mother nucleus is substituted with a group (substituent) having a structure that is identical to or different from the mother nucleus. Thus, a "substituent" is another group bound to a group serving as the mother nucleus. The number of substituents may be one or two or more. Two or more substituents may be identical to or different from each other.
The term "C1-6", for example, indicates that the number of carbon atoms of the group serving as the mother nucleus is 1 to 6. The number of carbon atoms does not include the number of carbon atoms present in substituents. For example, a butyl group having an ethoxy group as a substituent thereof is classified as a C2 alkoxy C4 alkyl group.
There are no particular limitations on "substituents" provided that they are chemically available and achieve the effects of the present invention. Specific examples of groups that can be "substituents" include the following groups:

C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group;
C2-6 alkenyl groups such as a vinyl group, a 1-propenyl group, a 2-propenyl group (an allyl group), a 1-butenyl group, a 2-butenyl group, a 3-butenyl group, a 1-methyl-2-propenyl group, and a 2-methyl-2-propenyl group;
C2-6 alkynyl groups such as an ethynyl group, a 1-propynyl group, a 2-propynyl group, a 1-butynyl group, a 2-butynyl group, a 3-butynyl group, and a 1-methyl-2-propynyl group;
C3-8 cycloalkyl groups such as a cyclopropyl group, a cyclobutyl group, a cyclopentyl group, a cyclohexyl group, and a cubanyl group;
C6-10 aryl groups such as a phenyl group and a naphthyl group;

C6-10 aryl C1-6 alkyl groups such as a benzyl group and a phenethyl group;
3- to 6-membered heterocyclyl groups;
3- to 6-membered heterocyclyl C1-6 alkyl groups;
a hydroxy group;
C1-6 alkoxy groups such as a methoxy group, an ethoxy group, a n-propoxy group, an i-propoxy group, a n-butoxy group, a s-butoxy group, an i-butoxy group, and a t-butoxy group;
C2-6 alkenyloxy groups such as a vinyloxy group, an allyloxy group, a propenyloxy group, and a butenyloxy group;
C2-6 alkynyloxy groups such as an ethynyloxy group, and a propargyloxy group;
C6-10 aryloxy groups such as a phenoxy group, and a naphthoxy group;
C6-10 aryl C1-6 alkoxy groups such as a benzyloxy group, and a phenethyloxy group;
5- or 6-membered heteroaryloxy groups such as a thiazolyloxy group, and a pyridyloxy group;
5- or 6-membered heteroaryl C1-6 alkyloxy groups such as a thiazolylmethyloxy group, and a pyridylmethyloxy group;
a formyl group;
C1-6 alkylcarbonyl groups such as an acetyl group, and a propionyl group;
a formyloxy group;
C1-6 alkylcarbonyloxy groups such as an acetyloxy group, and a propionyloxy group;
C6-10 arylcarbonyl groups such as a benzoyl group;
C1-6 alkoxycarbonyl groups such as a methoxycarbonyl group, an ethoxycarbonyl group, a n-propoxycarbonyl group, an i-propoxycarbonyl group, a **n-**butoxycarbonyl group, and a t-butoxycarbonyl group;
C1-6 alkoxycarbonyloxy groups such as a methoxycarbonyloxy group, an ethoxycarbonyloxy group, a n-propoxycarbonyloxy group, an i-propoxycarbonyloxy group, a n-butoxycarbonyloxy group, and a t-butoxycarbonyloxy group;
a carboxyl group;
halogeno groups such as a fluoro group, a chloro group, a bromo group, and an iodo group;
C1-6 haloalkyl groups such as a chloromethyl group, a chloroethyl group, a trifluoromethyl group, a 1,2-dichloro-n-propyl group, and a 1-fluoro-n-butyl group;
C2-6 haloalkenyl groups such as a 2-chloro-1-propenyl group, and a 2-fluoro-1-butenyl group;
C2-6 haloalkynyl groups such as a 4,4-dichloro-1-butynyl group, a 4-fluoro-1-pentynyl group, and a 5-bromo-2-pentynyl group;
C1-6 haloalkoxy groups such as a trifluoromethoxy group, a 2-chloro-n-propoxy group, and a 2,3-dichlorobutoxy group;
C2-6 haloalkenyloxy groups such as a 2-chloropropenyloxy group, and a 3-bromobutenyloxy group;
C1-6 haloalkylcarbonyl groups such as a chloroacetyl group, a trifluoroacetyl group, and a trichloroacetyl group;
an amino group;
C1-6 alkyl-substituted amino groups such as a methylamino group, a dimethylamino group, and a diethylamino group;
C6-10 arylamino groups such as an anilino group, and a naphthylamino group;
C6-10 aryl C1-6 alkylamino groups such as a benzylamino group, and a phenethylamino group;
a formylamino group;
C1-6 alkylcarbonylamino groups such as an acetylamino group, a propanoylamino group, a butyrylamino group, and an i-propylcarbonylamino group;
C1-6 alkoxycarbonylamino groups such as a methoxycarbonylamino group, an ethoxycarbonylamino group, a n-propoxycarbonylamino group, and an i-propoxycarbonylamino group;
unsubstituted or substituted aminocarbonyl groups such as a carbamoyl group, a dimethylaminocarbonyl group, a phenylaminocarbonyl group, and a N-phenyl-N-methylaminocarbonyl group;
imino C1-6 alkyl groups such as an iminomethyl group, a (1-imino)ethyl group, and a (1-imino)-n-propyl group;
substituted or unsubstituted N-hydroxyimino C1-6 alkyl groups such as a N-hydroxy-iminomethyl group, a (1-(N-hydroxy)-imino)ethyl group, a (1-(N-hydroxy)-imino)propyl group, a N-methoxy-iminomethyl group, and a (1-(N-methoxy)-imino)ethyl group;
an aminocarbonyloxy group;
C1-6 alkyl-substituted aminocarbonyloxy groups such as an ethylaminocarbonyloxy group, and a dimethylaminocarbonyloxy group;
a mercapto group;
C1-6 alkylthio groups such as a methylthio group, an ethylthio group, a n-propylthio group, an i-propylthio group, a n-butylthio group, an i-butylthio group, a s-butylthio group, and a t-butylthio group;
C1-6 haloalkylthio groups such as a trifluoromethylthio group, and a 2,2,2-trifluoroethylthio group;
C6-10 arylthio groups such as a phenylthio group, and a naphthylthio group;
5- or 6-membered heteroarylthio groups such as a thiazolylthio group, and a pyridylthio group;
C1-6 alkylsulfinyl groups such as a methylsulfinyl group, an ethylsulfinyl group, and a t-butylsulfinyl group;

C1-6 haloalkylsulfinyl groups such as a trifluoromethylsulfinyl group, and a 2,2,2-trifluoroethylsulfinyl group;
C6-10 arylsulfinyl groups such as a phenylsulfinyl group;
5- or 6-membered heteroarylsulfinyl groups such as a thiazolylsulfinyl group, and a pyridylsulfinyl group;
C1-6 alkylsulfonyl groups such as a methylsulfonyl group, an ethylsulfonyl group, and a t-butylsulfonyl group;
C1-6 haloalkylsulfonyl groups such as a trifluoromethylsulfonyl group, and a 2,2,2-trifluoroethylsulfonyl group;
C6-10 arylsulfonyl groups such as a phenylsulfonyl group;
5- or 6-membered heteroarylsulfonyl groups such as a thiazolylsulfonyl group, and a pyridylsulfonyl group;
C1-6 alkylsulfonyloxy groups such as a methylsulfonyloxy group, an ethylsulfonyloxy group, and a t-butylsulfonyloxy group;
C1-6 haloalkylsulfonyloxy groups such as a trifluoromethylsulfonyloxy group, and a 2,2,2-trifluoroethylsulfonyloxy group;
tri C1-6 alkyl-substituted silyl groups such as a trimethylsilyl group, a triethylsilyl group, and a t-butyldimethylsilyl group;
tri C6-10 aryl-substituted silyl groups such as a triphenylsilyl group;
a pentafluorosulfanyl group;
a cyano group; and a nitro group.

Any hydrogen atom of the "substituent" may be substituted with a group having a different structure. Examples of such a substituent include C1-6 alkyl groups, C1-6 haloalkyl groups, C1-6 alkoxy groups, C1-6 haloalkoxy groups, halogeno groups, a cyano group, and C1-6 alkyl-substituted silyl groups.

The "3- to 6-membered heterocyclyl group" is a 3-membered ring, a 4-membered ring, a 5-membered ring or a 6-membered ring which contains, as a ring member atom, one to four hetero atoms selected from the group consisting of a nitrogen atom, an oxygen atom and a sulfur atom. The heterocyclyl group may be monocyclic or polycyclic. If at least one ring of a polycyclic heterocyclyl group is a hetero ring, the remaining rings thereof may be any of saturated alicyclic rings, unsaturated alicyclic rings and aromatic rings. Examples of the "3- to 6-membered heterocyclyl group" include 3- to 6-membered saturated heterocyclyl groups, 5- or 6-membered heteroaryl groups, and 5- or 6-membered partially unsaturated heterocyclyl groups.

Examples of the 3- to 6-membered saturated heterocyclyl groups include an aziridinyl group, an epoxy group, a pyrrolidinyl group, a tetrahydrofuranyl group, a thiazolidinyl group, a piperidyl group, a piperazinyl group, a morpholinyl group, a dioxolanyl group, and a dioxanyl group.

Examples of the 5-membered heteroaryl groups include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the 6-membered heteroaryl group include a pyridyl group, a pyrazinyl group, a pyrimidinyl group, a pyridazinyl group, and a triazinyl group.

Examples of the 5-membered unsaturated heterocyclyl group include a pyrrolinyl group, a dihydrofuranyl group, an imidazolynyl group, a pyrazolynyl group, an oxazolynyl group, and isooxazolynyl group.

Examples of the 6-membered unsaturated heterocyclyl group include a dihydropyrany group.

($Z^{q-}$)

In the formula (II), $Z^{q-}$ is a counter ion, and q indicates a valence of the counter ion and is 1 or 2.

Specific examples of a monovalent anion $Z^-$ include $Cl^-$, $Br^-$, $I^-$, $NO_3^-$, $CH_3COO^-$, $CH_3SO_3^-$, $CF_3SO_3^-$, and $TolSO_3^-$. Tol is an abbreviation which refers to an o-methylphenyl group, m-methylphenyl group or a p-methylphenyl group.

Specific examples of a divalent anion $Z^{2-}$ include $SO_4^{2-}$.

In the present invention, $Z^{q-}$ is preferably a monovalent anion $Z^-$, and specifically preferably $Cl^-$, $Br^-$, or $I^-$.

($Q^1$, $G^1$)

In the formulae (I) and (II), $Q^1$ is a pyrazole ring, an imidazole ring, a 1,2,4-triazole ring, a pyridine ring, a pyrazine ring, a pyrimidine ring, or a pyridazine ring, and is preferably an imidazole ring, a 1,2,4-triazole ring, a pyridine ring, or a pyrazine ring.

$G^1$ is a nitrogen atom or a carbon atom, and is preferably a carbon atom.

($X^1$, m)

In the formulae (I) and (II), $X^1$ is a halogeno group, a substituted or unsubstituted C1-6 alkyl group, a substituted or unsubstituted C2-6 alkenyl group, a substituted or unsubstituted C2-6 alkynyl group, a substituted or unsubstituted C1-6

alkoxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C1-6 alkylcarbonyl group, a substituted or unsubstituted C6-10 aryl group, a substituted or unsubstituted 5- or 6-membered heteroaryl group, a pentafluorosulfanyl group, a nitro group, or a cyano group.

m indicates the number of $X^1$, and m is an integer of 1 to 3 (preferably 1) when $Q^1$ is a 1,2,4-triazole ring, is an integer of 1 to 4 (preferably 1) when $Q^1$ is a pyrazole ring or an imidazole ring, is an integer of 0 to 4 (preferably 0 or 1) when $Q^1$ is a pyrazine ring, a pyrimidine ring or a pyridazine ring, or is an integer of 0 to 5 (preferably 0 or 1) when $Q^1$ is a pyridine ring. When m is 2 or more, $X^1$ may be identical to or different from each other.

$X^1$ on adjacent carbon atoms may form together a cyclohexene ring including these two carbon atoms.

Examples of the "halogeno group" as $X^1$ include a fluoro group, a chloro group, a bromo group, and an iodo group.

The "C1-6 alkyl group" as $X^1$ may be a linear chain or a branched chain. Examples of the "C1-6 alkyl group" as $X^1$ include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methylbutyl group, and an i-hexyl group.

Examples of the "C2-6 alkenyl group" as $X^1$ include a vinyl group, a 1-propenyl group, a 2-propenyl group, a 1-butenyl group, a 2-butenyl group, a 3-butenyl group, a 1-methyl-2-propenyl group, a 2-methyl-2-propenyl group, a 1-pentenyl group, a 2-pentenyl group, a 3-pentenyl group, a 4-pentenyl group, a 1-methyl-2-butenyl group, a 2-methyl-2-butenyl group, a 1-hexenyl group, a 2-hexenyl group, a 3-hexenyl group, a 4-hexenyl group, and a 5-hexenyl group.

Examples of the "C2-6 alkynyl group" as $X^1$ include an ethynyl group, a 1-propynyl group, a 2-propynyl group, a 1-butynyl group, a 2-butynyl group, a 3-butynyl group, a 1-methyl-2-propynyl group, a 2-methyl-3-butynyl group, a 1-pentynyl group, a 2-pentynyl group, a 3-pentynyl group, a 4-pentynyl group, a 1-methyl-2-butynyl group, a 2-methyl-3-pentynyl group, a 1-hexynyl group, and a 1,1-dimethyl-2-butynyl group.

Examples of the "C1-6 alkoxy group" as $X^1$ include a methoxy group, an ethoxy group, a n-propoxy group, a n-butoxy group, a n-pentyloxy group, a n-hexyloxy group, an i-propoxy group, an i-butoxy group, a s-butoxy group, a t-butoxy group, and an i-hexyloxy group.

Examples of the "C1-6 alkylthio group" as $X^1$ include a methylthio group, an ethylthio group, a n-propylthio group, a n-butylthio group, a n-pentylthio group, a n-hexylthio group, and an i-propylthio group.

Examples of the "C1-6 alkylsulfinyl group" as $X^1$ include a methylsulfinyl group, an ethylsulfinyl group, and a t-butylsulfinyl group.

Examples of the "C1-6 alkylsulfonyl group" as $X^1$ include a methylsulfonyl group, an ethylsulfonyl group, and a t-butylsulfonyl group.

Examples of the "C1-6 alkylcarbonyl group" as $X^1$ include an acetyl group and a propionyl group.

As a substituent on the "C1-6 alkyl group", "C2-6 alkenyl group", "C2-6 alkynyl group", "C1-6 alkoxy group", "C1-6 alkylthio group", "C1-6 alkylsulfinyl group", "C1-6 alkylsulfonyl group" or "C1-6 alkylcarbonyl group" as $X^1$, a halogeno group such as a fluoro group, a chloro group, a bromo group, or an iodo group; a hydroxy group; a C1-6 alkoxy group such as a methoxy group, an ethoxy group, a n-propoxy group, an i-propoxy group, a n-butoxy group, a s-butoxy group, an i-butoxy group, or a t-butoxy group; a C1-6 haloalkoxy group such as a 2-chloro-n-propoxy group, a 2,3-dichlorobutoxy group, or a trifluoromethoxy group; a C3-6 cycloalkyl group such as a cyclopropyl group, a cyclobutyl group, a cyclopenthyl group, or a cyclohexyl group; a C6-10 aryl group such as a phenyl group, or a naphthyl group; a C6-10 aryl group substituted with a halogeno group, a C1-6 haloalkyl group, or a C1-6 haloalkoxy group, such as a 4-chlorophenyl group, a 4-trifluoromethylphenyl group, or a 4-trifluoromethoxyphenyl group; a tri C1-6 alkyl-substituted silyl group such as a trimethylsilyl group, a triethylsilyl group, or a t-butyldimethylsilyl group; a hydroxy group; or a cyano group is preferable, and a halogeno group is more preferable.

Examples of the "C3-8 cycloalkyl group" as $X^1$ include a cyclopropyl group, a cyclobutyl group, a cyclopentyl group, a cyclohexyl group, and a cycloheptyl group.

Examples of the "C6-10 aryl group" as $X^1$ include a phenyl group, and a naphthyl group.

The "5- or 6-membered heteroaryl group" as $X^1$ is a 5-membered ring or a 6-membered ring which contains, as a ring member atom, one, two, three, or four hetero atoms selected from the group consisting of a nitrogen atom, an oxygen atom and a sulfur atom. In the case where at least two hetero atoms are present, the hetero atoms may be identical to or different from each other.

Examples of the 5-membered heteroaryl groups include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, and a tetrazolyl group.

Examples of the 6-membered heteroaryl group include a pyridyl group, a pyrazinyl group, a pyrimidinyl group, a pyridazinyl group, and a triazinyl group.

Preferable examples of a substituent on the "C3-8 cycloalkyl group", "C6-10 aryl group", or "5- or 6-membered heteroaryl group" as $X^1$ include: halogeno groups such as a fluoro group, a chloro group, a bromo group, and an iodo group; C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group; C1-6 haloalkyl groups such as a chloromethyl group, a chloroethyl group, a trifluoromethyl group, a 1,2-dichloro-n-propyl group, and a 1-fluoro-n-butyl group; a hydroxy group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; and a cyano group.

In the present invention, $X^1$ is preferably a halogeno group; a halogeno-substituted, C1-6 alkoxy-substituted, C3-6 cycloalky-substituted, hydroxy-substituted or unsubstituted C1-6 alkyl group; an unsubstituted C2-6 alkynyl group; an unsubstituted C1-6 alkoxy group; an unsubstituted C1-6 alkylthio group; an unsubstituted C1-6 alkylcarbonyl group; or a cyano group, and is more preferably a halogeno group; a halogeno-substituted or unsubstituted C1-6 alkyl group; or an unsubstituted C1-6 alkoxy group.

Examples of the "halogeno-substituted C1-6 alkyl group" as $X^1$ include a difluoromethyl group, a trifluoromethyl group, a 2,2,2-trifluoroethyl group, and a 2,2,2,1,1-pentafluoroethyl group.

Specific examples of the structure including $Q^1$ and $X^1$ (aromacyclic quaternary ammonium cation) are shown below. The symbol "-*" in the structure indicates a bond with "N-" in formula (I) and a bond with "NH" in formula (II).

[Chemical Formula 4]

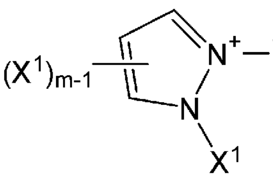
$Q^1$-1

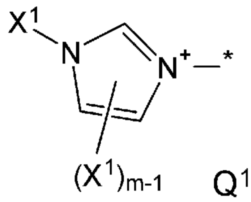
$Q^1$-2

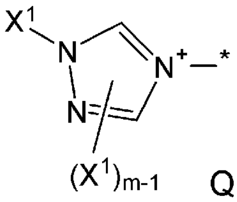
$Q^1$-3

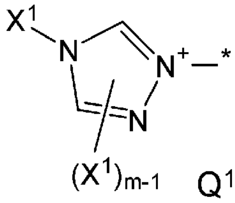
$Q^1$-4

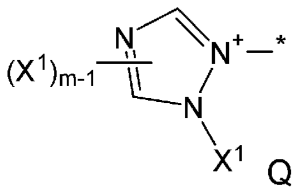
$Q^1$-5

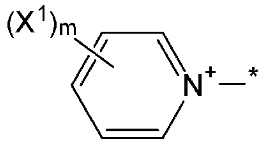
$Q^1$-6

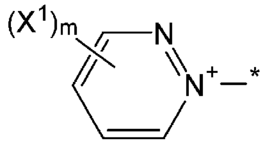
$Q^1$-7

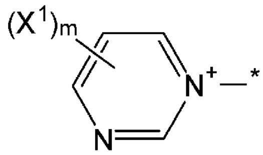
$Q^1$-8

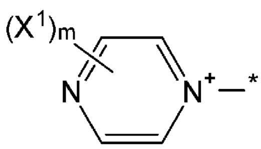
$Q^1$-9

In the present invention, the structure including $Q^1$ and $X^1$ is preferably $Q^1$-2, $Q^1$-3, $Q^1$-6, or $Q^1$-9, and more preferably $Q^1$-2, $Q^1$-3, $Q^1$-6, or $Q^1$-9, among the structures specifically shown above.

(A) In the formulae (I) and (II), A is an oxygen atom or a sulfur atom, and is preferably an oxygen atom.

($Q^2$)

$Q^2$ is a substituted or unsubstituted phenyl group, a substituted or unsubstituted 5- or 6-membered heteroaryl group, or a substituted or unsubstituted 9- or 10-membered heteroaryl group, preferably a substituted or unsubstituted phenyl group, a substituted or unsubstituted pyridyl group, a substituted or unsubstituted thienyl group, a substituted or unsubstituted pyrazolyl group, or a substituted or unsubstituted quinolyl group, more preferably a substituted or unsubstituted phenyl group or a substituted or unsubstituted pyridyl group, and even more preferably a substituted or unsubstituted phenyl group.

Examples of the "5- or 6-membered heteroaryl group" as $Q^2$ include the same groups as those exemplified as the "5- or 6-membered heteroaryl group" as $X^1$. Among these, a pyridyl group, a thienyl group, or a pyrazolyl group is preferable.

Examples of the "9- or 10-membered heteroaryl group" as $Q^2$ include bicyclic groups formed by condensing 5- or 6-membered saturated or partially-saturated heterocycles and benzene rings, such as an indolyl group, a benzimidazolyl group, a benzotriazolyl group, a quinolyl group, an isoquinolyl group, a quinazolyl group, a quinoxalyl group, a benzopyridazinyl group, a buterizinyl group, a chromenyl group, and an isochromenyl group, and a quinolyl group is preferable among them.

As a substituent on the "phenyl group", "5- or 6-membered heteroaryl group" or "9- or 10-membered heteroaryl group" as $Q^2$, a halogeno group such as a fluoro group, a chloro group, a bromo group, or an iodo group; a C1-6 alkyl group such as a methyl group, an ethyl group, a n-propyl group, an i-propyl group, a n-butyl group, a s-butyl group, an i-butyl group, a t-butyl group, a n-pentyl group, or a n-hexyl group; a C1-6 haloalkyl group such as a chloromethyl group, a chloroethyl group, a trifluoromethyl group, a 1,2-dichloro-n-propyl group, or a 1-fluoro-n-butyl group; a C2-6 haloalkenyl group such as a 2-chloro-1-propenyl group, a 2,2-dichloroethenyl group or a 2-fluoro-1-butenyl group; a C3-8 halocycloalkyl group such as a dichlorocyclopropyl group or a difluorocyclopropyl group; a C1-6 haloalkyl C3-8 cycloalkyl group such as a trifluoromethyl cyclopropyl group; a cyano-substituted C3-8 cycloalkyl group such as a cyanocyclopropyl group; a hydroxy group; a C1-6 alkoxy group such as a methoxy group, an ethoxy group, a n-propoxy group, an i-propoxy group, a n-butoxy group, a s-butoxy group, an i-butoxy group, or a t-butoxy group; a C1-6 haloalkoxy group such as a 2-chloro-n-propoxy group, a 2,3-dichlorobutoxy group, or a trifluoromethoxy group; a C1-6 haloalkoxy C1-6 haloalkoxy group such as a trifluoromethoxy trifluoroethoxy group or a heptafluoropropoxy trifluoroethoxy group; a C1-6 haloalkoxy C1-6 haloalkoxy C1-6 haloalkoxy group such as a trifluoromethoxy hexafluoropropoxy trifluoroethoxy group, or a heptafluoropropoxy hexafluoropropoxy trifluoroethoxy group; a C1-6 haloalkylthio group such as a trifluoromethylthio group or a tetrafluoroethylthio group; a pentafluorosulfanyl group; a C6-10 haloaryloxy group such a fluorophenoxy group; a C1-6 haloalkyl C6-10 aryloxy group such as a trifluoromethylphenoxy group; or a cyano group is preferable.

As a substituent on the "phenyl group" as $Q^2$, a halogeno group; a C1-6 alkyl group; a C1-6 haloalkyl group; a C1-6 haloalkyl C3-8 cycloalkyl group; a hydroxy group; a C1-6 alkoxy group; a C1-6 haloalkoxy group; a C1-6 haloalkoxy C1-6 haloalkoxy group; a C1-6 haloalkoxy C1-6 haloalkoxy C1-6 haloalkoxy group; a C1-6 haloalkylthio group; or a cyano group is preferable, and a C1-6 haloalkyl group; a C1-6 haloalkyl C3-8 cycloalkyl group; a C1-6 haloalkoxy group; a C1-6 haloalkoxy C1-6 haloalkoxy group; a C1-6 haloalkoxy C1-6 haloalkoxy C1-6 haloalkoxy group; or a C1-6 haloalkylthio group is more preferable.

As a substituent on the "pyridyl group" as $Q^2$, a C1-6 haloalkyl group such as a chloromethyl group, a chloroethyl group, a trifluoromethyl group, a 1,2-dichloro-n-propyl group, or a 1-fluoro-n-butyl group; or a C1-6 haloalkoxy group such as a 2-chloro-n-propoxy group, a 2,3-dichlorobutoxy group, or a trifluoromethoxy group is preferable.

As a substituent on the "thienyl group" as $Q^2$, a C1-6 haloalkyl group such as a chloromethyl group, a chloroethyl group, a trifluoromethyl group, a 1,2-dichloro-n-propyl group, or a 1-fluoro-n-butyl group is preferable.

As a substituent on the "pyrazolyl group" as $Q^2$ include a C2-6 haloalkenyl group such as a 2-chloro-1-propenyl group, a 2,2-dichloroethenyl group or a 2-fluoro-1-butenyl group is preferable.

As a substituent on the "quinolyl group" as $Q^2$ include a C1-6 haloalkyl groups such as a chloromethyl group, a chloroethyl group, a trifluoromethyl group, a 1,2-dichloro-n-propyl group, or a 1-fluoro-n-butyl group is preferable.

($X^2$)

$X^2$ is a halogeno group, a substituted or unsubstituted C1-6 alkyl group, a substituted or unsubstituted C2-6 alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C3-8 cycloalkyl group, a substituted or unsubstituted C6-10 aryl group, a substituted or unsubstituted C6-10 aryloxy group, a substituted or unsubstituted pyrazolyl group, an aldehyde group, a cyano group, or $-C=N-OCH_3$.

Specific examples of the "halogeno group", "C1-6 alkyl group", "C2-6 alkynyl group", "C1-6 alkoxy group", "C1-6

alkylthio group", "C3-8 cycloalkyl group" and "C6-10 aryl group" as $X^2$ include the same groups as $X^1$.

Examples of the "C1-6 alkoxycarbonyl group" as $X^2$ include a methoxycarbonyl group, an ethoxycarbonyl group, a n-propoxycarbonyl group, an i-propoxycarbonyl group, a n-butoxycarbonyl group, and t-butoxycarbonyl group.

Examples of the "C6-10 aryloxy group" as $X^2$ include a phenoxy group and a naphthoxy group.

As a substituent on the "C1-6 alkyl group", "C1-6 alkoxy group", "C1-6 alkoxycarbonyl group", or "C1-6 alkylthio group" as $X^2$, a halogeno group such as a fluoro group, a chloro group, a bromo group, or an iodo group; a hydroxy group; a C1-6 alkoxy group such as a methoxy group, an ethoxy group, a n-propoxy group, an i-propoxy group, a n-butoxy group, a s-butoxy group, an i-butoxy group, or a t-butoxy group; a C1-6 haloalkoxy group such as a 2-chloro-n-propoxy group, a 2,3-dichlorobutoxy group, or a trifluoromethoxy group; a C6-10 aryl group such as a phenyl group or a naphthyl group; a halogeno-substituted, C1-6 haloalkyl-substituted, or C1-6 haloalkoxy-substituted C6-10 aryl group such as a 4-chlorophenyl group, a 4-trifluoromethylphenyl group or a 4-trifluoromethoxyphenyl group; a C6-10 aryloxy group such as a phenoxy group; a halogeno-substituted, C1-6 haloalkyl-substituted, or C1-6 haloalkoxy-substituted C6-10 aryloxy group such as a 4-chlorophenoxy group, a 4-trifluoromethylphenoxy group or a 4-trifluoromethoxyphenoxy group; or a cyano group is preferable, a halogeno group or a C1-6 haloalkoxy group is more preferable, and a halogeno group is even more preferable.

As a substituent on the "C2-6 alkynyl group" as $X^2$, a tri C1-6 alkyl-substituted silyl group such as a trimethylsilyl group, a triethylsilyl group, or a t-butyldimethylsilyl group is preferable, and a trimethylsilyl group is more preferable.

As a substituent on the "C3-8 cycloalkyl group", "C6-10 aryl group", "C6-10 aryloxy group" or "pyrazolyl group" as $X^2$, a halogeno group such as a fluoro group, a chloro group, a bromo group, or an iodo group; or a C1-6 haloalkoxy group such as a 2-chloro-n-propoxy group, a 2,3-dichlorobutoxy group, or a trifluoromethoxy group is preferable, and a C1-6 haloalkoxy group is more preferable.

In the present invention, $X^2$ is preferably a halogeno group; an unsubstituted C1-6 alkyl group; a C1-6 haloalkyl group; an unsubstituted C1-6 alkoxy group; a C1-6 haloalkoxy group; a C1-6 alkoxycarbonyl group; a C2-6 alkynyl group which may be substituted with a tri C1-6 alkyl-substituted silyl group; an unsubstituted C1-6 alkylthio group; an unsubstituted C3-8 cycloalkyl group; a phenyl group; a C1-6 haloalkoxy phenyl group; a halogeno-substituted phenyl group; a C1-6 haloalkoxy phenoxy group; a C1-6 haloalkoxy phenyl group; an unsubstituted pyrazolyl group; an aldehyde group; a cyano group; or $-C=N-OCH_3$ is preferable, a halogeno group; a C1-6 haloalkyl group; an unsubstituted C1-6 alkoxy group; a C2-6 alkynyl group which may be substituted with a tri C1-6 alkyl-substituted silyl group; or a cyano group is more preferable, a halogeno group; a C2-6 alkynyl group which may be substituted with a tri C1-6 alkyl-substituted silyl group; or a cyano group is even more preferable, and a halogeno group is even more preferable.

($X^3$, n)

$X^3$ is a halogeno group, a substituted or unsubstituted C1-6 alkyl group, a substituted or unsubstituted C1-6 alkoxy group, or a cyano group.

Specific examples of these groups as $X^3$ include the same groups as those provided as exemplary examples of $X^1$.

**In** the present invention, $X^3$ is preferably a halogeno group; a halogeno-substituted or unsubstituted C1-6 alkyl group; a halogeno-substituted or unsubstituted C1-6 alkoxy group; or a cyano group, and more preferably a halogeno group; an unsubstituted C1-6 alkyl group; or a cyano group.

n indicates the number of $X^3$, and is an integer of 0 to 2, and preferably 0. When n is 2, $X^3$ may be identical to or different from each other.

$X^2$ and $X^3$ on adjacent carbon atoms may form together a substituted or unsubstituted 5-membered hetero ring including these two carbon atoms. The "5-membered hetero ring" is a 5-membered ring which contains, as (a) ring member atom(s), one, two, or three hetero atom(s) selected from the group consisting of a nitrogen atom, an oxygen atom and a sulfur atom. In the case where at least two hetero atoms are contained, the hetero atoms may be identical to or different from each other.

Specific examples thereof include: partially unsaturated oxygen-containing hetero rings such as a 2,3-dihydrofuran ring, a 2,5-dihydrofuran ring, and a 1,3-dioxole ring; and 5-membered heteroaryl rings such as a furan ring, a thiophen ring, an oxazole ring, an isoxazole ring, a thiazole ring, and an isothiazole ring.

Preferable examples of a substituent on the "5-membered hetero ring" include: halogeno groups such as a fluoro group, a chloro group, a bromo group, and an iodo group; C1-6 alkyl groups such as a methyl group, an ethyl group, a n-propyl group, an i-propyl group, a n-butyl group, a s-butyl group, an i-butyl group, a t-butyl group, a n-pentyl group, and a n-hexyl group; C1-6 haloalkyl groups such as a chloromethyl group, a chloroethyl group, a trifluoromethyl group, a 1,2-dichloro-n-propyl group, and a 1-fluoro-n-butyl group; a hydroxy group; C1-6 alkoxy groups such as a methoxy group, an ethoxy group, a n-propoxy group, an i-propoxy group, a n-butoxy group, a s-butoxy group, an i-butoxy group, and a t-butoxy group; C1-6 haloalkoxy groups such as a 2-chloro-n-propoxy group, a 2,3-dichlorobutoxy group, and a trifluoromethoxy group; and a cyano group.

The aromacyclic quaternary ammonium compound of the present invention is not particularly limited by the preparation method thereof. For example, the aromacyclic quaternary ammonium compound of the present invention (hereinafter, may be abbreviated as "the compound of the present invention") may be obtained by the method mentioned in examples using conventionally-known reactions.

The compound of the present invention may also be prepared by the following methods, for example.

(Preparation of N-aminoonium salt)

[Chemical Formula 5]

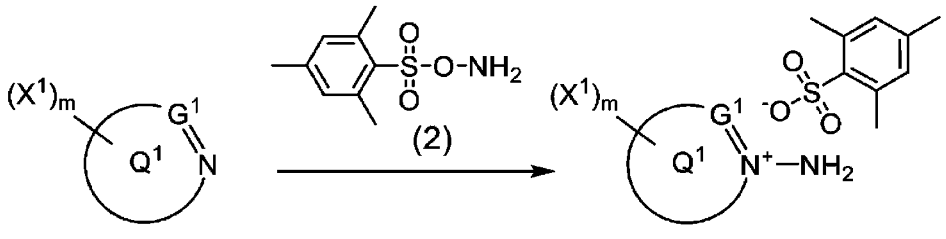

(1) (2) (3)

A heteroaryl compound (1) and an O-(mesitylsulfonyl) hydroxylamine (2) are reacted to prepare a N-aminoonium salt (3).

A hydroxylamine O-sulfonate, or an O-(diphenylphosphinyl) hydroxylamine may also be used instead of the O-(mesitylsulfonyl) hydroxylamine.

In the reaction formulae, $G^1$, $Q^1$, $X^1$, and m are the same groups as those in the above-mentioned formulae (I) and (II), respectively.

[Chemical Formula 6]

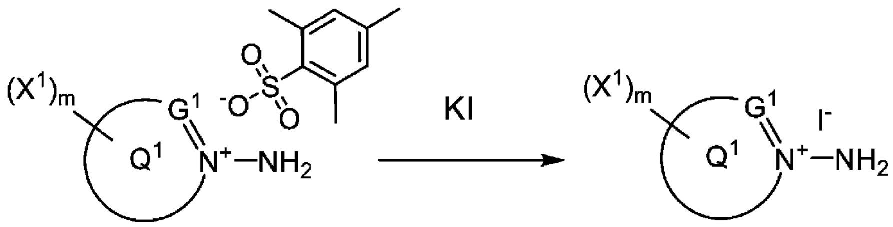


(3) (4)

Although the N-aminoonium salt (3) may be directly subjected to the succeeding condensation reaction with a carboxylic acid compound (5), the N-aminoonium salt (3) may be subjected to salt exchange to obtain an N-aminoonium salt (4), which is an iodide salt, in the case where the safety of a substrate is required to be considered.

In the reaction formulae, $G^1$, $Q^1$, $X^1$, and m are the same groups as those in the formulae (I) and (II), respectively.

(Condensation reaction)

[Chemical Formula 7]

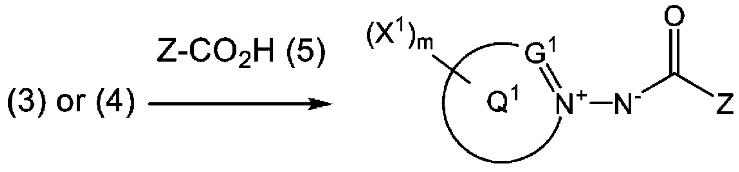


(6)

The N-aminoonium salt (3) or (4) and the carboxylic acid compound (5) are condensed in the presence of a condensation agent available in amide synthesis to prepare the compound of the present invention (6).
Examples of the condensation agent include DCC (dicyclohexylcarbodiimide), DIC (diisopropylcarbodiimide), and EDI (1-ethyl-3-(3-dimethylaminopropyl)-carbodiimide).
In the reaction formulae, $G^1$, $Q^1$, $X^1$, and m are the same groups as those in the formula (I) and formula (II), respectively, and Z indicates a pyridine ring having $X^2$ and $(X^3)n$, $-O-Q^2$ being bonded to the pyridine ring, in the formulae (I) and (II). A substituent on $Q^2$ may be changed appropriately after the condensation reaction.
In the case where a compound of formula (I) or (II) in which A is a sulfur atom is prepared, the compound may be prepared by using a dithiocarboxylic acid ester (7) instead of the carboxylic acid compound (5).

[Chemical Formula 8]

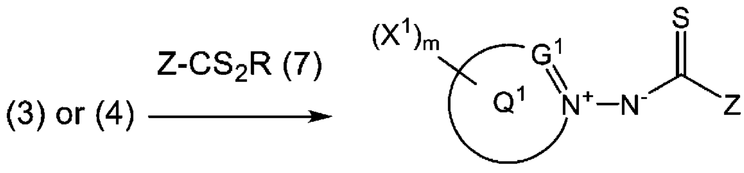


(8)

In the condensation reaction with the dithiocarboxylic acid ester (7), a base such as calcium carbonate or sodium ethoxide is preferably used.
In the reaction formulae, $G^1$, $Q^1$, $X^1$, and m are the same groups as those in the formulae (I) and (II), respectively, Z indicates a pyridine ring having $X^2$ and $(X^3)n$, $-O-Q^2$ being bonded to the pyridine ring, in the formula (I) and formula (II), and R is a C1-6 alkyl group such as a methyl group or an ethyl group. A substituent on $Q^2$ may be changed appropriately after the condensation reaction.
The compound of the present invention exhibits excellent control effects against pests, such as various agricultural insect pests or acarians which affect plant growth.
In addition, the compound of the present invention is a highly safe substance as it exhibits low phytotoxicity against crop plants and low toxicity to fish and warm-blooded animals. Thus, the compound of the present invention is useful as an active ingredient of an insecticide or acaricide.
In addition, in recent years, as a result of increased resistance to various existing drugs in many insect pests such as plutellidae, delphacidae, cicadellidae, and aphids, the problem of a lack of efficacy of these drugs has arisen, and thus drugs effective against insect pests in resistant strains are also desired. The compound of the present invention has excellent control effects not only on susceptible strains but also on insect pests of various resistant strains and even acarians of acaricide-resistant strains.
The compound of the present invention has excellent control effects against ectoparasites and endoparasites that cause harm to humans or livestock. In addition, the compound according to the present invention is a highly safe substance as it has low toxicity to fish and warm-blooded animals. Thus, the compound of the present invention is useful as an active ingredient of an ectoparasite-control agent or an endoparasite-control agent.
The compound of the present invention exhibits efficacy in all developmental stages of organisms to be controlled, and exhibits excellent control effects against eggs, nymphs, larvae, pupae, and adults of acarians or insects, for example.

(Pest control agent, insecticide or acaricide)

A pest control agent, insecticide or acaricide of the present invention contains at least one selected from the

compounds of the present invention, as an active ingredient thereof. The amount of the compound of the present invention contained in the pest control agent, insecticide or acaricide of the present invention is not particularly limited, provided that control effects are exhibited against pests, agricultural insect pests or acarians.

It is preferable that the pest control agent, insecticide or acaricide of the present invention be applied to cereals; vegetables; root vegetables; potatoes; flowers and ornamental plants; fruit-bearing trees; foliage plants; trees, such as tea, coffee, or cacao; feed crops; lawn grasses; or plants such as cotton.

In the application to plants, the pest control agent, insecticide or acaricide of the present invention may be applied to any portions, such as leaves, stems, stalks, flowers, buds, fruits, seeds, sprouts, roots, tubers, tuberous roots, shoots, or slips.

The pest control agent, insecticide or acaricide of the present invention is not particularly limited by the species of plant to which it is applied. Examples of the plant species include original species, varieties, improved varieties, cultivars, mutants, hybrid bodies, and gene recombinants (GMO).

The pest control agent according to the present invention may be used to control various agricultural insect pests and acarians by conducting seed treatment, foliage application, soil application, or submerged application.

Specific examples of agricultural insect pests and acarians to be controlled with the pest control agent of the present invention are shown below.

(1) Butterflies and moths of the order Lepidoptera

(a) Moths belonging to the family Arctiidae, such as Hyphantria cunea, and Lemyra imparilis;
(b) Moths belonging to the family Bucculatricidae, such as Bucculatrix pyrivorella;
(c) Moths belonging to the family Carposinidae, such as Carposina sasakii;
(d) Moths belonging to the family Crambidae, such as Diaphania indica, and Diaphania nitidalis, of Diaphania spp.; Ostrinia furnacalis, Ostrinia nubilalis, and Ostrinia scapulalis, of Ostrinia spp.; and others such as Chilo suppressalis, Cnaphalocrocis medinalis, Conogethes punctiferalis, Diatraea grandiosella, Glyphodes pyloalis, Hellula undalis, and Parapediasia teterrella;
(e) Moths belonging to the family Gelechiidae, such as Helcystogramma triannulella, Pectinophora gossypiella, Phthorimaea operculella, and Sitotroga cerealella;
(f) Moths belonging to the family Geometridae, such as Ascotis selenaria;
(g) Moths belonging to the family Gracillariidae, such as Caloptilia theivora, Phyllocnistis citrella, and Phyllonorycter ringoniella;
(h) Butterflies belonging to the family Hesperiidae, such as Parnara guttata;
(i) Moths belonging to the family Lasiocampidae, such as Malacosoma neustria;
(j) Moths belonging to the family Lymantriidae, such as Lymantria dispar, and Lymantria monacha, of Lymantria spp.; and others such as Euproctis pseudoconspersa, and Orgyia thyellina;
(k) Moths belonging to the family Lyonetiidae, such as Lyonetia clerkella, and Lyonetia prunifoliella malinella, of Lyonetia spp.;
(l) Moths belonging to the family Noctuidae, such as Spodoptera depravata, Spodoptera eridania, Spodoptera exigua, Spodoptera frugiperda, Spodoptera littoralis, and Spodoptera litura, of Spodoptera spp.; Autographa gamma, and Autographa nigrisigna, of Autographa spp.; Agrotis ipsilon, and Agrotis segetum, of Agrotis spp.; Helicoverpa armigera, Helicoverpa assulta, and Helicoverpa zea, of Helicoverpa spp.; Heliothis armigera, and Heliothis virescens, of Heliothis spp.; and others such as Aedia leucomelas, Ctenoplusia agnata, Eudocima tyrannus, Mamestra brassicae, Mythimna separata, Naranga aenescens, Panolis japonica, Peridroma saucia, Pseudoplusia includens, and Trichoplusia ni;
(m) Moths belonging to the family Nolidae, such as Earias insulana;
(n) Butterflies belonging to the family Pieridae, such as Pieris brassicae, and Pieris rapae crucivora, of Pieris spp.;
(o) Moths belonging to the family Plutellidae, such as Acrolepiopsis sapporensis, and Acrolepiopsis suzukiella, of Acrolepiopsis spp.; and others such as Plutella xylostella;
(p) Moths belonging to the family Pyralidae, such as Cadra cautella, Elasmopalpus lignosellus, Etiella zinckenella, and Galleria mellonella;
(q) Moths belonging to the family Sphingidae, such as Manduca quinquemaculata, and Manduca sexta, of Manduca spp.;
(r) Moths belonging to the family Stathmopodidae, such as Stathmopoda masinissa;
(s) Moths belonging to the family Tineidae, such as Tinea translucens;
(t) Moths belonging to the family Tortricidae, such as Adoxophyes honmai, and Adoxophyes orana, of Adoxophyes spp.; Archips breviplicanus, and Archips fuscocupreanus of Archips spp.; and others such as Choristoneura fumiferana, Cydia pomonella, Eupoecilia ambiguella, Grapholitha molesta, Homona magnanima,

Leguminivora glycinivorella, Lobesia botrana, Matsumuraeses phaseoli, Pandemis heparana, and Sparganothis pilleriana;
(u) Moths belonging to the family Yponomeutidae, such as Argyresthia conjugella.

(2) Insect pests of the order Thysanoptera

(a) Insect pests belonging to the family Phlaeothripidae, such as Ponticulothrips diospyrosi;
(b) Insect pests belonging to the family Thripidae, such as Frankliniella intonsa, and Frankliniella occidentalis, of Frankliniella spp.; Thrips palmi, and Thrips tabaci, of Thrips spp.; and others such as Heliothrips haemorrhoidalis, and Scirtothrips dorsalis.

(3) Insect pests of the order Hemiptera

(A) The infraorder Archaeorrhyncha

(a) Insect pests belonging to the family Delphacidae, such as Laodelphax striatella, Nilaparvata lugens, Perkinsiella saccharicida, and Sogatella furcifera.

(B) The infraorder Clypeorrhyncha

(a) Insect pests belonging to the family Cicadellidae, such as Empoasca fabae, Empoasca nipponica, Empoasca onukii, and Empoasca sakaii, of Empoasca spp.; and others such as Arboridia apicalis, Balclutha saltuella, Epiacanthus stramineus, Macrosteles striifrons, and Nephotettix cinctinceps.

(C) The infraorder Heteroptera

(a) Insect pests belonging to the family Alydidae, such as Riptortus clavatus;
(b) Insect pests belonging to the family Coreidae, such as Cletus punctiger, and Leptocorisa chinensis;
(c) Insect pests belonging to the family Lygaeidae, such as Blissus leucopterus, Cavelerius saccharivorus, and Togo hemipterus;
(d) Insect pests belonging to the family Miridae, such as Halticus insularis, Lygus lineolaris, Psuedatomoscelis seriatus, Stenodema sibiricum, Stenotus rubrovittatus, and Trigonotylus caelestialium;
(e) Insect pests belonging to the family Pentatomidae, such as Nezara antennata, and Nezara viridula, of Nezara spp.; Eysarcoris aeneus, Eysarcoris lewisi, and Eysarcoris ventralis, of Eysarcoris spp.; and others such as Dolycoris baccarum, Eurydema rugosum, Glaucias subpunctatus, Halyomorpha halys, Piezodorus hybneri, Plautia crossota, and Scotinophora lurida;
(f) Insect pests belonging to the family Pyrrhocoridae, such as Dysdercus cingulatus;
(g) Insect pests belonging to the family Rhopalidae, such as Rhopalus msculatus;
(h) Insect pests belonging to the family Scutelleridae, such as Eurygaster integriceps);
(i) Insect pests belonging to the family Tingidae, such as Stephanitis nashi.

(D) The infraorder Sternorrhyncha

(a) Insect pests belonging to the family Adelgidae, such as Adelges laricis;
(b) Insect pests belonging to the family Aleyrodidae, such as Bemisia argentifolii, and Bemisia tabaci, of Bemisia spp.; and others such as Aleurocanthus spiniferus, Dialeurodes citri, and Trialeurodes vaporariorum;
(c) Insect pests belonging to the family Aphididae, such as Aphis craccivora, Aphis fabae, Aphis forbesi, Aphis gossypii, Aphis pomi, Aphis sambuci, and Aphis spiraecola, of Aphis spp.; Rhopalosiphum maidis, and Rhopalosiphum padi, of Rhopalosiphum spp.; Dysaphis plantaginea, and Dysaphis radicola, of Dysaphis spp.; Macrosiphum avenae, and Macrosiphum euphorbiae, of Macrosiphum spp.; Myzus cerasi, Myzus persicae, and Myzus varians, of Myzus spp.; and others such as Acyrthosiphon pisum, Aulacorthum solani, Brachycaudus helichrysi, Brevicoryne brassicae, Chaetosiphon fragaefolii, Hyalopterus pruni, Hyperomyzus lactucae, Lipaphis erysimi, Megoura viciae, Metopolophium dirhodum, Nasonovia ribis-nigri, Phorodon humuli, Schizaphis graminum, Sitobion avenae, and Toxoptera aurantii;
(d) Insect pests belonging to the family Coccidae, such as Ceroplastes ceriferus, and Ceroplastes rubens, of Ceroplastes spp.;
(e) Insect pests belonging to the family Diaspididae, such as Pseudaulacaspis pentagona, and Pseudaulacaspis prunicola, of Pseudaulacaspis spp.; Unaspis euonymi, and Unaspis yanonensis, of Unaspis spp.; and others such as Aonidiella aurantii, Comstockaspis perniciosa, Fiorinia theae, and Pseudaonidia paeoniae;

(f) Insect pests belonging to the family Margarodidae, such as Drosicha corpulenta, and Icerya purchasi;

(g) Insect pests belonging to the family Phylloxeridae, such as Viteus vitifolii;

(h) Insect pests belonging to the family Pseudococcidae, such as Planococcus citri, and Planococcus kuraunhiae, of Planococcus spp.; and others such as Phenacoccus solani, and Pseudococcus comstocki;

(i) Insect pests belonging to the family Psyllidae, such as Psylla mali, and Psylla pyrisuga, of Psylla spp.; and others such as Diaphorina citri.

(4) Insect pests of the infraorder Polyphaga

(a) Insect pests belonging to the family Anobiidae, such as Lasioderma serricorne;

(b) Insect pests belonging to the family Attelabidae, such as Byctiscus betulae, and Rhynchites heros;

(c) Insect pests belonging to the family Bostrichidae, such as Lyctus brunneus;

(d) Insect pests belonging to the family Brentidae, such as Cylas formicarius;

(e) Insect pests belonging to the family Buprestidae, such as Agrilus sinuatus;

(f) Insect pests belonging to the family Cerambycidae, such as Anoplophora malasiaca, Monochamus alternatus, Psacothea hilaris, and Xylotrechus pyrrhoderus;

(g) Insect pests belonging to the family Chrysomelidae, such as Bruchus pisorum, and Bruchus rufimanus, of Bruchus spp.; Diabrotica barberi, Diabrotica undecimpunctata, and Diabrotica virgifera, of Diabrotica spp.; Phyllotreta nemorum, and Phyllotreta striolata, of Phyllotreta spp.; and others such as Aulacophora femoralis, Callosobruchus chinensis, Cassida nebulosa, Chaetocnema concinna, Leptinotarsa decemlineata, Oulema oryzae, and Psylliodes angusticollis;

(h) Insect pests belonging to the family Coccinellidae, such as Epilachna varivestis, and Epilachna vigintioctopunctata, of Epilachna spp.;

(i) Insect pests belonging to the family Curculionidae, such as Anthonomus grandis, and Anthonomus pomorum, of Anthonomus spp.; Sitophilus granarius, and Sitophilus zeamais, of Sitophilus spp.; and others such as Echinocnemus squameus, Euscepes postfasciatus, Hylobius abietis, Hypera postica, Lissohoptrus oryzophilus, Otiorhynchus sulcatus, Sitona lineatus, and Sphenophorus venatus;

(j) Insect pests belonging to the family Elateridae, such as Melanotus fortnumi, and Melanotus tamsuyensis, of Melanotus spp.;

(k) Insect pests belonging to the family Nitidulidae, such as Epuraea domina;

(l) Insect pests belonging to the family Scarabaeidae, such as Anomala cuprea, and Anomala rufocuprea, of Anomala spp.; and others such as Cetonia aurata, Gametis jucunda, Heptophylla picea, Melolontha melolontha, and Popillia japonica;

(m) Insect pests belonging to the family Scolytidae, such as Ips typographus;

(n) Insect pests belonging to the family Staphylinidae, such as Paederus fuscipes;

(o) Insect pests belonging to the family Tenebrionidae, such as Tenebrio molitor, and Tribolium castaneum;

(p) Insect pests belonging to the family Trogossitidae, such as Tenebroides mauritanicus.

(5) Insect pests of the order Diptera

(A) The infraorder Brachycera

(a) Insect pests belonging to the family Agromyzidae, such as Liriomyza bryoniae, Liriomyza chinensis, Liriomyza sativae, and Liriomyza trifolii, of Liriomyza spp.; and others such as Chromatomyia horticola, and Agromyza oryzae;

(b) Insect pests belonging to the family Anthomyiidae, such as Delia platura, and Delia radicum, of Delia spp.; and others such as Pegomya cunicularia;

(c) Insect pests belonging to the family Drosophilidae, such as Drosophila melanogaster, and Drosophila suzukii, of Drosophila spp.;

(d) Insect pests belonging to the family Ephydridae, such as Hydrellia griseola;

(e) Insect pests belonging to the family Psilidae, such as Psila rosae;

(f) Insect pests belonging to the family Tephritidae, such as Bactrocera cucurbitae, and Bactrocera dorsalis, of Bactrocera spp.; Rhagoletis cerasi, and Rhagoletis pomonella, of Rhagoletis spp.; and others such as Ceratitis capitata, and Dacus oleae.

(B) The infraorder Nematocera

(a) Insect pests belonging to the family Cecidomyiidae, such as Asphondylia yushimai, Contarinia sorghicola, Mayetiola destructor, and Sitodiplosis mosellana.

(6) Insect pests of the order Orthoptera

(a) Insect pests belonging to the family Acrididae, such as Schistocerca americana, and Schistocerca gregaria, of Schistocerca spp.; and others such as Chortoicetes terminifera, Dociostaurus maroccanus, Locusta migratoria, Locustana pardalina, Nomadacris septemfasciata, and Oxya yezoensis;
(b) Insect pests belonging to the family Gryllidae, such as Acheta domestica, and Teleogryllus emma;
(c) Insect pests belonging to the family Gryllotalpidae, such as Gryllotalpa orientalis;
(d) Insect pests belonging to the family Tettigoniidae, such as Tachycines asynamorus.

(7) Acarians (Acari)

(A) Acaridida of the order Astigmata

(a) Acarians belonging to the family Acaridae, such as Rhizoglyphus echinopus, and Rhizoglyphus robini, of Rhizoglyphus spp.; Tyrophagus neiswanderi, Tyrophagus perniciosus, Tyrophagus putrescentiae, and Tyrophagus similis, of Tyrophagus spp.; and others such as Acarus siro, Aleuroglyphus ovatus, and Mycetoglyphus fungivorus;

(B) Actinedida of the order Prostigmata

(a) Acarians belonging to the family Tetranychidae, such as Bryobia praetiosa, and Bryobia rubrioculus, of Bryobia spp.; Eotetranychus asiaticus, Eotetranychus boreus, Eotetranychus celtis, Eotetranychus geniculatus, Eotetranychus kankitus, Eotetranychus pruni, Eotetranychus shii, Eotetranychus smithi, Eotetranychus suginamensis, and Eotetranychus uncatus, of Eotetranychus spp.; Oligonychus hondoensis, Oligonychus ilicis, Oligonychus karamatus, Oligonychus mangiferus, Oligonychus orthius, Oligonychus perseae, Oligonychus pustulosus, Oligonychus shinkajii, and Oligonychus ununguis, of Oligonychus spp.; Panonychus citri, Panonychus mori, and Panonychus ulmi, of Panonychus spp.; Tetranychus cinnabarinus, Tetranychus evansi, Tetranychus kanzawai, Tetranychus ludeni, Tetranychus quercivorus, Tetranychus phaselus, Tetranychus urticae, and Tetranychus viennensis, of Tetranychus spp.; Aponychus corpuzae, and Aponychus firmianae, of Aponychus spp.; Sasanychus akitanus, and Sasanychus pusillus, of Sasanychus spp.; Shizotetranychus celarius, Shizotetranychus longus, Shizotetranychus miscanthi, Shizotetranychus recki, and Shizotetranychus schizopus, of Shizotetranychus spp.; and others such as Tetranychina harti, Tuckerella pavoniformis, and Yezonychus sapporensis;
(b) Acarians belonging to the family Tenuipalpidae, such as Brevipalpus lewisi, Brevipalpus obovatus, Brevipalpus phoenicis, Brevipalpus russulus, and Brevipalpus californicus, of Brevipalpus spp.; Tenuipalpus pacificus, and Tenuipalpus zhizhilashviliae, of Tenuipalpus spp.; and others such as Dolichotetranychus floridanus;
(c) Acarians belonging to the family Eriophyidae, such as Aceria diospyri, Aceria ficus, Aceria japonica, Aceria kuko, Aceria paradianthi, Aceria tiyingi, Aceria tulipae, and Aceria zoysiea, of Aceria spp.; Eriophyes chibaensis, and Eriophyes emarginatae, of Eriophyes spp.; Aculops lycopersici, and Aculops pelekassi, of Aculops spp.; Aculus fockeui, and Aculus schlechtendali, of Aculus spp.; and others such as Acaphylla theavagrans, Calacarus carinatus, Colomerus vitis, Calepitrimerus vitis, Epitrimerus pyri, Paraphytoptus kikus, Paracalacarus podocarpi, and Phyllocotruta citri;
(d) Acarians belonging to the family Transonemidae, such as Tarsonemus bilobatus, and Tarsonemus waitei, of Tarsonemus spp.; and others such as Phytonemus pallidus, and Polyphagotarsonemus latus;
(e) Acarians belonging to the family Penthaleidae, such as Penthaleus erythrocephalus, and Penthaleus major, of Penthaleus spp.

The pest control agent of the present invention may be mixed or used with other active ingredients of fungicides, insecticides, acaricides, nematicides, or soil insect pest control agents, or plant regulatory agents, synergists, fertilizers, soil improvement agents, or animal feeds.
The combination of the compound of the present invention with other active ingredients may exhibit synergistic effects on insecticidal, acaricidal, or nematicidal activity. The synergistic effects can be confirmed by a commonly used

method using the Colby formula (Colby. S. R., Calculating Synergistic and Antagonistic Responses of Herbicide Combinations; Weeds 15, pages 20 to 22, 1967).

Specific examples of the insecticides, acaricides, nematicides, soil insect pest control agents, and anthelmintic agents which can be mixed or used with the pest control agent of the present invention are shown below.

(1) Acetylcholinesterase inhibitors:

(a) Carbamate-based: alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC, xylylcarb, fenothiocarb, aldoxycarb, allyxycarb, aminocarb, bufencarb, chloethocarb, fentiocarb, promecarb;

(b) Organophosphate-based: acephate, azamethiphos, azinphos-ethyl, azinphos-methyl, cadusafos, chloroethoxyfos, chlorfenvinphos, chlormephos, chlorpyrifos, chlorpyrifos-methyl, coumaphos, cyanophos, demeton-S-methyl, diazinon, dichlorvos / DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl=O-(methoxyaminothiophosphoryl)salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimphos, temephos, terbufos, tetrachlorvinphos, thiometon, triazophos, trichlorfon, vamidothion, bromophos-ethyl, cyanofenphos, demeton-S methyl sulfone, dialifos, dichlofenthion, dioxabenzofos, etrimfos, fensulfothion, fonofos, formothion, isazophos, iodofenphos, isocarbophos, methacrifos, pirimiphos-ethyl, phosphocarb, propaphos, prothoate, sulprophos.

(2) GABAergic chloride ion channel antagonists: acetoprole, chlordene, endosulfan, ethiprole, fipronil, pyrafluprole, pyriprole, camphlechlor, heptachlor, dienochlor, flufiprole.

(3) Sodium channel modulators: acrinathrin, allethrin, d-cis-trans allethrin, d-trans allethrin, bifenthrin, bioallethrin, bioallethrin S-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans isomers], deltamethrin, empenthrin [(EZ)-(1R)-isomer], esfenvalerate, ethofenprox, fenpropathrin, fenvalerate, flucythrinate, flumethrin, tau-fluvalinate, halfenprox, imiprothrin, kadethrin, permethrin, phenothrin [(1R)-trans isomer], prallethrin, pyrethrin, resmethrin, silafluofen, tetramethrin, tetramethrin [(1R)-isomer], tralomethrin, transfluthrin, profluthrin, dimefluthrin, bioethanomethrin, biopermethrin, transpermethrin, fenfluthrin, fenpirithrin, flufenprox, metofluthrin, protrifenbute, terallethrin.

(4) Nicotinic acetylcholine receptor agonist: acetamiprid, clothianidin, dinotefuran, imidacloprid, nitenpyram, nithiazine, thiacloprid, thiamethoxam, sulfoxaflor, nicotine, flupyradifurone, flupyrimin, triflumezopyrim, dicloromezotiaz.

(5) Nicotinic acetylcholine receptor allosteric modulators: spinetoram, spinosad.

(6) Chloride channel activators: abamectin, emamectin, emamectin benzoate, lepimectin, milbemectin, ivermectin, selamectin, doramectin, eprinomectin, moxidectin.

(7) Juvenile hormone-like substances: hydroprene, kinoprene, methoprene, fenoxycarb, pyriproxyfen, diofenolan, epofenonane, triprene.

(8) Other non-specific inhibitors: methyl bromide, halogenated alkyls, chloropicrin, sodium aluminum fluoride, sulfuryl fluoride, borax, boracic acid, disodium octaborate, sodium borate, sodium metaborate, tartar emetic, dazomet, metam, metam-potassium, metam-sodium.

(9) Homoptera selective feeding inhibitors: flonicamid, pymetrozine, pyrifluquinazon, afidopyropen.

(10) Acarian growth inhibitors: clofentezine, diflovidazin, hexythiazox, etoxazole.

(11) Insectan midgut inner membrane disrupting agent derived from microorganisms: Bacillus thuringiensis subspecies Isuraerenshi, Bacillus sphaericus, Bacillus thuringiensis subsp. Aizawai, Bacillus thuringiensis subspecies Kurstaki, Bacillus thuringiensis subspecies Tenebrionis, Bt crop protein, Cry1Ab, Cry1Ac, Cry1Fa, Cry1A.105, Cry2Ab, Vip3A, mCry3A, Cry3Ab, Cry3Bb, Cry34Ab1 / Cry35Ab1.

(12) Mitochondrial ATP biosynthetic enzyme inhibitors: diafenthiuron, azocyclotin, cyhexatin, fenbutatin oxide, propargite, tetradifon.

(13) Oxidative phosphorylation uncouplers: chlorfenapyr, sulfluramid, DNOC, binapacryl, dinobuton, dinocap.

(14) Nicotinic acetylcholine receptor channel blockers: bensultap, cartap hydrochloride, thiosultap-sodium, thiocyclam.

(15) Chitin synthesis inhibitors: bistrifluron, chlorfluazuron, diflubenzuron, flucycloxuron, flufenoxuron, hexaflumuron, lufenuron, novaluron, noviflumuron, teflubenzuron, triflumuron, buprofezin, fluazuron.

(16) Diptera molting disrupting agents: cyromazine.

(17) Molting hormone receptor agonists: chromafenozide, halofenozide, methoxyfenozide, tebufenozide.
(18) Octopamine receptor agonists: amitraz, chlordimeform.
(19) Mitochondrial electron transport system complex III inhibitors: acequinocyl, fluacrypyrim, hydramethylnon, bifenazate.
(20) Mitochondrial electron transport system complex I inhibitors: fenazaquin, fenpyroximate, pyrimidifen, pyridaben, tebufenpyrad, tolfenpyrad, rotenone.
(21) Voltage-dependent sodium channel blockers: indoxacarb, metaflumizone.
(22) Acetyl CoA carboxylase inhibitors: spirodiclofen, spiromesifen, spirotetramat, spiropidion.
(23) Mitochondrial electron transport system complex IV inhibitors: aluminum phosphide, calcium phosphide, phosphine, zinc phosphide, cyanide.
(24) Mitochondrial electron transport system complex II inhibitors: cyenopyrafen, cyflumetofen, pyflubumide.
(25) Ryanodine receptor modulators: chlorantraniliprole, cyantraniliprole, flubendiamide, cyclaniliprole, tetraniliprole, cyhalodiamide, tetrachlorantraniliprole.
(26) GABAergic chloride ion channel allosteric modulator: broflanilide, fluxametamide, isocycloseram, afoxolaner, fluralaner, lotilaner, sarolanar.
(27) Other agents (mechanisms of which are unknown): acynonapyr, azadirachtin, benzoximate, bromopropylate, chinomethionat, cryolite, dicofol, pyridalyl, benclothiaz, sulfur, amidoflumet, 1,3-dichloropropene, DCIP, phenisobromolate, benzomate, metaldehyde, chlorobenzilate, clothiazoben, dicyclanil, fenoxacrim, fentrifanil, flubenzimin, fluphenazine, gossyplure, japonilure, metoxadiazone, petroleum, sodium oleate, tetrasul, triarathene, afidopyropen, flometoquin, fluensulfone, meperfluthrin, tetramethylfluthrin, tralopyril, methylneodecanamide, triflumezopyrim, dicloromezotiaz, oxazosulfyl, tyclopyrazoflor.
(28) Anthelmintic agents:

(a) Benzimidazole-based: fenbendazole, albendazole, triclabendazole, oxibendazole, mebendazole, oxfendazole, parbendazole, flubendazole, febantel, netobimin, thiophanate, thiabendazole, cambendazole;
(b) Salicylanilide-based: closantel, oxyclozanide, rafoxanide, niclosamide;
(c) Substituted phenol-based: nitroxinil, nitroscanate;
(d) Pyrimidine-based: pyrantel, morantel;
(e) Imidazothiazole-based: levamisole, tetramisole;
(f) Tetrahydropyrimidine-based: praziquantel, epsiprantel;
(g) Other anthelmintic agents: cyclodiene, ryania, clorsulon, metronidazole, demiditraz, piperazine, diethylcarbamazine, dichlorophene, monepantel, tribendimidine, amidantel, thiacetarsamide, melarsomine, arsenamide.

Specific examples of fungicides which may be mixed or used with the pest control agent of the present invention are shown below.

(A) Nucleic acid biosynthesis inhibitors

(a) RNA polymerase I inhibitors: benalaxyl, benalaxyl-M, furalaxyl, metalaxyl, metalaxyl-M, oxadixyl, clozylacon, ofurace.
(b) Adenosine deaminase inhibitors: bupirimate, dimethirimol, ethirimol.
(c) DNA/RNA synthesis inhibitors: hymexazol, octhilinone.
(d) DNA topoisomerase II inhibitors: oxolinic acid.

(2) Karyokinesis inhibitors and cell division inhibitors

(a) β-Tubulin polymerization inhibitors: benomyl, carbendazim, fuberidazole, thiabendazole, thiophanate, thiophanate-methyl, diethofencarb, zoxamide, ethaboxam; chlorfenazole, debacarb, trichlaamide, zarilamide.
(b) Cell division inhibitors: pencycuron.
(c) Delocalization inhibitors of spectrin-like protein: fluopicolide, fluopimomide.
(d) Actin / myosin / fimbrin inhibitors: phenamacril, metrafenone, pyriofenone.

(3) Respiration inhibitor:

(a) Complex I NADH oxidation-reduction inhibitor: diflumetorim; tolfenpyrad, fenazaquin;
(b) Complex II succinic acid dehydrogenase inhibitor: benodanil, flutolanil, mepronil, isofetamid, fluopyram,

fenfuram, carboxin, oxycarboxin, thifluzamide, benzovindiflupyr, bixafen, fluxapyroxad, furametpyr, inpyrfluxam, isopyrazam, penflufen, penthiopyrad, sedaxane, isoflucypram, pydiflumetofen, boscalid, pyraziflumid , furmecyclox;

(c) Complex III ubiquinol oxidase Qo inhibitor: azoxystrobin, coumoxystrobin, coumethoxystrobin, enoxastrobin, flufenoxystrobin, picoxystrobin, pyraoxystrobin, mandestrobin, pyraclostrobin, pyrametostrobin, triclopyricarb, kresoxim-methyl, trifloxystrobin, dimoxystrobin, fenaminstrobin, metominostrobin, orysastrobin, famoxadone, fluoxastrobin, fenamidone, pyribencarb, metyltetraprole;

(d) Complex III ubiquinol reductase Qi inhibitor: cyazofamid, amisulbrom;

(e) Oxidative phosphorylation uncoupling agent: binapacryl, meptyldinocap, dinocap, fluazinam, ferimzone;

(f) Oxidative phosphorylation inhibitor (ATP synthase inhibitor): fenthin acetate, fentin chloride, fentin hydroxide;

(g) ATP production inhibitor: silthiofam;

(h) Complex III cytochrome bc1 (ubiquinone reductase) Qx (unknown) inhibitor: ametoctradin.

(4) Amino acid and protein synthesis inhibitor

(a) Methionine biosynthesis inhibitor: cyprodinil, mepanipyrim, pyrimethanil;

(b) Protein synthesis inhibitor: blasticidin-S, kasugamycin, kasugamycin hydrochloride, streptomycin, oxytetracycline.

(5) Signal transfer inhibitor:

(a) Signal transfer inhibitor: quinoxyfen, proquinazid;

(b) MAP/histidine kinase inhibitor in osmotic pressure signal transfer: fenpiconil, fludioxonil, chlozolinate, dimethachlon, iprodione, procymidone, vinclozolin.

(6) Lipid and cell membrane synthesis inhibitor:

(a) Phospholipid biosynthesis and methyltransferase inhibitor: edifenphos, iprobenfos, pyrazophos, isoprothiolane;

(b) Lipid peroxide agent: biphenyl, chloroneb, dichloran, quintozene, tecnazene, tolclofos methyl, etridiazole;

(c) Agents affecting cell membrane: iodocarb, propamocarb, propamocarb hydrochloride, propamocarb-fosetylate, prothiocarb;

(d) Microorganisms disturbing cell membrane of pathogenic bacteria: bacillus subtilis, bacillus subtilis strain QST713, bacillus subtilis strain FZB24, bacillus subtilis strain MBI600, bacillus subtilis strain D747;

(e) Agents disturbing cell membrane: melaleuca alternifolia (tea tree) extract;

(f) Agents affecting ergosterol: natamycin;

(g) Agents affecting lipid homeostasis and transport/storage: oxathiapiprolin, fluoxapiprolin.

(7) Cell membrane sterol biosynthesis inhibitor:

(a) C14 position demethylation inhibitor in sterol biosynthesis: triforine; pyrifenox, pyrisoxazole, fenarimol, nuarimol, imazalil, imazalil-sulphate, oxpoconazole, pefurazoate, prochloraz, triflumizole, azaconazole, bitertanol, bromconazole, cyproconazole, difenoconazole, diniconazole, diniconazole-M, epoxyconazole, etaconazole, fenbuconazole, fluquinconazole, flusilazole, flutriafol, hexaconazole, imibenconazole, ipuconazole, metconazole, myclobutanil, penconazole, propiconazole, simeconazole, tebuconazole, tetraconazole, triadimefon, triadimenol, triticonazole, prothioconazole, furconazole, furconazole-cis, diniconazole-M;

(b) $\Delta$14 reductase and $\Delta 8 \rightarrow \Delta 7$-isomerase inhibitor in sterol biosynthesis: aldimorph, dodemorph, dodemorph-acetate, fenpropimorph, tridemorph, fenpropidine, piperalin, spiroxamine, buthiobate;

(c) 3-Keto reductase inhibitor in C4 position demethylation in sterol biosynthesis system: fenhexamid, fenpyrazamine;

(d) Squalene epoxidase inhibitor in sterol biosynthesis system: pyributicarb, naftifen, terbinafine.

(8) Cell wall synthesis inhibitor

(a) Chitin synthetase inhibitor: polyoxin, polyoxorim;

(b) Cellulose synthetase inhibitor: dimethomorph, flumorph, pyrimorph, benthiavalicarb, iprovalicarb, valifenalate, mandipropamide.

(9) Melanin biosynthesis inhibitor

(a) Reductase inhibitor in melanin biosynthesis: fthalide, pyroquilon, tricyclazole;
(b) Anhydrase inhibitor in melanin biosynthesis: carpropamid, diclocymet, fenoxanil.

(10) Resistance-inducing agents of host plant:

(a) Agents affecting salicylic acid synthetic pathway: acibenzolar-s-methyl;
(b) Others: probenazole, tiadinil, isotianil, laminarin, extract liquid of reynoutria sachalinensis, fosetyl, phosphorus acid and salt, fosetyl·calcium, fosetyl·sodium.

(11) Agents of which the activity is unknown: cymoxanil, tecloftalam, triazoxide, flusulfamide, diclomezine, methasulfocarb, cyflufenamid, dodine, dodine free base, flutianil, tebufloquin, picarbutrazox, validamycin; bethoxazin, cyprofuram, flumetover, nitrothal isopropyl, propamidine; florylpicoxamid, ipflufenoquin, pyridachlometyl, pyrapropoyne, aminopyrifen, dichlobentiazox, ipfentrifluconazole, mefentrifluconazole, quinofumelin, dipymetitrone.
(12) Agent having multiple activities: copper (copper salt), bordeaux mixture, copper hydroxide, copper naphthalate, copper oxide, oxychloride copper, copper sulfate, sulfur, sulfur product, calcium polysulfide, ferbam, manzeb, maneb, metiram, propineb, thiuram, zinc thiazole, zineb, ziram, captan, captafol, folpet, chlorothalonil, dichlofluanid, tolylfluanid, guazatine, iminoctadine triacetate, iminoctadine trialbesilate, anilazine, dithianon, chinomethionat, fluoroimide, metasulfotap , dazomet, curfraneb, mancopper, polycarbamate.
(13) Other agents: DBEDC, fluor folpet, chloropicrin, agrobacterium, diphenylamine, methyl isothiocyanate (MITC), mildew-mycin, capsaicin, cyprosulfamide, difenzoquat, difenzoquat·methyl sulfonate, irmamycin, oxamocarb, puropamocin sodium, pyrrolnitrin, tolnifanide, algophase, amicarthiazol, benthiazole.

Specific examples of plant regulatory agents which can be mixed or used with the pest control agent of the present invention are shown below.
1-Methylcyclopropene, 2,3,5-triiodobenzoic acid, IAA, IBA, MCPA, MCPB, 4-CPA, 5-aminolevulinic acid hydrochloride, 6-benzylaminopurine, abscisic acid, aviglycine hydrochloride, ancymidol, butralin, calcium carbonate, calcium chloride, calcium formate, calcium peroxide, lime sulfur, calcium sulfate, chlormequat chloride, chlorpropham, choline chloride, cloprop, cyanamide, cyclanilide, daminozide, decyl alcohol, dichlorprop, dikegulac, dimethipin, diquat, ethephon, ethychlozate, flumetralin, flurprimidol, forchlorfenuron, gibberellin A, gibberellin A3, hymexazol, inabenfide, isoprothiolane, kinetin, maleic acid hydrazide, mefluidide, mepiquat chloride, oxidation type glutathione, paclobutrazol, pendimethalin, prohexadione calcium, prohydrojasmon, pyraflufen-ethyl, sintofen, sodium 1-naphthalene acetate, sodium cyanate, streptomycin, thidiazuron, triapenthenol, tribufos, trinexapac-ethyl, uniconazole P, and 1-nathtylacetamide.

(Ectoparasite control agent)

An ectoparasite control agent of the present invention contains at least one selected from the aromacyclic quaternary ammonium compounds of the present invention as an active ingredient thereof. The amount of the compound of the present invention contained in the ectoparasite control agent of the present invention is not particularly limited within the range in which ectoparasite control effects are exhibited.
Examples of host animals to be treated with the ectoparasite control agent of the present invention include warm-blooded animals such as: pet animals such as dogs or cats; pet birds; farm animals such as cattle, horses, pigs, and sheep; and poultry. Additional examples thereof include honey-bees, stag beetles, and unicorn beetles.
The ectoparasite control agent of the present invention may be applied by a known veterinary method (topical, oral, parenteral or subcutaneous administration). Examples of the method include: a method in which a tablet, capsule or feed mixed with the ectoparasite control agent is orally administered to the animals; a method in which an immersion liquid, suppository or injection (intramuscular, subcutaneous, intravenous, intraabdominal or the like) is administered to the animals; a method in which an oilbased or aqueous liquid preparation is topically administered by conducting spraying, pouring on, spotting on or the like; and a method in which the ectoparasite control agent is kneaded into a resin, and the resultant kneaded product is molded in an appropriate form such as a collar or an ear tag, and then topically administered to animals.
Ectoparasites parasitize host animals, especially parasitize in or on the body of warm-blooded animals. More specifically, ectoparasites parasitize the back, armpit, underbelly, inner thigh or the like of host animals and obtain nutritional sources such as blood or dandruff from animals to live. Examples of ectoparasites include acarians, lice, fleas, mosquitoes, stable flies, and flesh flies. Specific examples of ectoparasites which can be controlled by the ectoparasite control agent of the present invention are shown below.

(1) Acarians (Acari)

Acarians belonging to the family Dermanyssidae, acarians belonging to the family Macronyssidae, acarians belonging to the family Laelapidae, acarians belonging to the family Varroidae, acarians belonging to the family Argasidae, acarians belonging to the family Ixodidae, acarians belonging to the family Psoroptidae, acarians belonging to the family Sarcoptidae, acarians belonging to the family Knemidokoptidae, acarians belonging to the family Demodixidae, acarians belonging to the family Trombiculidae, and insect-parasitic acari such as Coleopterophagus berlesei.

(2) Order Phthiraptera

Lice belonging to the family Haematopinidae, lice belonging to the family Linognathidae, biting lice belonging to the family Menoponidae, biting lice belonging to the family Philopteridae, and biting lice belonging to the family Trichodectidae.

(3) Order Siphonaptera

Fleas belonging to the family Pulicidae, such as Ctenocephalides canis and Ctenocephalides felis of Ctenocephalides spp.; fleas belonging to the family Tungidae, fleas belonging to the family Ceratophyllidae, and fleas belonging to the family Leptopsyllidae.

(4) Order Hemiptera.

(5) Insect pests of the order Diptera

Mosquitoes belonging to the family Culicidae, black flies belonging to the family Simuliidae, punkie belonging to the family Ceratopogonidae, horseflies belonging to the family Tabanidae, flies belonging to the family Muscidae, tsetse flies belonging to the family Glossinidae, flesh flies belonging to the family Sarcophagidae, flies belonging to the family Hippoboscidae, flies belonging to the family Calliphoridae, and flies belonging to the family Oestridae.

(Endoparasite control agent or expellant)

An endoparasite control agent or expellant of the present invention contains at least one selected from the aromacyclic quaternary ammonium compounds of the present invention as an active ingredient thereof. The amount of the compound of the present invention contained in the endoparasite control agent or expellant of the present invention is not particularly limited within the range in which endoparasite control effects are exhibited.

Parasites to be controlled or expelled by the endoparasite control agent or expellant of the present invention parasitize in host animals, particularly in warm-blooded animals or fish (endoparasite). Examples of the host animals on which the endoparasite control agent or expellant of the present invention is effective include: warm-blooded animals such as humans, domestic mammals (such as cows, horses, pigs, sheep, and goats), laboratory animals (such as mice, rats, and jirds), pet animals (such as hamsters, guinea pigs, dogs, cats, horses, squirrels, rabbits, and ferrets), mammals in nature or zoos (such as monkeys, foxes, deer, and buffalos), poultry (such as turkeys, ducks, chickens, quail, and geese), and pet birds (such as pigeons, parrots, magpies, java sparrows, parakeets, finches, and canaries); and fish such as salmon, trout, and koi carp. It is possible to prevent or treat parasitic diseases mediated by parasites by controlling or expelling the parasites.

Examples of the parasites to be controlled or expelled include the following.

(1) Nematodes of the order Dioctophymatida

(a) Kidney worms belonging to the family Dioctophymatidae, such as Dioctophyma renale of Dioctophyma spp.; and
(b) kidney worms belonging to the family Soboliphymatidae, such as Soboliphyme abei and Soboliphyme baturini of Soboliphyme spp.

(2) Nematodes of the order Trichocephalida

(a) Trichinae belonging to the family Trichinellidae, such as Trichinella spiralis of Trichinella spp.; and
(b) whipworms belonging to the family Trichuridae, such as Capillaria annulata, Capillaria contorta, Capillaria hepatica, Capillaria perforans, Capillaria plica, and Capillaria suis, of Capillaria spp.; and Trichuris vulpis, Trichuris discolor, Trichuris ovis, Trichuris skrjabini, and Trichuris suis, of Trichuris spp.

(3) Nematodes of the order Rhabditida

Strongyloides stercoralis belonging to the family Strongyloididae, such as Strongyloides papillosus, Strongyloides planiceps, Strongyloides ransomi, Strongyloides suis, Strongyloides stercoralis, Strongyloides tumefaciens, and Strongyloides ratti, of Strongyloides spp.

(4) Nematodes of the order Strongylida

Ancylostomas belonging to the family Ancylostomatidae, such as Ancylostoma braziliense, Ancylostoma caninum, Ancylostoma duodenale, and Ancylostoma tubaeforme, of Ancylostoma spp.; Uncinaria stenocephala of Uncinaria spp.; and Bunostomum phlebotomum, and Bunostomum trigonocephalum, of Bunostomum spp.

(5) Nematodes of the order Strongylida

(a) Nematodes belonging to the family Angiostrongylidae, such as Aelurostrongylus abstrusus of Aelurostrongylus spp.; and Angiostrongylus vasorum, and Angiostrongylus cantonesis, of Angiostrongylus spp.;
(b) nematodes belonging to the family Crenosomatidae, such as Crenosoma aerophila, and Crenosoma vulpis, of Crenosoma spp.;
(c) nematodes belonging to the family Filaroididae, such as Filaroides hirthi, and Filaroides osleri, of Filaroides spp.;
(d) metastrongyles belonging to the family Metastrongylidae, such as Metastrongylus apri, Metastrongylus asymmetricus, Metastrongylus pudendotectus, and Metastrongylus salmi, of Metastrongylus spp.; and
(e) gapeworms belonging to the family Syngamidae, such as Cyathostoma bronchialis of Cyathostoma spp.; and Syngamus skrjabinomorpha, and Syngamus trachea, of Syngamus spp.

(6) Nematodes of the order Strongylida

(a) Nematodes belonging to the family Molineidae, such as Nematodirus filicollis, and Nematodirus spathiger, of Nematodirus spp.;
(b) nematodes belonging to the family Dictyocaulidae, such as Dictyocaulus filaria, and Dictyocaulus viviparus, of Dictyocaulus spp.;
(c) nematodes belonging to the family Haemonchidae, such as Haemonchus contortus of Haemonchus spp.; and Mecistocirrus digitatus of Mecistocirrus spp.;
(d) nematodes belonging to the family Haemonchidae, such as Ostertagia ostertagi of Ostertagia spp.;
(e) nematodes belonging to the family Heligmonellidae, such as Nippostrongylus braziliensis of Nippostrongylus spp.; and
(f) nematodes belonging to the family Trichostrongylidae, such as Trichostrongylus axei, Trichostrongylus colubriformis, and Trichostrongylus tenuis, of Trichostrongylus spp.; Hyostrongylus rubidus of Hyostrongylus spp.; and Obeliscoides cuniculi of Obeliscoides spp.

(7) Nematodes of the order Strongylida

(a) Nematodes belonging to the family Chabertiidae, such as Chabertia ovina of Chabertia spp.; and Oesophagostomum brevicaudatum (pig), Oesophagostomum columbianum, Oesophagostomum dentatum, Oesophagostomum georgianum (pig), Oesophagostomum maplestonei, Oesophagostomum quadrispinulatum (pig), Oesophagostomum radiatum, Oesophagostomum venulosum, and Oesophagostomum watanabei (hog), of Oesophagostomum spp.;
(b) nematodes belonging to the family Stephanuridae, such as Stephanurus dentatus of Stephanurus spp.; and

(c) nematodes belonging to the family Strongylidae, such as Strongylus asini, Strongylus edentatus, Strongylus equinus, and Strongylus vulgaris, of Strongylus spp.

(8) Nematodes of the order Oxyurida

Nematodes belonging to the family Oxyuridae, such as Enterobius anthropopitheci, and Enterobius vermicularis, of Enterobius spp.; Oxyuris equi of Oxyuris spp.; and Passalurus ambiguus of Passalurus spp.

(9) Nematodes of the order Ascaridida

(a) Nematodes belonging to the family Ascaridiidae, such as Ascaridia galli of Ascaridia spp.;
(b) nematodes belonging to the family Heterakidae, such as Heterakis beramporia, Heterakis brevispiculum, Heterakis gallinarum, Heterakis pusilla, and Heterakis putaustralis, of Heterakis spp.;
(c) nematodes belonging to the family Anisakidae, such as Anisakis simplex of Anisakis spp.;
(d) nematodes belonging to the family Ascarididae, such as Ascaris lumbricoides, and Ascaris suum, of Ascaris spp.; and Parascaris equorum of Parascaris spp.; and
(e) nematodes belonging to the family Toxocaridae, such as Toxocara canis, Toxocara leonina, Toxocara suum, Toxocara vitulorum, and Toxocara cati, of Toxocara spp.

(10) Nematodes of the order Spirurida

(a) Nematodes belonging to the family Onchocercidae, such as Brugia malayi, Brugia pahangi, and Brugia patei, of Brugia spp.; Dipetalonema reconditum of Dipetalonema spp.; Dirofilaria immitis of Dirofilaria spp.; Filaria oculi of Filaria spp.; and Onchocerca cervicalis, Onchocerca gibsoni, and Onchocerca gutturosa, of Onchocerca spp.;
(b) nematodes belonging to the family Setariidae, such as Setaria digitata, Setaria equina, Setaria labiatopapillosa, and Setaria marshalli, of Setaria spp.; and Wuchereria bancrofti of Wuchereria spp.; and
(c) nematodes belonging to the family Filariidae, such as Parafilaria multipapillosa of Parafilaria spp.; and Stephanofilaria assamensis, Stephanofilaria dedoesi, Stephanofilaria kaeli, Stephanofilaria okinawaensis, and Stephanofilaria stilesi of Stephanofilaria spp.

(11) Nematodes of the Order Spirurida

(a) Nematodes belonging to the family Gnathostomatidae, such as Gnathostoma doloresi, and Gnathostoma spinigerum, of Gnathostoma spp.;
(b) nematodes belonging to the family Habronematidae, such as Habronema majus, Habronema microstoma, and Habronema muscae, of Habronema spp.; and Draschia megastoma of Draschia spp.;
(c) nematodes belonging to the family Physalopteridae, such as Physaloptera canis, Physaloptera cesticillata, Physaloptera erdocyona, Physaloptera felidis, Physaloptera gemina, Physaloptera papilloradiata, Physaloptera praeputialis, Physaloptera pseudopraerutialis, Physaloptera rara, Physaloptera sibirica, and Physaloptera vulpineus, of Physaloptera spp.;
(d) nematodes belonging to the family Gongylonematidae, such as Gongylonema pulchrum of Gongylonema spp.;
(e) nematodes belonging to the family Spirocercidae, such as Ascarops strongylina of Ascarops spp.; and
(f) nematodes belonging to the family Thelaziidae, such as Thelazia callipaeda, Thelazia gulosa, Thelazia lacrymalis, Thelazia rhodesi, and Thelazia skrjabini, of Thelazia spp.

(Control agent against other pests)

In addition, the compound of the present invention exhibits an excellent effect of controlling insect pests that have a sting or venom that can harm humans or livestock, insect pests carrying various pathogens / pathogenic bacteria, or insect pests that impart a discomforting sensation to humans (such as toxic insect pests, sanitary insect pests, or unpleasant insect pests).
Specific examples thereof are shown below.

(1) Insect pests of the order Hymenoptera

Sawflies belonging to the family Argidae, wasps belonging to the family Cynipidae, sawflies belonging to the family Diprionidae, ants belonging to the family Formicidae, wasps belonging to the family Mutillidae, and wasps belonging to the family Vespidae.

(2) Other insect pests

Blattodea, termite, araneae, cetipede, millipede, crustacea and cimex lectularius.

(Preparation formulation)

Although some preparation formulations of the pest control agent, insecticide, acaricide, ectoparasite control agent, endoparasite control agent or expellant of the present invention are shown below, additives and addition amounts thereof are not limited to these examples, and may be varied in a wide range. In the preparation formulation, the term "part" indicates "parts by mass" and the term "%" indicates "% by mass".

The agricultural, horticultural or paddy preparation formulations are shown below.

(Formulation 1: wettable powders)

40 parts of a compound of the present invention, 53 parts of diatomaceous earth, 4 parts of higher alcohol sulfuric acid ester, and 3 parts of alkyl naphthalene sulfonate are mixed uniformly, and then finely pulverized to obtain wettable powders containing 40% of the active ingredient.

(Formulation 2: emulsion)

30 parts of a compound of the present invention, 33 parts of xylene, 30 parts of dimethylformamide, and 7 parts of polyoxyethylene alkyl allyl ether are mixed and dissolved to obtain an emulsion containing 30% of the active ingredient.

(Formulation 3: granules)

5 parts of a compound of the present invention, 40 parts of talc, 38 parts of clay, 10 parts of bentonite, and 7 parts of sodium alkyl sulfate are mixed uniformly, and then finely pulverized, followed by conducting granulation to obtain a particle diameter of 0.5 to 1.0 mm, and thus granules containing 5% of the active ingredient are obtained.

(Formulation 4: granules)

5 parts of a compound of the present invention, 73 parts of clay, 20 parts of bentonite, 1 part of sodium dioctyl sulfosuccinate, and 1 part of potassium phosphate are mixed well and then pulverized, followed by adding water thereto, and then kneading the mixture. Then, granulation and drying are carried out to obtain granules containing 5% of the active ingredient.

(Formulation 5: suspension)

10 parts of a compound of the present invention, 4 parts of polyoxyethylene alkyl allyl ether, 2 parts of sodium polycarboxylate, 10 parts of glycerin, 0.2 parts of xanthan gum, and 73.8 parts of water are mixed, and then wet-pulverized until the particle size becomes 3 $\mu$m or less to obtain a suspension containing 10% of the active ingredient.

Preparation formulations of ectoparasite control agents, endoparasite control agents or expellants are shown below.

(Formulation 6: granules)

5 parts of a compound of the present invention is dissolved in an organic solvent to obtain a solution. The solution is sprayed on a mixture of 94 parts of kaolin and 1 part of white carbon, followed by evaporating the solvent under reduced pressure to obtain granules. The granules may be mixed with animal feed to be used.

(Formulation 7: injection agent)

0.1 to 1 part of a compound of the present invention and 99 to 99.9 parts of peanut oil are mixed uniformly, and then filter-sterilized using a sterilizing filter.

(Formulation 8: pour-on agent)

5 parts of a compound of the present invention, 10 parts of a myristic acid ester and 85 parts of isopropanol are mixed uniformly to obtain a pour-on agent.

(Formulation 9: spot-on agent)

10 to 15 parts of a compound of the present invention, 10 parts of a palmitic acid ester and 75 to 80 parts of isopropanol are mixed uniformly to obtain a spot-on agent.

(Formulation 10: spray agent)

1 part of a compound of the present invention, 10 parts of propylene glycol and 89 parts of isopropanol are mixed uniformly to obtain a spray agent.

The present invention is explained further specifically by showing examples below. The present invention is not limited to the following examples.

Example 1

Synthesis of (2-chloro-6-(4-((trifluoromethyl)thio)phenoxy)isonicotinoyl) (pyridine-1-ium-1-yl)amide [English name: (2-chloro-6-(4-((trifluoromethyl)thio) phenoxy)isonicotinoyl)(pyridin-1-ium-1-yl)amide]

(Step 1)

Synthesis of t-butyl-2-chloro-6-(4-((trifluoromethyl)thio)phenoxy)isonicotinate [English name: tert-butyl-2-chloro-6-(4-((trifluoromethyl)thio)phenoxy) isonicotinate]

[Chemical Formula 9]

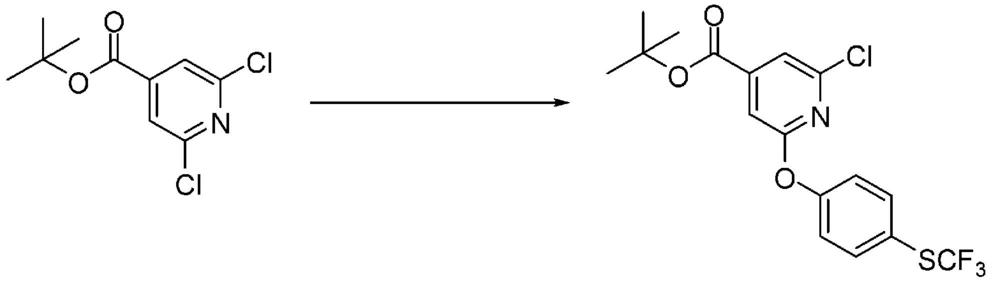

t-Butyl-2,6-dichloroisonicotinate (1.5 g) and 4-((trifluoromethyl)thio)phenol (1.3 g) were dissolved in DMF (18 ml), and potassium carbonate (1.3 g) was added thereto, followed by heating and stirring the mixture at 60°C overnight.

After the reaction was ended, water was added to the resultant, and the mixture was extracted with ethyl acetate, and washed with saturated saline. Then, an organic phase was dried over anhydrous magnesium sulfate, and filtered, and then the filtrate was condensed under reduced pressure. The obtained concentrate was purified by silica-gel chromatography to obtain 2.1 g of the above-identified compound at a yield of 86%.

(Step 2)

Synthesis of 2-chloro-6-(4-((trifluoromethyl)thio)phenoxy)isonicotinic acid [English name: 2-chloro-6-(4-((trifluoromethyl)thio)phenoxy)isonicotinic acid]

[Chemical Formula 10]

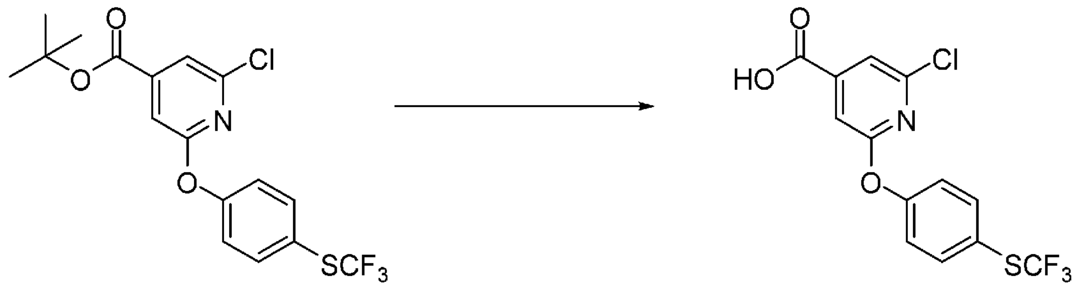

t-Butyl-2-chloro-6-(4-((trifluoromethyl)thio)phenoxy)isonicotinate (2.1 g) was dissolved in dichloromethane (10 ml), and trifluoroacetic acid (5 ml) was added thereto, followed by stirring the mixture at room temperature overnight.
After the reaction was ended, the solvent was condensed under reduced pressure, hexane was added to the obtained crystal, and then the resultant was filtered and dried to obtain 1.49 g of the above-identified compound at a yield of 82%.

(Step 3)

Synthesis of (2-chloro-6-(4-((trifluoromethyl)thio)phenoxy)isonicotinoyl) (pyridin-1-ium-1-yl)amide [English name: (2-chloro-6-(4-((trifluoromethyl)thio)phenoxy) isonicotinoyl)(pyridin-1-ium-1-yl)amide]

[Chemical Formula 11]

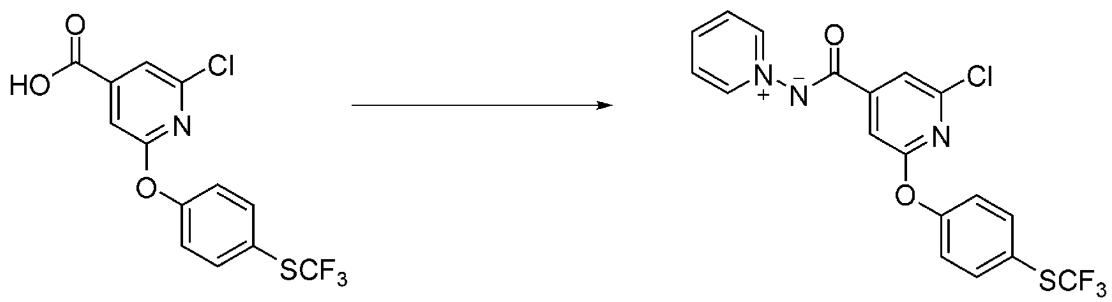

2-Chloro-6-(4-((trifluoromethyl)thio)phenoxy)isonicotinic acid (0.17 g) and 1-aminopyridinium iodide (0.14 g) were dissolved in DMF (2 ml), and then 1-ethyl-3-(3-dimethylaminopropyl) carbodiimide hydrochloride (0.14 g), 1-hydroxybenzotriazole (0.07 g) and triethylamine (0.1 g) were added thereto, followed by stirring the mixture at room temperature overnight.
After the reaction was ended, the residue was purified by reverse-phase chromatography to obtain 0.136 g of the above-identified compound (compound No. 25) at a yield of 64%.

Example 2

Synthesis of (2-chloro-6-(4-(trifluoromethoxy)phenoxy)isonicotinoyl) (1-ethyl-1H-1,2,4-triazol-4-ium-4-yl)amide [English name: (2-chloro-6-(4-(trifluoromethoxy)phenoxy)isonicotinoyl)(1-ethyl-1H-1,2,4-triazol-4-ium-4-yl)amide]

(Step 1)

Synthesis of tertiary butyl 2-chloro-6-(4-(trifluoromethoxy)phenoxy) isonicotinate [English name: tert-butyl 2-chloro-6-(4-(trifluoromethoxy)phenoxy) isonicotinate]

[Chemical Formula 12]

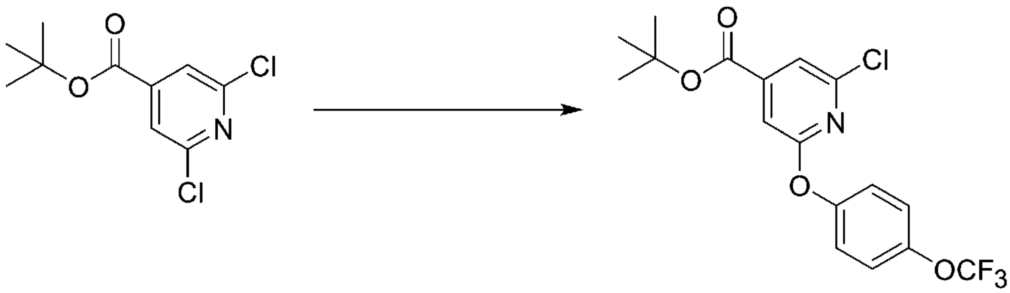

Tertiary butyl 2,6-dichloroisonicotinate (1.5 g) and 4-(trifluoromethoxy)phenol (1.3 g) were dissolved in DMF (18 ml), and then potassium carbonate (1.3 g) was added thereto, followed by heating and stirring the mixture at 60°C overnight. After the reaction was ended, water was added to the resultant, and then the mixture was extracted with ethyl acetate, and washed with saturated saline. Then, an organic phase was dried over anhydrous magnesium sulfate, and then filtered, followed by condensing the filtrate under reduced pressure. The obtained concentrate was purified by silica-gel chromatography to obtain 1.9 g of the above-identified compound at a yield of 86%.

(Step 2)

Synthesis of (2-chloro-6-(4-(trifluoromethoxy)phenoxy)isonicotinoyl) (1-ethyl-1H-1,2,4-triazol-4-ium-4-yl)amide [English name: (2-chloro-6-(4-(trifluoromethoxy)phenoxy)isonicotinoyl)(1-ethyl-1H-1,2,4-triazol-4-ium-4-yl)amide]

[Chemical Formula 13]

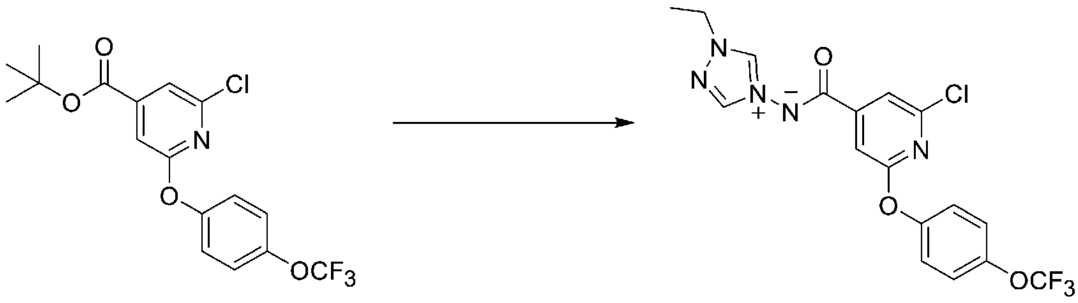

Tertiary butyl 2-chloro-6-(4-(trifluoromethoxy)phenoxy)isonicotinate (0.18 g) was dissolved in dichloromethane (10 ml), and then trifluoroacetic acid (5 ml) was added thereto, followed by stirring the mixture at room temperature overnight. After the reaction was ended, the solvent was condensed under reduced pressure, and then the obtained crystal was dissolved in dichloromethane (10 ml). 1-Aminopyridinium iodide (0.14 g), 1-ethyl-3-(3-dimethylaminopropyl) carbodiimide hydrochloride (0.14 g), 1-hydroxybenzotriazole (0.07 g), and triethylamine (0.15 ml) were added to the solution, followed by stirring the mixture at room temperature overnight. After the reaction was ended, the residue was purified by reverse-phase chromatography to obtain 0.12 g of the above-identified compound (compound No. 165) at a yield of 58%.

Example 3

Synthesis of (1-ethyl-1H-1,2,4-triazol-4-ium-4-yl)(2-(4-(perfluoroethoxy)phenoxy)-6-(2,2,2-trifluoroethoxy)isonicotinoyl) amide [English name: (1-ethyl-1H-1,2,4-triazol-4-ium-4-yl)(2-(4-(perfluoroethoxy)phenoxy)-6-(2,2,2-trifluoroethoxy)isonicotinoyl)amide]

(Step 1)

Synthesis of tertiary butyl 2-chloro-6-(4-(perfluoroethoxy)phenoxy)isonicotinate [English name: tert-butyl 2-chloro-6-(4-(perfluoroethoxy)phenoxy)isonicotinate]

[Chemical Formula 14]

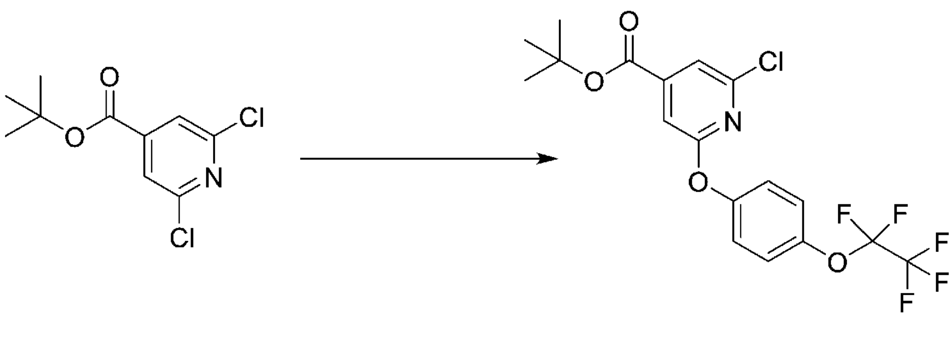

Tertiary butyl 2,6-dichloroisonicotinate (3.0 g) and 4-(perfluoroethoxy)phenol (2.8 g) were dissolved in DMF (36 ml), and then potassium carbonate (3.0 g) was added thereto, followed by heating and stirring the mixture at 60°C overnight. After the reaction was ended, water was added to the resultant, and then the mixture was extracted with ethyl acetate, and then washed with saturated saline. Thereafter, an organic phase was dried over anhydrous magnesium sulfate and filtered, followed by condensing the filtrate under reduced pressure. The obtained concentrate was purified by silica-gel chromatography to obtain 3.9 g of the above-identified compound at a yield of 89%.

(Step 2)

Synthesis of tertiary butyl 2-(4-(perfluoroethoxy)phenoxy)-6-(2,2,2-trifluoroethoxy)isonicotinate [English name: tert-butyl 2-(4-(perfluoroethoxy)phenoxy)-6-(2,2,2-trifluoroethoxy)isonicotinate]

[Chemical Formula 15]

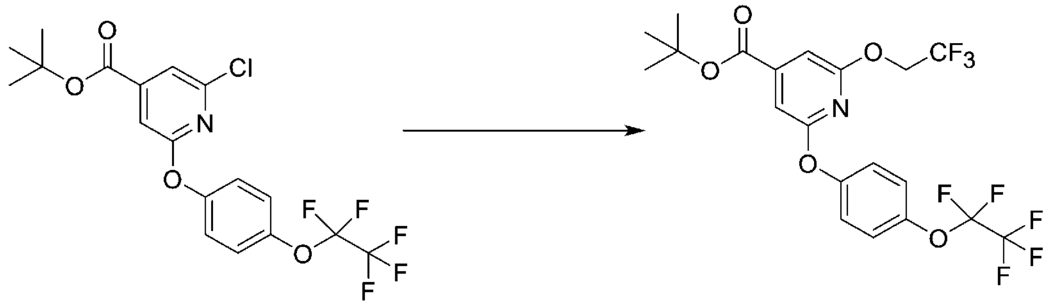

Tertiary butyl 2-chloro-6-(4-(perfluoroethoxy)phenoxy)isonicotinate (0.15 g) and trifluoroethanol (0.11 g) were dissolved in toluene (2 ml). 2-Di-tert-butylphosphino-2',4',6'-triisopropylbiphenyl (0.04 g), tris(dibenzylideneacetone) dipalladium (0) (0.07 g), and cesium carbonate (0.36 g) were added to the solution, and then heated and stirred at 100°C for one hour. After the reaction was ended, water was added to the resultant, and then the mixture was extracted with ethyl acetate, and then washed with saturated saline. Thereafter, an organic phase was dried over anhydrous magnesium sulfate, and then filtered, followed by condensing the filtrate under reduced pressure. The obtained concentrate was purified by silica-gel chromatography to obtain 0.05 g of the above-identified compound at a yield of 36%.

(Step 3)

Synthesis of (1-ethyl-1H-1,2,4-triazol-4-ium-4-yl)(2-(4-(perfluoroethoxy)phenoxy)-6-(2,2,2-trifluoroethoxy)isonicotinoyl) amide [English name: (1-ethyl-1H-1,2,4-triazol-4-ium-4-yl)(2-(4-(perfluoroethoxy)phenoxy)-6-(2,2,2-trifluoroethoxy)isonicotinoyl)amide]

[Chemical Formula 16]

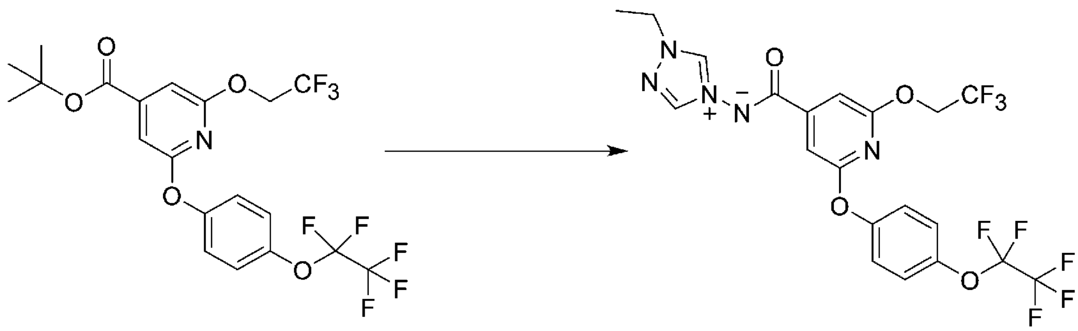

Tertiary butyl 2-(4-(perfluoroethoxy)phenoxy)-6-(2,2,2-trifluoroethoxy)isonicotinate (0.05 g) was dissolved in dichloromethane (2 ml), and then trifluoroacetic acid (0.5 ml) was added thereto, followed by stirring the mixture at room temperature overnight. After the reaction was ended, the solvent was condensed under reduced pressure, and then the obtained crystal was dissolved in dichloromethane (2 ml). 1-aminopyridinium iodide (0.04 g), 1-ethyl-3-(3-dimethylaminopropyl)carbodiimide hydrochloride (0.05 g), 1-hydroxybenzotriazole (0.01 g) and triethylamine (0.07 ml) were added to the solution, followed by stirring the mixture at room temperature overnight. After the reaction was ended, the residue was purified by reverse-phase chromatography to obtain 0.02 g of the above-identified compound (compound No. 179) at a yield of 37%.

Example 4

Synthesis of (2-cyclopropyl-6-(4-(perfluoroethoxy)phenoxy)isonicotinoyl)(pyridin-1-ium-1-yl)amide [English name: (2-cyclopropyl-6-(4-(perfluoroethoxy)phenoxy)isonicotinoyl)(pyridin-1-ium-1-yl)amide]

(Step 1)

Synthesis of tertiary butyl 2-cyclopropyl-6-(4-(perfluoroethoxy)phenoxy)isonicotinate [English name: tert-butyl 2-cyclopropyl-6-(4-(perfluoroethoxy)phenoxy)isonicotinate]

[Chemical Formula 17]

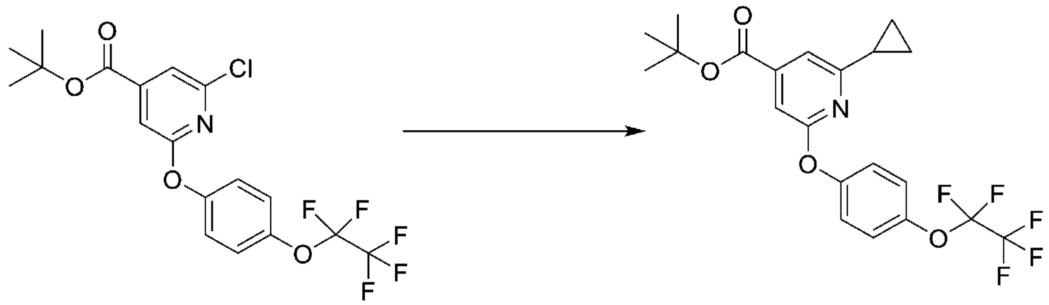

Tertiary butyl 2-chloro-6-(4-(perfluoroethoxy)phenoxy)isonicotinate (0.3 g) was dissolved in propionitrile (3 ml) and water (0.5 ml). Cyclopropylboronic acid (0.18 g), potassium phosphate (0.43 g) and bis(di-tert-butyl(4-dimethylaminophenyl)phosphine)dichloropalladium (II) (0.043 g) were added to the solution, followed by heating and stirring the mixture at 100°C for two hours. After the reaction was ended, water was added to the resultant, and the mixture was extracted with ethyl acetate, and then washed with saturated saline. Thereafter, an organic phase was dried over anhydrous magnesium sulfate and filtered, followed by condensing the filtrate under reduced pressure. The obtained concentrate was purified by silica-gel chromatography to obtain 0.2 g of the above-identified compound at a yield of 70%.

(Step 2)

Synthesis of (2-cyclopropyl-6-(4-(perfluoroethoxy)phenoxy)isonicotinoyl)(pyridin-1-ium-1-yl)amide [English name: (2-cyclopropyl-6-(4-(perfluoroethoxy)phenoxy)isonicotinoyl)(pyridin-1-ium-1-yl)amide]

[Chemical Formula 18]

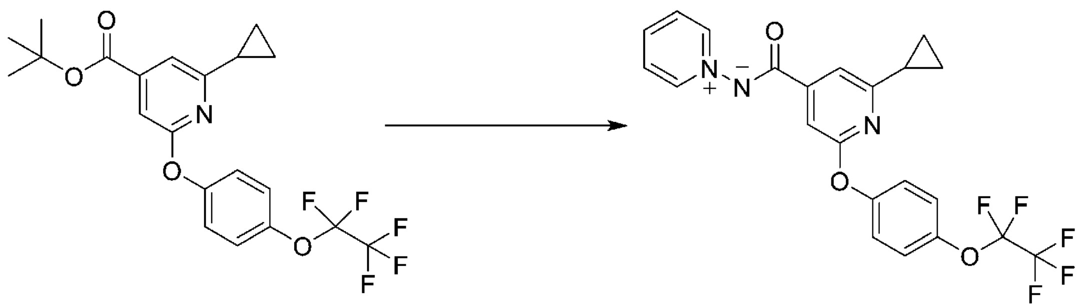

Trtiary butyl 2-cyclopropyl-6-(4-(perfluoroethoxy)phenoxy)isonicotinate (0.2 g) was dissolved in dichloromethane (4 ml), and trifluoroacetic acid (0.7 ml) was added thereto, followed by stirring the mixture at room temperature overnight. After the reaction was ended, the solvent was condensed under reduced pressure, and the obtained crystal was dissolved in dichloromethane (2 ml). 1-Aminopyridinium iodide (0.1 g), 1-ethyl-3-(3-dimethylaminopropyl)carbodiimide hydrochloride (0.1 g), 1-hydroxybenzotriazole (0.005 g) and triethylamine (0.07m l ) were added to the solution, followed by stirring the mixture at room temperature overnight. After the reaction was ended, the residue was purified by reverse-phase chromatography to obtain 0.052 g of the above-identified compound (compound No. 215) at a yield of 40%.

Example 5

Synthesis of (2-iodo-6-(4-(perfluoroethoxy)phenoxy)isonicotinoyl)(pyridin-1-ium-1-yl)amide [English name: (2-iodo-6-(4-(perfluoroethoxy)phenoxy)isonicotinoyl) (pyridin-1-ium-1-yl)amide]

(Step 1)

Synthesis of 2-iodo-6-(4-(perfluoroethoxy)phenoxy)isonicotinic acid [English name: 2-iodo-6-(4-(perfluoroethoxy)phenoxy)isonicotinic acid]

[Chemical Formula 19]

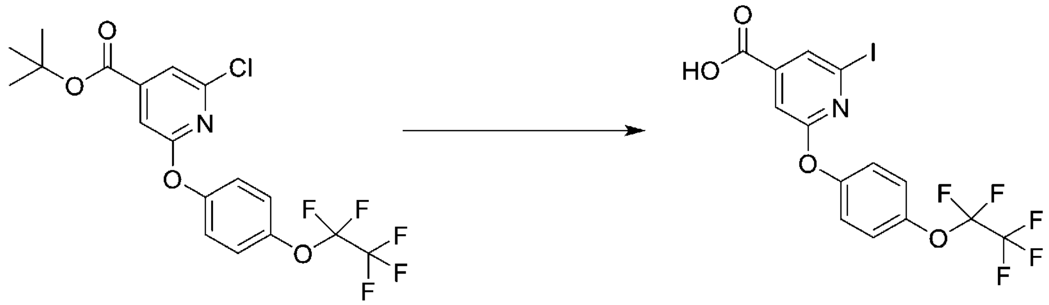

Tertiary butyl 2-chloro-6-(4-(perfluoroethoxy)phenoxy)isonicotinate (0.25 g) was dissolved in 1,4-dioxane (3 ml). Chlorotrimethylsilane (0.22 ml) and sodium iodide (0.85 g) were added to the solution, and the mixture was heated and stirred at 100°C for four hours. After the reaction was ended, water was added to the resultant, and the mixture was extracted with ethyl acetate and washed with saturated saline. Thereafter, an organic phase was dried over anhydrous magnesium sulfate and filtered, followed by condensing the filtrate under reduced pressure. The obtained concentrate was purified by reverse-phase chromatography to obtain 0.06 g of the above-identified compound at a yield of 20%.

(Step 2)

Synthesis of (2-iodo-6-(4-(perfluoroethoxy)phenoxy)isonicotinoyl)(pyridin-1-ium-1-yl)amide [English name: (2-iodo-6-(4-(perfluoroethoxy)phenoxy)isonicotinoyl) (pyridin-1-ium-1-yl)amide]

[Chemical Formula 20]

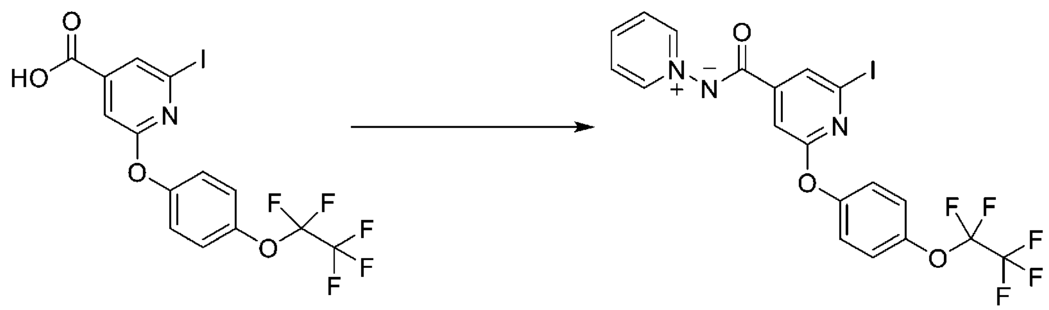

2-Iodo-6-(4-(perfluoroethoxy)phenoxy)isonicotinic acid (0.02 g) was dissolved in dichloromethane (2 ml). 1-Aminopyridinium iodide (0.02 g), 1-ethyl-3-(3-dimethylaminopropyl)carbodiimide hydrochloride (0.02 g), 1-hydroxybenzotriazole (0.005 g) and triethylamine (0.03 ml) were added to the solution, followed by stirring the mixture at room

temperature overnight. After the reaction was ended, the residue was purified by reverse-phase chromatography to obtain 0.011 g of the above-identified compound (compound No. 219) at a yield of 50%.

Example 6

Synthesis of (2-chloro-6-(4-(trifluoromethoxy)phenoxy)isonicotinoyl)(pyridin-1-ium-1-yl)amide [English name: (2-chloro-6-(4-(trifluoromethoxy)phenoxy) isonicotinoyl)(pyridin-1-ium-1-yl)amide]

(Step 1)

Synthesis of (2,6-dichloroisonicotinoyl)(pyridin-1-ium-1-yl)amide [English name: (2,6-dichloroisonicotinoyl)(pyridin-1-ium-1-yl)amide]

[Chemical Formula 21]

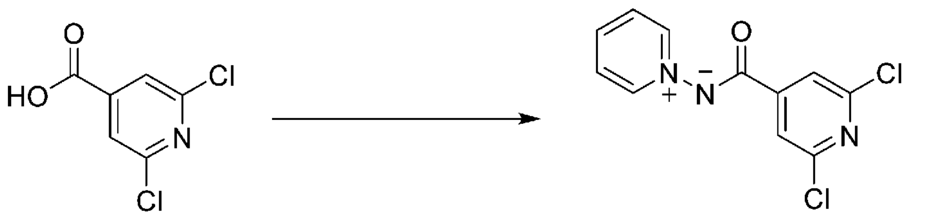

2,6-Dichloroisonicotinic acid (10.48 g) and 1-aminopyridinium iodide (14.5 g) were dissolved in dichloromethane (181 ml), and then 1-ethyl-3-(3-dimethylaminopropyl)carbodiimide hydrochloride (15.7 g), 1-hydroxybenzotriazole (0.636 g) and triethylamine (15 ml) were added thereto, followed by heating and stirring the mixture at room temperature overnight. After the reaction was ended, water was added to the resultant, and the mixture was extracted with dichloromethane/methanol = 9: 1, and washed with saturated saline. Thereafter, an organic phase was dried over anhydrous magnesium sulfate and filtered, followed by condensing the filtrate under reduced pressure. The obtained concentrate was washed with hexane to obtain 10.4 g of the above-identified compound at a yield of 71%.

(Step 2)

Synthesis of (2-chloro-6-(4-(trifluoromethoxy)phenoxy)isonicotinoyl)(pyridin-1-ium-1-yl)amide [English name: (2-chloro-6-(4-(trifluoromethoxy)phenoxy) isonicotinoyl)(pyridin-1-ium-1-yl)amide]

[Chemical Formula 22]

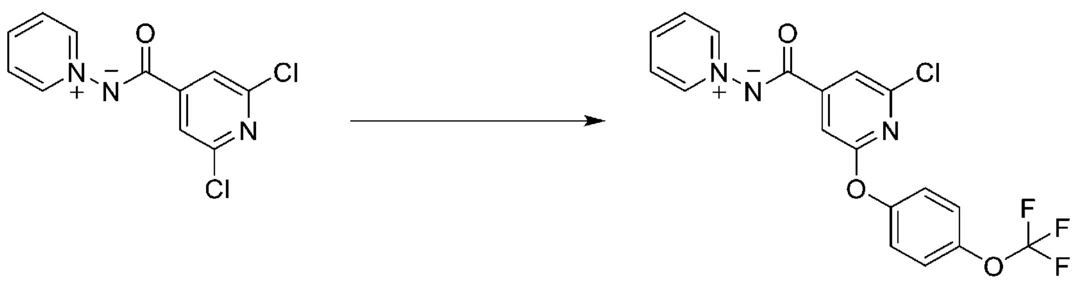

(2,6-Dichloroisonicotinoyl)(pyridin-1-ium-1-yl)amide (2.0 g) was dissolved in DMF (25 ml), and 4-trifluoromethoxyphenol (1.6 g), cesium carbonate (4.9 g) and triethylamine (1.5 g) were added thereto, followed by stirring the mixture at 100°C overnight. After the reaction was ended, water was added to the resultant, and the mixture was extracted with dichloromethane/methanol = 9:1, and washed with saturated saline. Thereafter, an organic phase was dried over anhydrous magnesium sulfate and filtered, followed by condensing the filtrate under reduced pressure. The obtained

concentrate was purified by silica-gel chromatography to obtain 2.14 g of the above-identified compound (compound No. 175) at a yield of 81%.

Some examples of the compound of the present invention prepared in a similar manner to that of the above-mentioned example are shown in Table 1. The data of the physical properties of the compound are indicated in the column "physical properties". The melting point (m.p.: °C) and the retention time measured by the liquid chromatography-mass spectrometry (LCMS) using the below-mentioned method are described as the physical properties.

The retention time of the compound 16 by LCMS was measured by the following method.

The measurement was carried out with a system using a HPLC system (Waters Alliance e2695 separation module, manufactured by Waters Corporation), a single quadrupole MS detector (LC/MS) (SQ Detector 2 (PDA detection was carried out in an atmospheric pressure ionization mode), manufactured by Waters Corporation) and a HPLC/UHPLC detector (2998 PDA Photodiode Array Detector (manufactured by Waters Corporation)), a Xbridge BEH C18 column (manufactured by Waters Corporation, 2.1×50 mm, 2.5 μm) being attached to the system, at a temperature of 40°C, a flow rate of 0.4mL/minute, and an injection amount of 6 μL. Water containing 0.1% formic acid was used as the mobile phase (A), and acetonitrile was used as the mobile phase (B). The mobile phase (A) and the mobile phase (B) were passed through the column at the below-mentioned concentration and a flow rate of 0.4 mL/minute, followed by measuring the retention time (minute) of a sample at a sample injection amount of 6 μL. The concentration of the mobile phase (B) was maintained at 30% by mass for 0.3 minutes at an inlet of the column, and then increased from 30% by mass to 95% by mass at a constant velocity over 1.7 minutes, followed by maintaining the concentration at 95% by mass for 1.5 minutes, immediately decreasing the concentration of the mobile phase (B) to 30% by mass, and then finally maintaining the concentration of the mobile phase (B) at 30% by mass for 2.5 minutes.

The retention times of the compounds 17, 20, 22, 24, 31, 36, 45, 46, 62, 65, 74, 84, 87, 95, 117, 127, 133, 135, 136, 142 and 146 by LCMS were measured by the following method.

Waters CORTECS UPLC C18 column (manufactured by Waters Corporation, 2.1×50 mm, 1.6 μm) was used under the conditions in which the temperature was set at 40°C, the flow rate was set at 0.6 mL/minute, the injection amount was set at 2 μL, water containing 0.1% formic acid was used as the mobile phase (A), and acetonitrile was used as the mobile phase (B), and the retention time was count by minute. Migration was carried out using ACQUITY UPLC H-Class (manufactured by Waters Corporation) with ACQUITY UPLC photodiode array (PDA) eλ detector (manufactured by Waters Corporation) and ACQUITY QDa detector (manufactured by Waters Corporation, in which UV PDA detection was carried out in positive and negative ion electrospray modes). The concentration of the mobile phase (B) was maintained at 30% by mass for 0.15 minutes, and then increased in a linear gradient from 30% by mass to 95% by mass for 1.35 minutes, followed by maintaining the concentration at 95% by mass for 0.5 minutes (II), immediately decreasing the concentration to 30% by mass (III), and then maintaining the concentration at 30% by mass for 0.50 minutes (IV).

The retention times of the compounds 33, 38, 39, 41, 42, 49, 51, 55, 57, 60, 61, 98, 101, 103, 108, 109, 112, 121, 122, 124, 125, 126, 131, 148, 183, 186, 210, 211 and 212 by LCMS were measured by the following method.

The measurement was carried out with a system using an ultra high performance liquid chromatography (ACQUITY UPLC H-Class: manufactured by Waters Corporation), a HPLC/UHPLC detector (ACQUITY UPLC photodiode array (PDA) eλ detector (manufactured by Waters Corporation)) and a single quadrupole MS detector (LC/MS) (SQ Detector 2 detector (positive and negative ion electrospray modes and an atmospheric pressure ionization mode), a CORTECS UPLC C18 column (manufactured by Waters Corporation, 2.1×50 mm, 1.6 μm) being attached to the system, at a temperature of 40°C, a flow rate of 0.6 mL/minute, and an injection amount of 2 μL. Water containing 0.1% formic acid was used as the mobile phase (A), and acetonitrile was used as the mobile phase (B). The concentration of the mobile phase (B) was maintained at 30% by mass for 0.15 minutes, and then increased from 30% by mass to 95% by mass at a constant velocity over 1.35 minutes, followed by maintaining the concentration at 95% by mass for 0.5 minutes, immediately decreasing the concentration of the mobile phase (B) to 30% by mass, and then finally maintaining the concentration of the mobile phase (B) at 30% by mass for 0.50 minutes.

[Table 1]

| No. | Chemical formula | Physical properties |
|---|---|---|
| 1 | 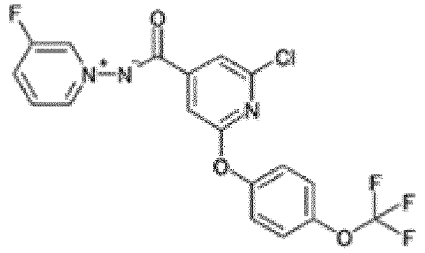 | m. p. 146-148 |
| 2 | 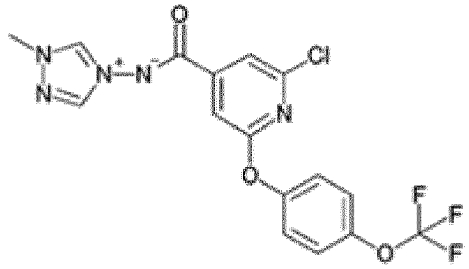 | m. p. 159-161 |
| 3 | 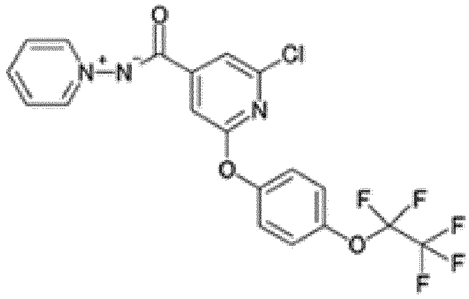 | m. p. 142-144 |
| 4 | 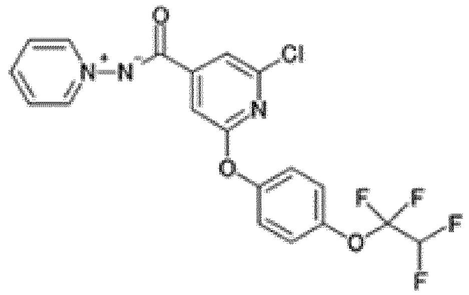 | m. p. 152-154 |

[Table 2]

Table 1 (continued)

| No. | Chemical formula | Physical properties |
|---|---|---|
| 5 | 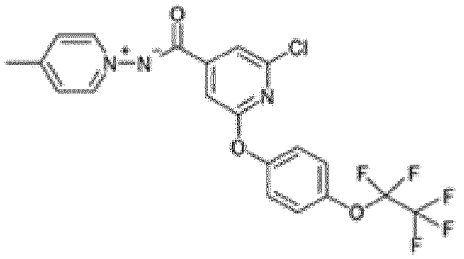 | m. p. 166-168 |
| 6 | 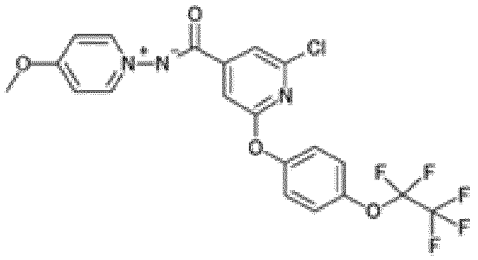 | m. p. 142-144 |
| 7 | 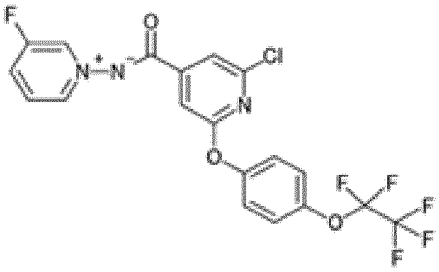 | m. p. 170-172 |
| 8 | 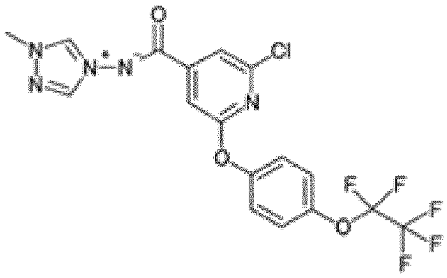 | m. p. 210-212 |

[Table3]

Table 1 (continued)

| No. | Chemical formula | Physical properties |
|---|---|---|
| 9 | 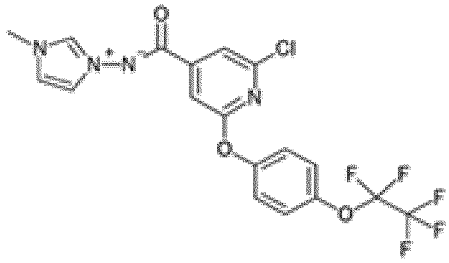 | m. p. 142-144 |
| 10 | 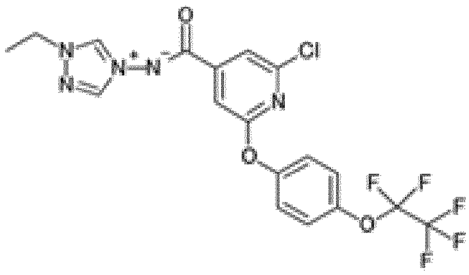 | m. p. 122-124 |
| 11 | 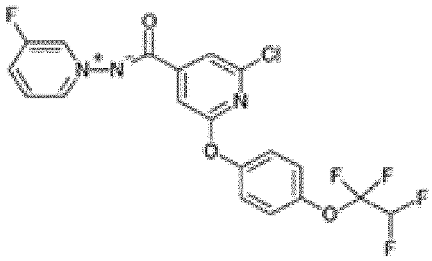 | m. p. 135-137 |
| 12 | 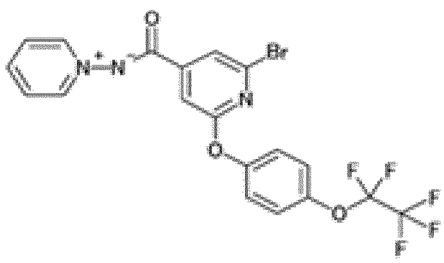 | m. p. 150-152 |

[Table 4]

# Table 1 (continued)

| No. | Chemical formula | Physical properties |
|---|---|---|
| 13 | 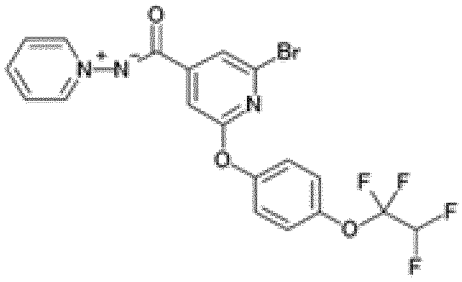 | m. p. 150-152 |
| 14 | 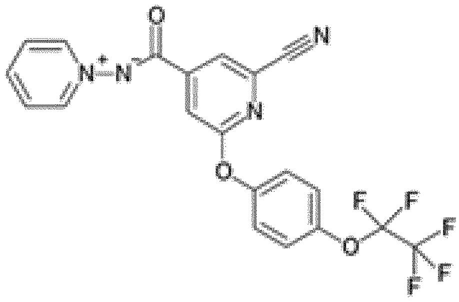 | m. p. 136-138 |
| 15 | 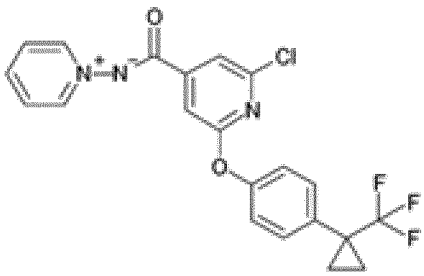 | m. p. 146-148 |
| 16 | 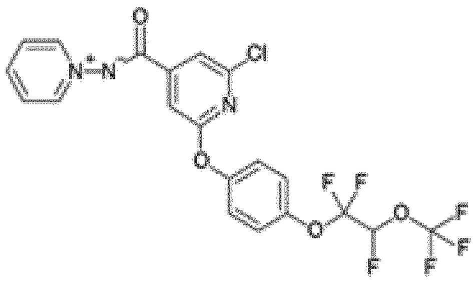 | VISCOUS OIL<br>LCMS retention time: 3.86 min. |

[Table 5]

Table 1 (continued)

| No. | Chemical formula | Physical properties |
|---|---|---|
| 17 | 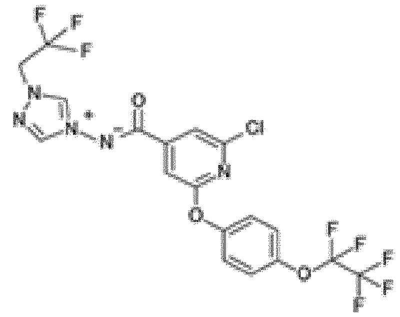 | VISCOUS OIL<br>LCMS retention time: 1.77 min |
| 18 | 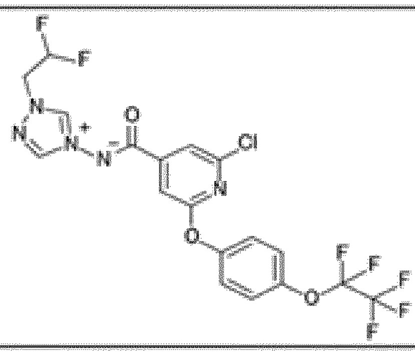 | m.p. 129-131 |
| 19 | 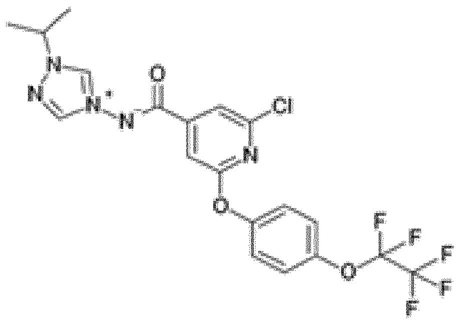 | m.p. 152-154 |
| 20 | 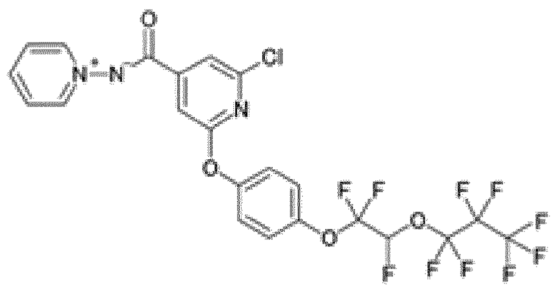 | AMORPHOUS<br>LCMS retention time: 1.76 min |

[Table 6]

## Table 1 (continued)

| No. | Chemical formula | Physical properties |
|---|---|---|
| 21 | 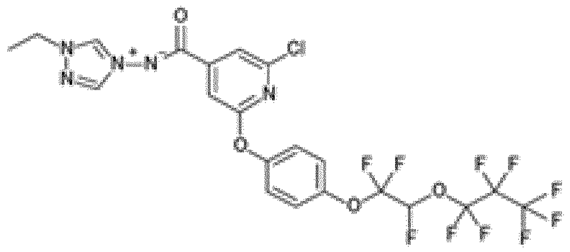 | m. p. 115-117 |
| 22 | 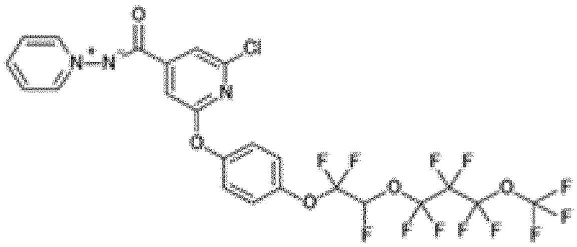 | AMORPHOUS<br>LCMS retention time: 1.87 min. |
| 23 | 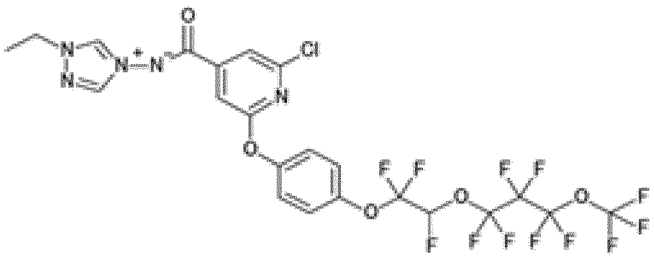 | m. p. 116-118 |
| 24 | 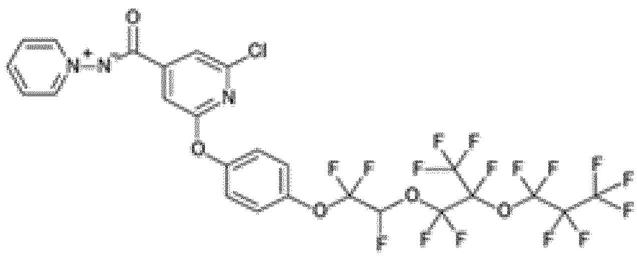 | AMORPHOUS<br>LCMS retention time: 2.03 min |
| 25 | 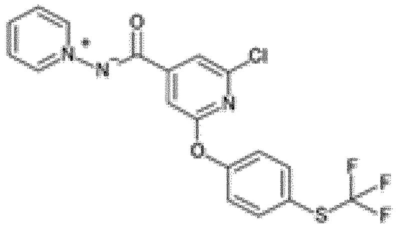 | m. p. 119-121 |

[Table 7]

Table 1 (continued)

| No. | Chemical formula | Physical properties |
|---|---|---|
| 26 | 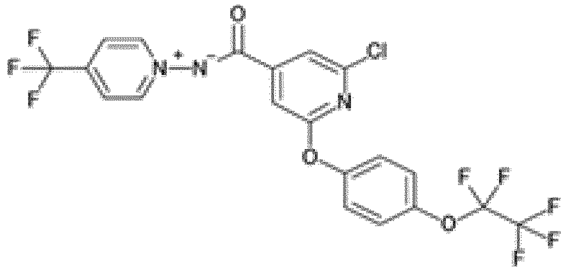 | m. p. 160-162 |
| 27 | 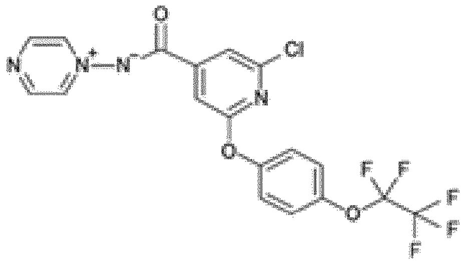 | m. p. 153-155 |
| 28 | 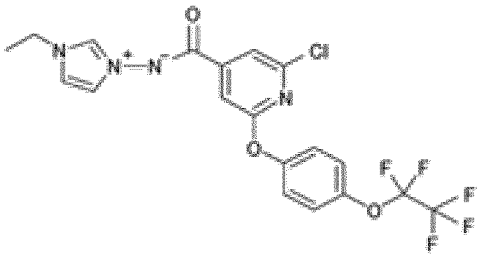 | m. p. 106-108 |
| 29 | 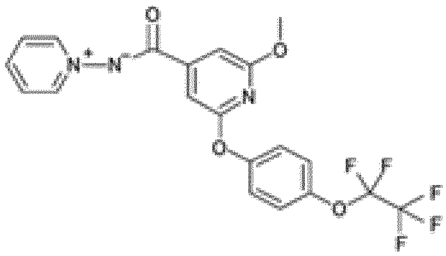 | m. p. 134-136 |

[Table 8]

Table 1 (continued)

| No. | Chemical formula | Physical properties |
|---|---|---|
| 30 | 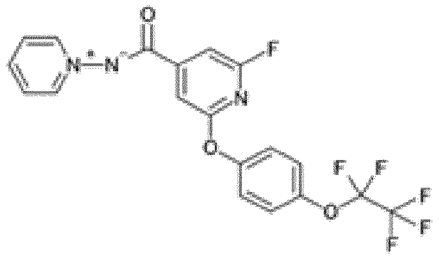 | m. p. 127-129 |
| 31 | 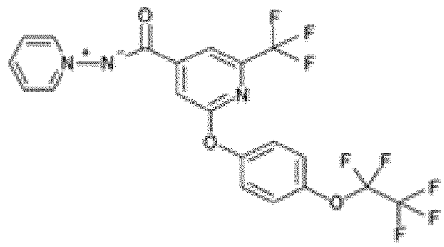 | AMORPHOUS<br>LCMS retention time: 1.70 min. |
| 32 | 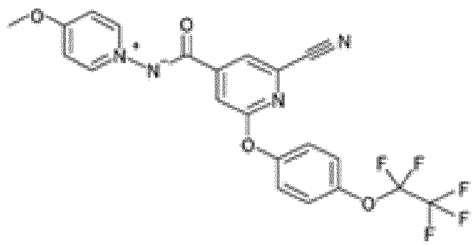 | m. p. 192-194 |
| 33 | 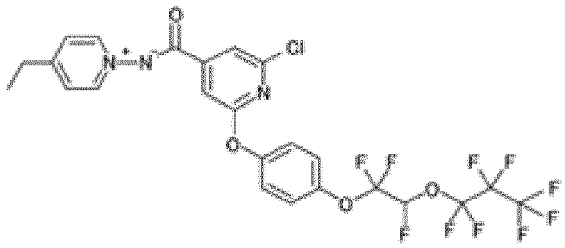 | AMORPHOUS<br>LCMS retention time: 1.87 min. |
| 34 | 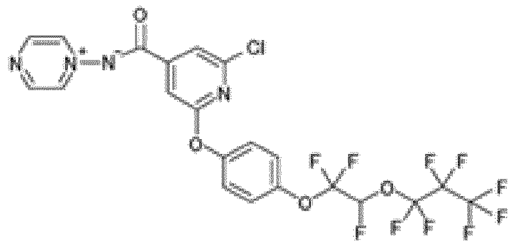 | m. p. 65-67 |

[Table 9]

Table 1 (continued)

| No. | Chemical formula | Physical properties |
|---|---|---|
| 35 | 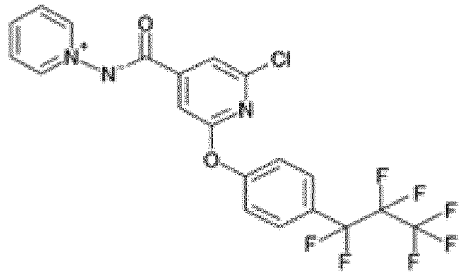 | m.p. 129-131 |
| 36 | 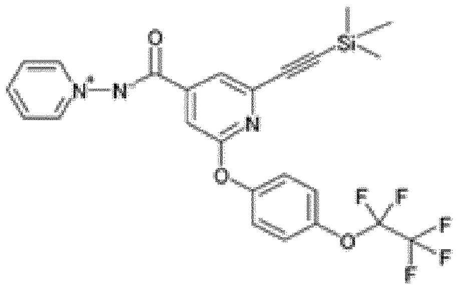 | LCMS retention time: 1.87 min. |

[Table 10]

# Table 1 (continued)

| No. | Chemical formula | Physical properties |
|---|---|---|
| 37 | 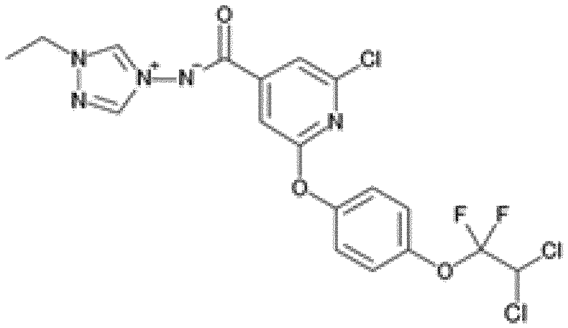 | m. p. 116-118 |
| 38 | 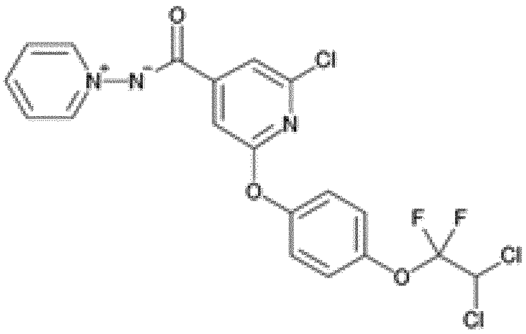 | VISCOUS OIL<br>LCMS retention time: 1.62 min. |
| 39 | 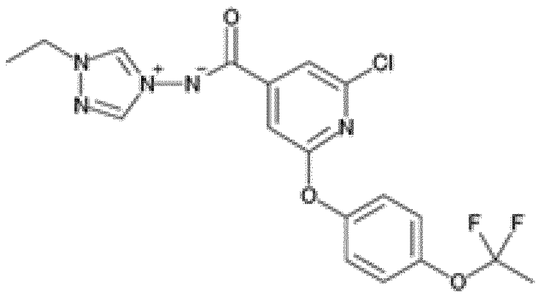 | VISCOUS OIL<br>LCMS retention time: 1.43 min. |
| 40 | 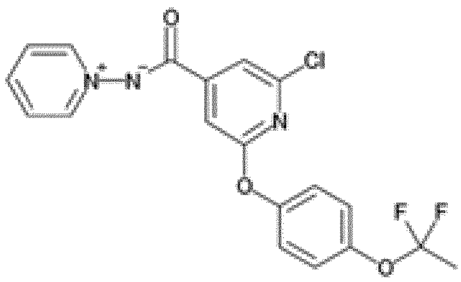 | m. p. 121-123 |

[Table 11]

Table 1 (continued)

| No. | Chemical formula | Physical properties |
|---|---|---|
| 41 | 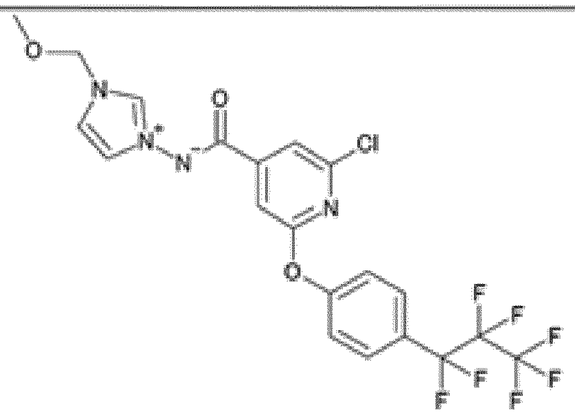 | VISCOUS OIL<br>LCMS retention time 1.66 min. |
| 42 | 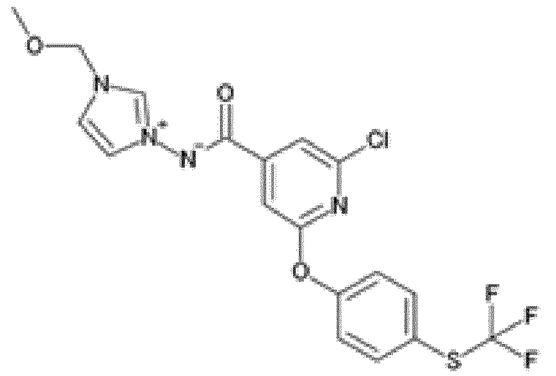 | VISCOUS OIL<br>LCMS retention time 1.53 min. |
| 43 | 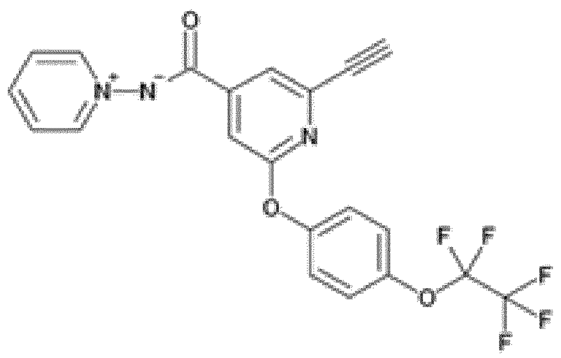 | m.p. 134-136 |

[Table 12]

# Table 1 (continued)

<table>
<tr><th>No.</th><th>Chemical formula</th><th>Physical properties</th></tr>
<tr><td>44</td><td>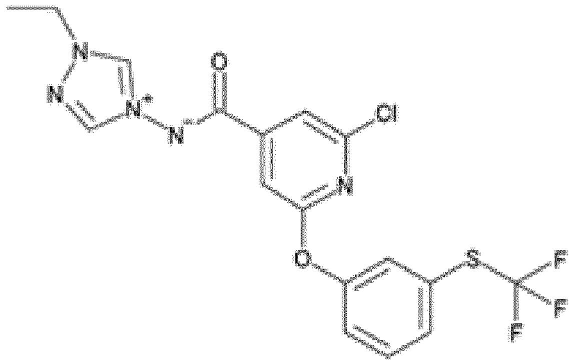</td><td>m. p. 134-137</td></tr>
<tr><td rowspan="2">45</td><td rowspan="2">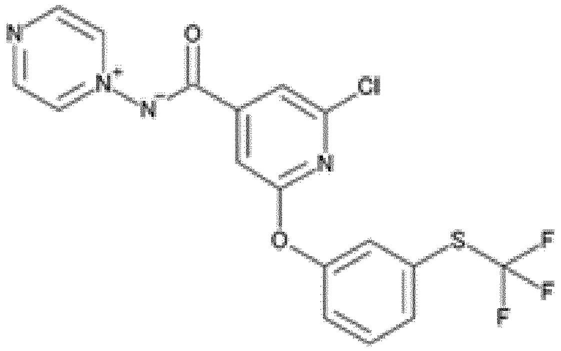</td><td>VISCOUS OIL</td></tr>
<tr><td>LCMS retention time 1.62 min.</td></tr>
<tr><td rowspan="2">46</td><td rowspan="2">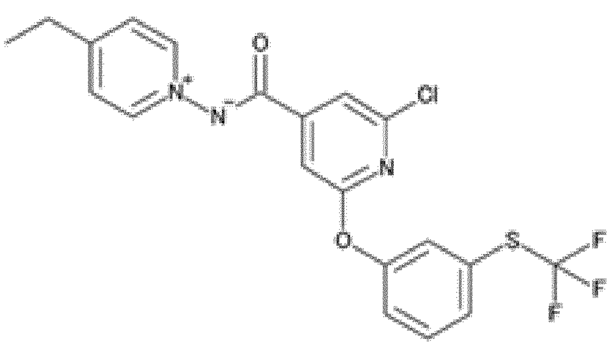</td><td>VISCOUS OIL</td></tr>
<tr><td>LCMS retention time 1.67 min.</td></tr>
<tr><td>47</td><td>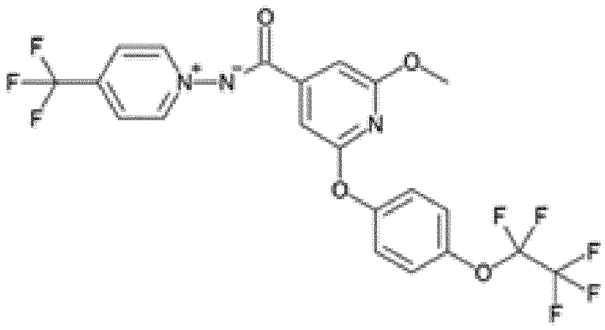</td><td>m. p. 158-160</td></tr>
</table>

[Table 13]

Table 1 (continued)

| No. | Chemical formula | Physical properties |
|---|---|---|
| 48 | 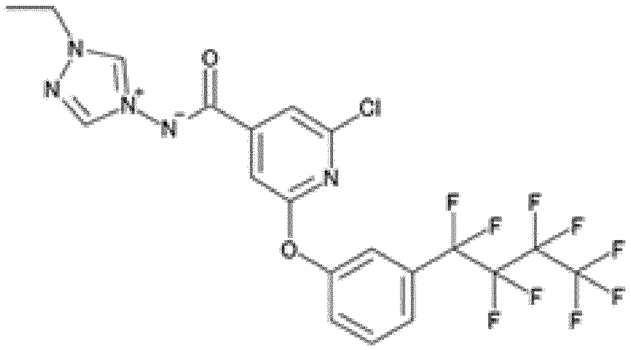 | m.p. 119-121 |
| 49 | 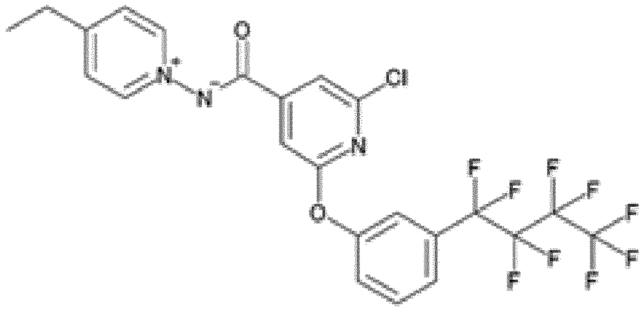 | VISCOUS OIL<br>LCMS retention time 1.90 min. |
| 50 | 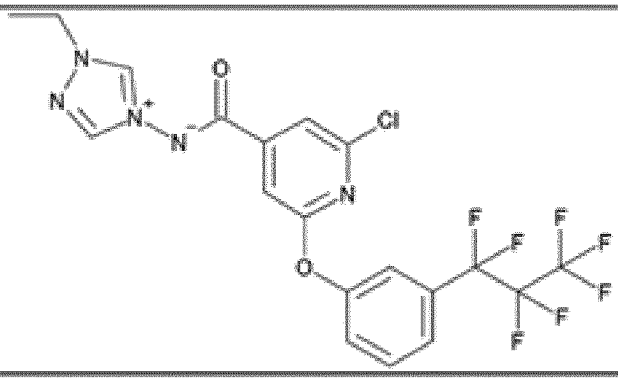 | m.p. 138-139 |
| 51 | 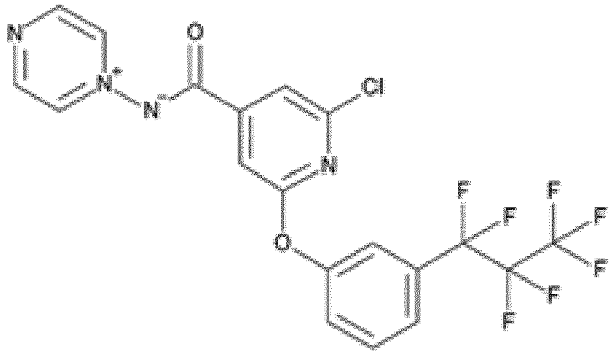 | VISCOUS OIL<br>LCMS retention time 1.80 min. |

[Table 14]

Table 1 (continued)

| No. | Chemical formula | Physical properties |
| --- | --- | --- |
| 52 | 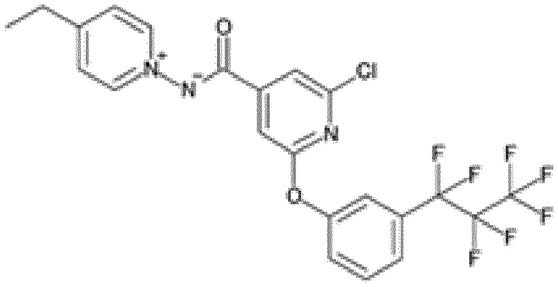 | m. p. 97-100 |
| 53 | 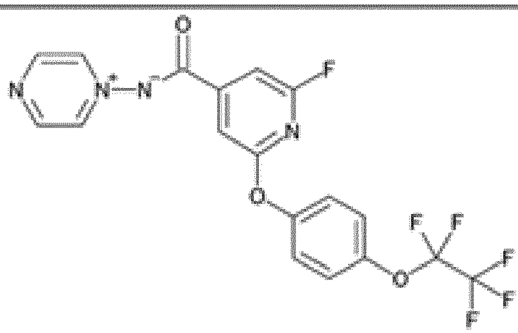 | m. p. 106-108 |
| 54 | 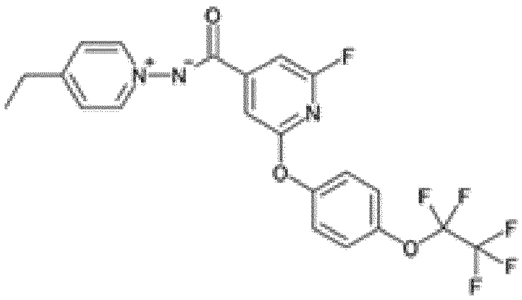 | m. p. 168-170 |
| 55 | 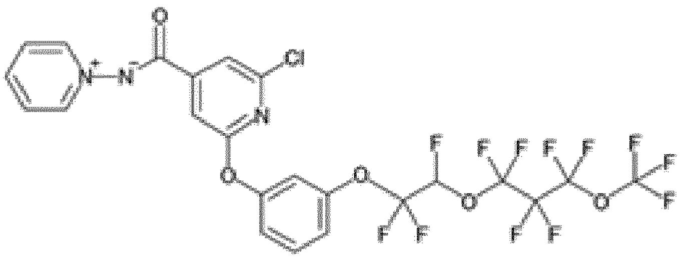 | VISCOUS OIL<br>LCMS retention time 1.95 min. |

[Table 15]

Table 1 (continued)

| No. | Chemical formula | Physical properties |
|---|---|---|
| 56 | 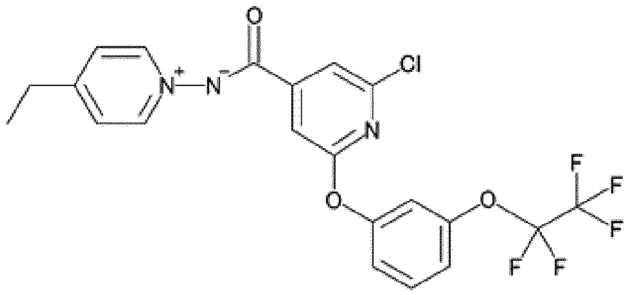 | m. p. 84-86 |
| 57 | 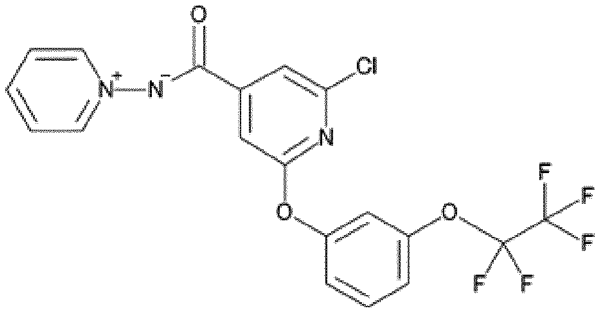 | VISCOUS OIL<br>LCMS retention time 1.69 min |
| 58 | 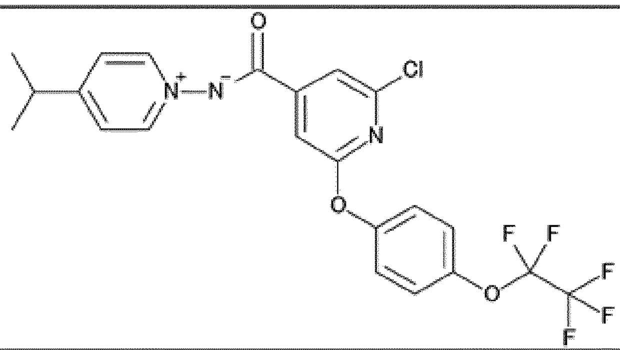 | m. p. 122-142 |
| 59 | 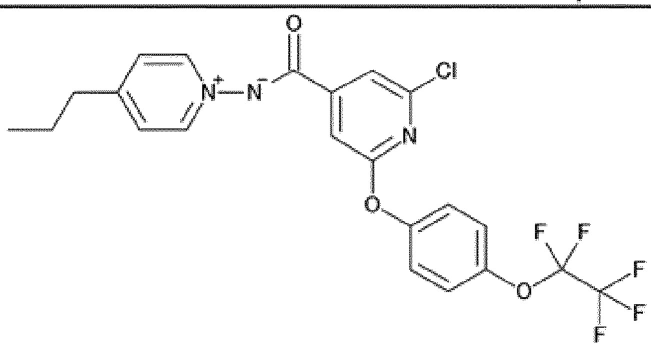 | m. p. 140-142 |

[Table 16]

Table 1 (continued)

| No. | Chemical formula | Physical properties |
|---|---|---|
| 60 | 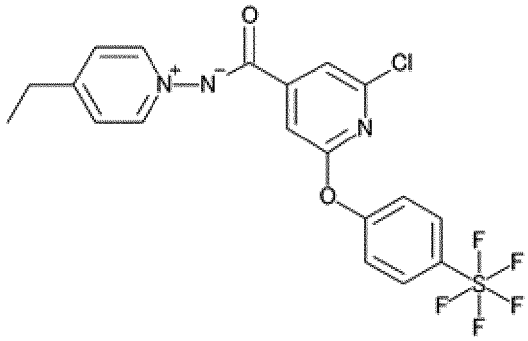 | VISCOUS OIL<br>LCMS retention time 1.65 min |
| 61 | 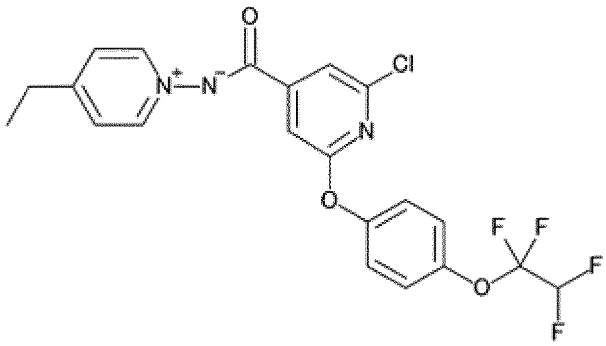 | VISCOUS OIL<br>LCMS retention time 1.55 min |
| 62 | 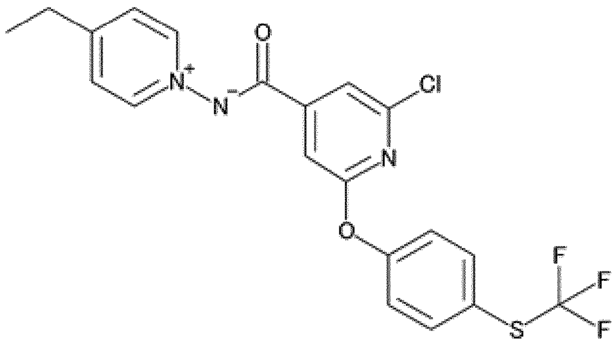 | VISCOUS OIL<br>LCMS retention time 1.71 min |
| 63 | 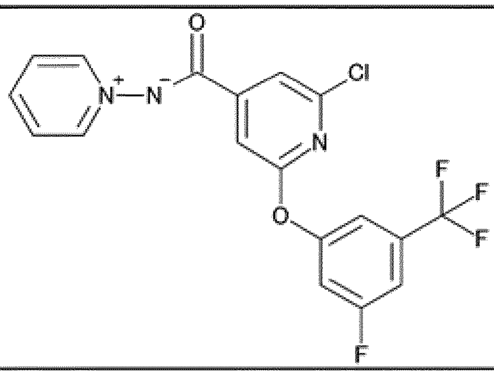 | m. p. 165-167 |

[Table 17]

Table 1 (continued)

| No. | Chemical formula | Physical properties |
|---|---|---|
| 64 | 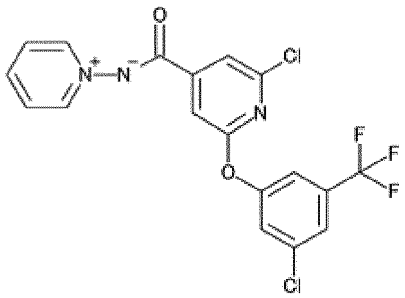 | m.p. 175-177 |
| 65 | 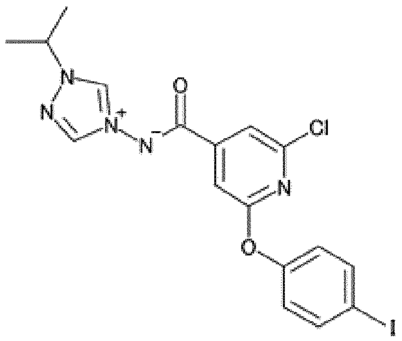 | VISCOUS OIL<br>LCMS retention time 1.58 min |
| 66 | 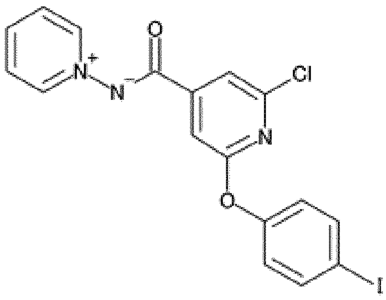 | m.p. 180-182 |
| 67 | 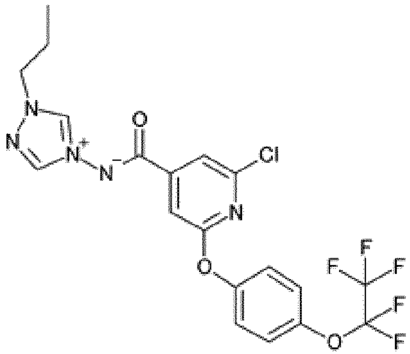 | m.p. 119-120 |

[Table 18]

Table 1 (continued)

| No. | Chemical formula | Physical properties |
|---|---|---|
| 68 | 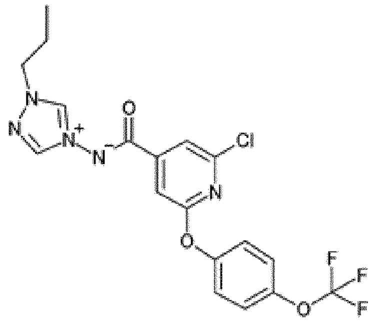 | m.p. 95-97 |
| 69 | 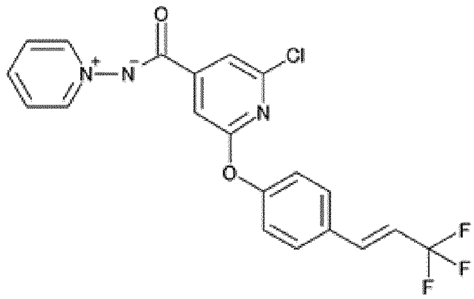 | m.p. 188-190 |
| 70 | 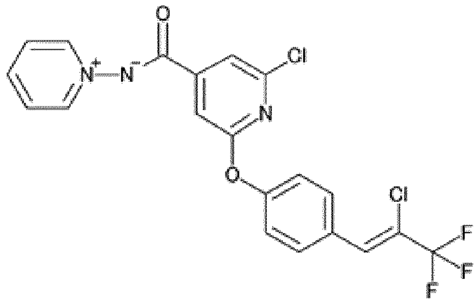 | m.p. 135-137 |
| 71 | 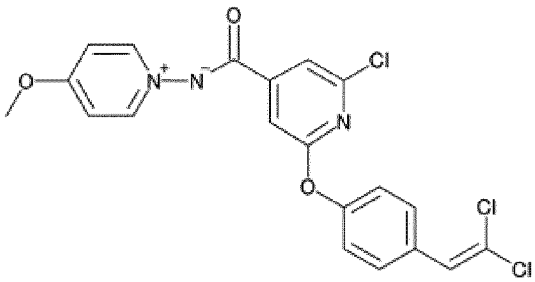 | m.p. 190-192 |

[Table 19]

Table 1 (continued)

| No. | Chemical formula | Physical properties |
|---|---|---|
| 72 | 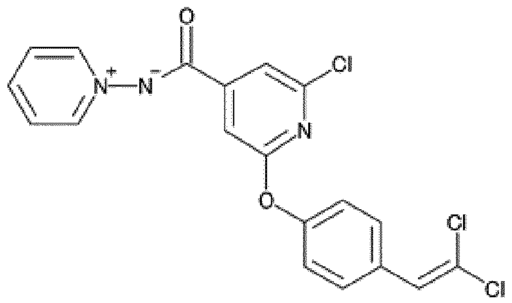 | m.p. 170-172 |
| 73 | 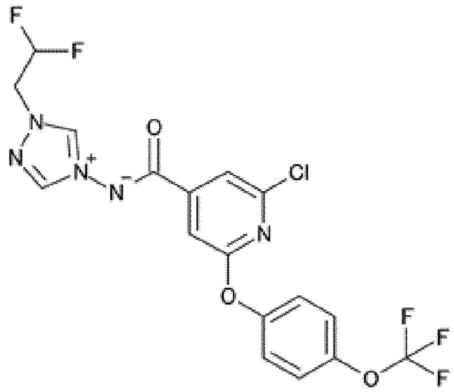 | m.p. 99-102 |
| 74 | 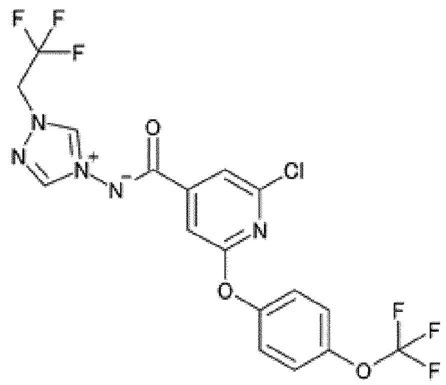 | VISCOUS OIL<br>LCMS retention time 1.64 min. |

[Table 20]

Table 1 (continued)

| No. | Chemical formula | Physical properties |
|---|---|---|
| 75 | 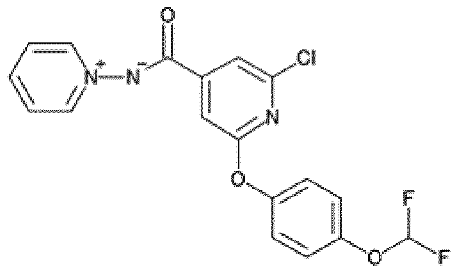 | m. p. 122-124 |
| 76 | 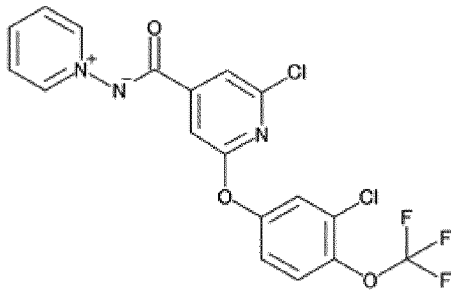 | m. p. 126-129 |
| 77 | 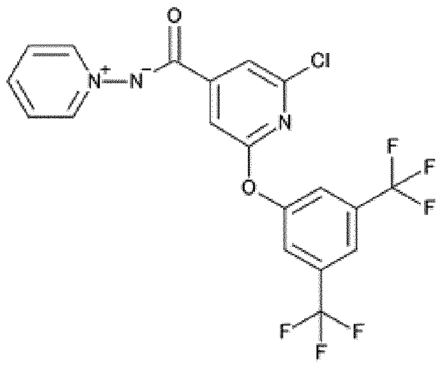 | m. p. 160-162 |
| 78 | 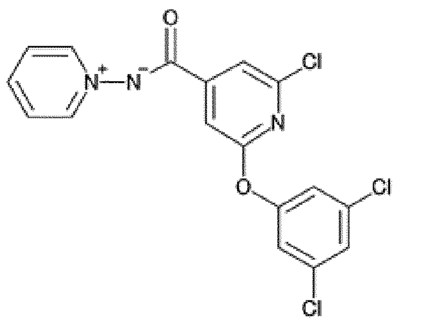 | m. p. 193-195 |

[Table 21]

Table 1 (continued)

| No. | Chemical formula | Physical properties |
|---|---|---|
| 79 | 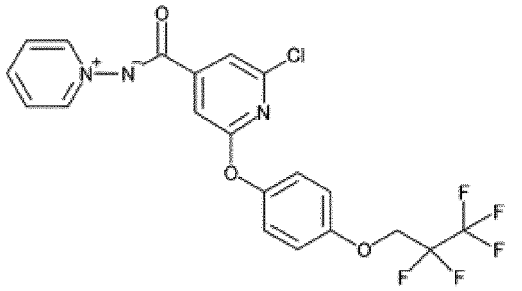 | m. p. 136-138 |
| 80 | 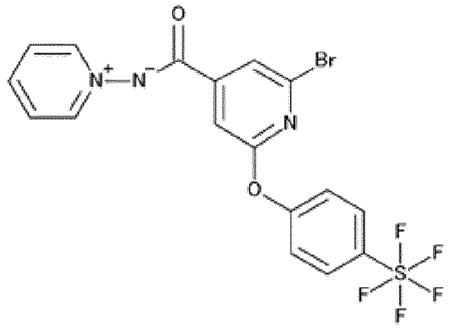 | m. p. 160-162 |
| 81 | 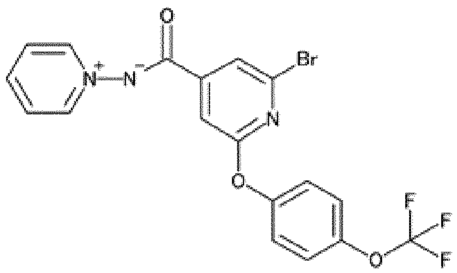 | m. p. 168-170 |
| 82 | 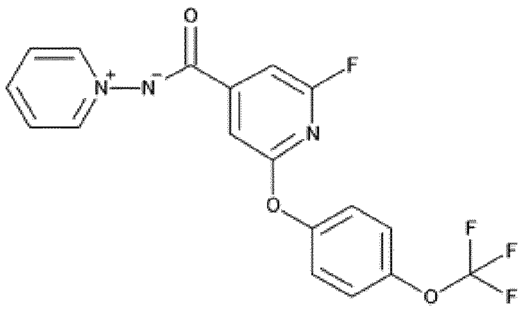 | m. p. 130-132 |

[Table 22]

Table 1 (continued)

| No. | Chemical formula | Physical properties |
|---|---|---|
| 83 | 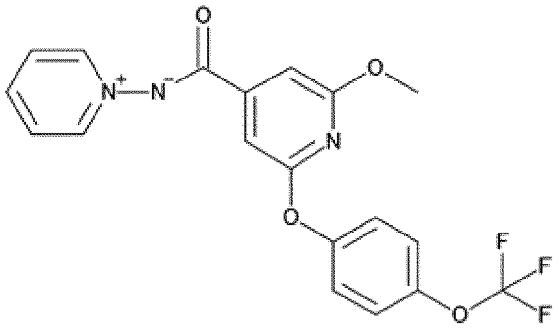 | m. p. 133-135 |
| 84 | 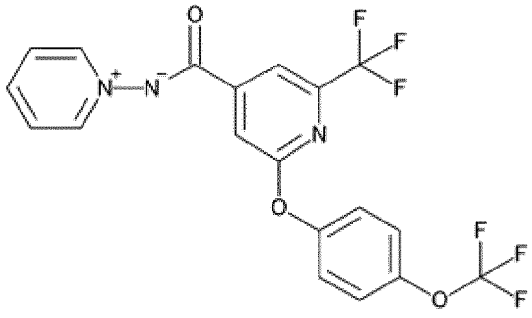 | AMORPHOUS<br>LCMS retention time 1.52 min. |
| 85 | 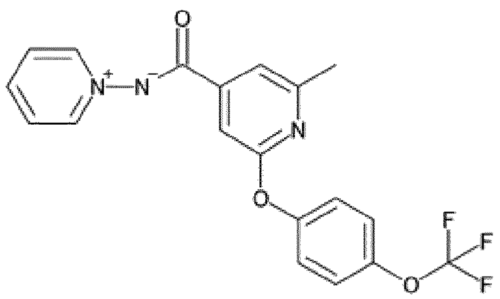 | m. p. 130-132 |
| 86 | 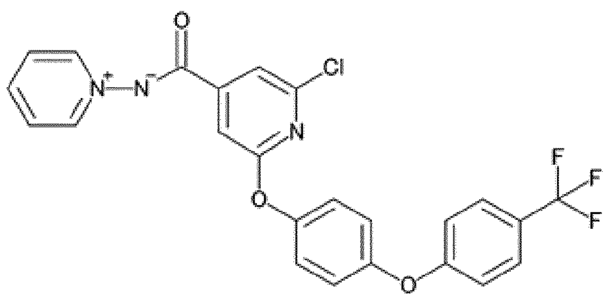 | m. p. 70-72 |

[Table 23]

Table 1 (continued)

| No. | Chemical formula | Physical properties |
|---|---|---|
| 87 | 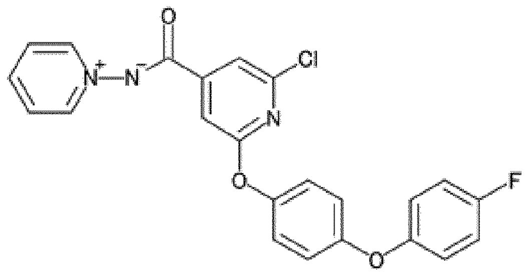 | AMORPHOUS<br>LCMS retention time 1.52 min. |
| 88 | 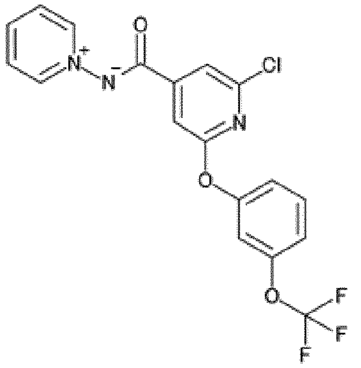 | m. p. 75-77 |
| 89 | 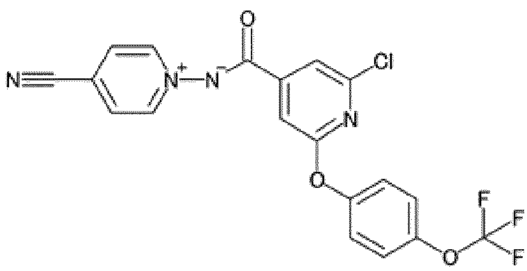 | m. p. 144-146 |
| 90 | 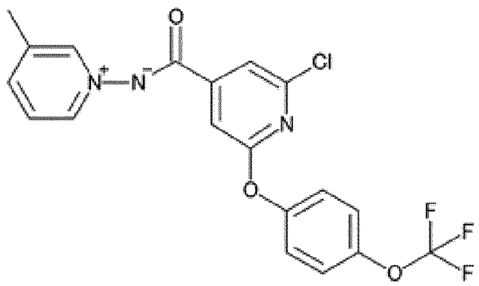 | m. p. 145-147 |

[Table 24]

Table 1 (continued)

| No. | Chemical formula | Physical properties |
|---|---|---|
| 91 | 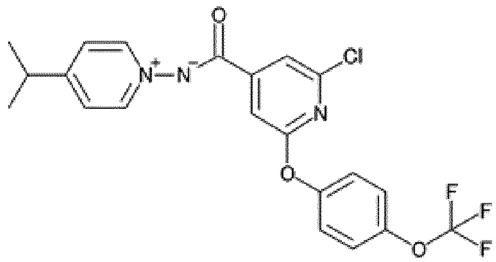 | m. p. 142-144 |
| 92 | 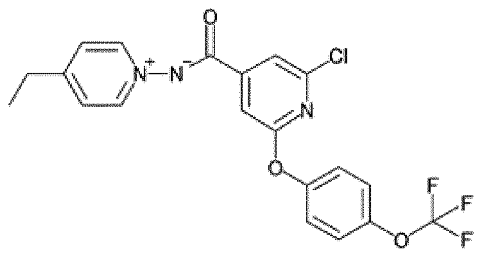 | m. p. 128-130 |
| 93 | 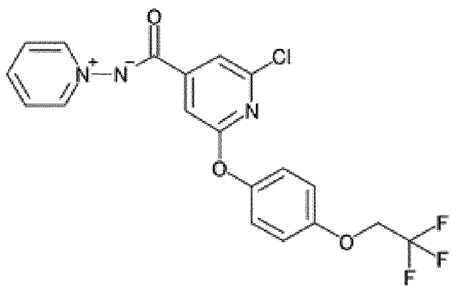 | m. p. 182-184 |
| 94 | 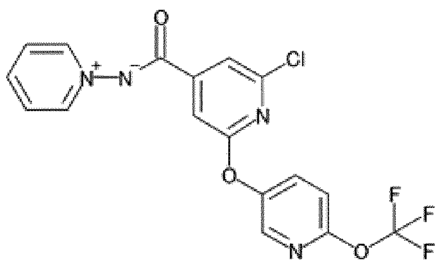 | m. p. 153-155 |

[Table 25]

Table 1 (continued)

| No. | Chemical formula | Physical properties |
|---|---|---|
| 95 | 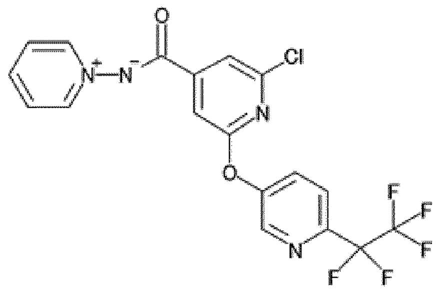 | VISCOUS OIL<br>LCMS retention time 1.39 min. |
| 96 | 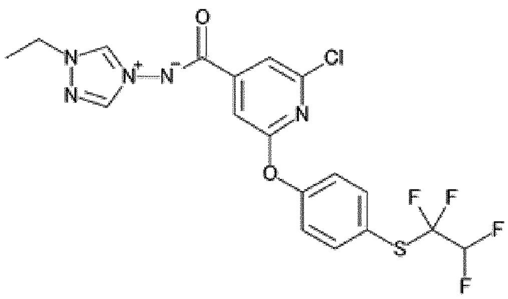 | m. p. 130-132 |
| 97 | 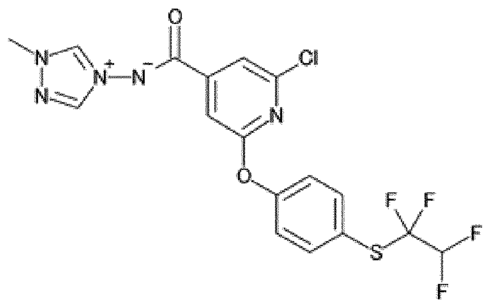 | m. p. 158-160 |
| 98 | 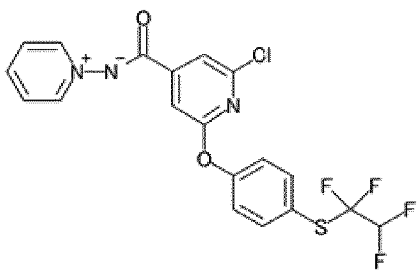 | AMORPHOUS<br>LCMS retention time 1.57 min. |

[Table 26]

Table 1 (continued)

| No. | Chemical formula | Physical properties |
|---|---|---|
| 99 | 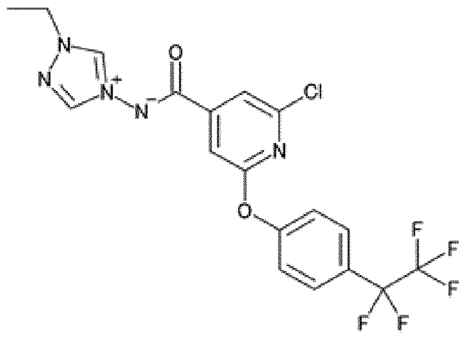 | m. p. 131-133 |
| 100 | 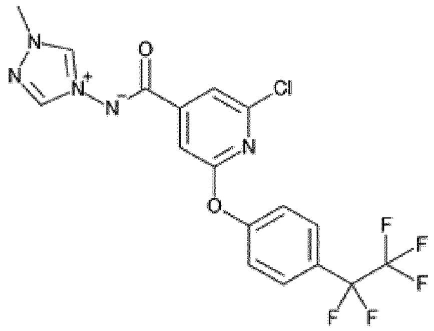 | m. p. 171-173 |
| 101 | 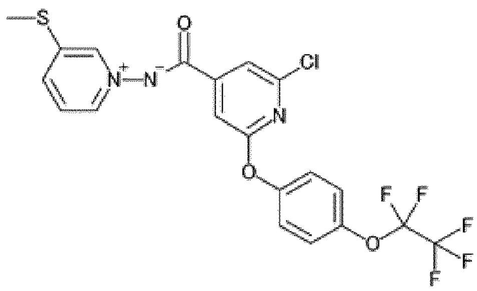 | VISCOUS OIL<br>LCMS retention time 1.75 min. |
| 102 | 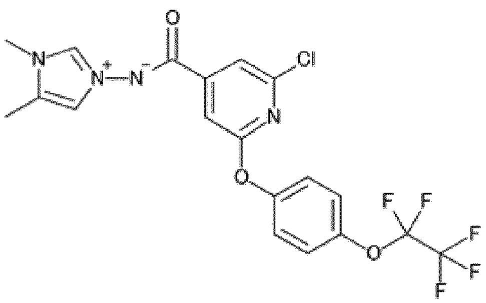 | m. p. 168-170 |

[Table 27]

Table 1 (continued)

<table>
<tr><th>No.</th><th>Chemical formula</th><th>Physical properties</th></tr>
<tr><td rowspan="2">103</td><td rowspan="2">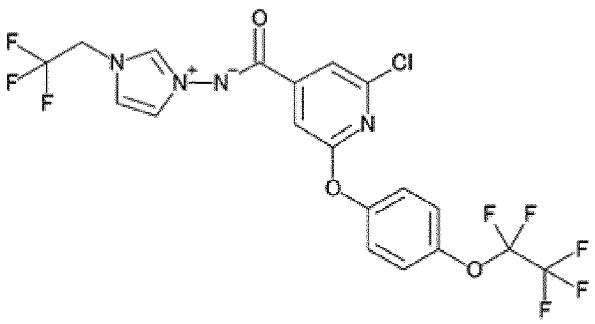</td><td>VISCOUS OIL</td></tr>
<tr><td>LCMS retention time 1.67 min.</td></tr>
<tr><td>104</td><td>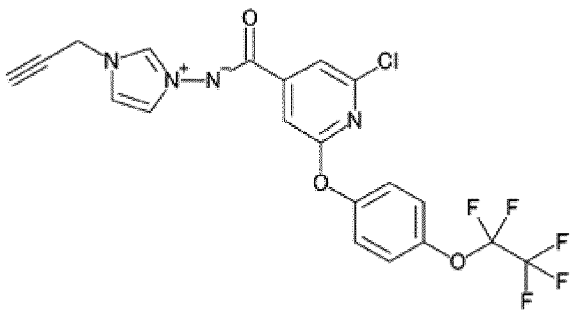</td><td>m. p. 158-160</td></tr>
<tr><td>105</td><td>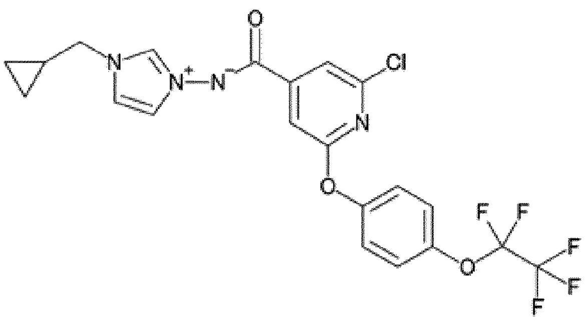</td><td>m. p. 108-110</td></tr>
<tr><td>106</td><td>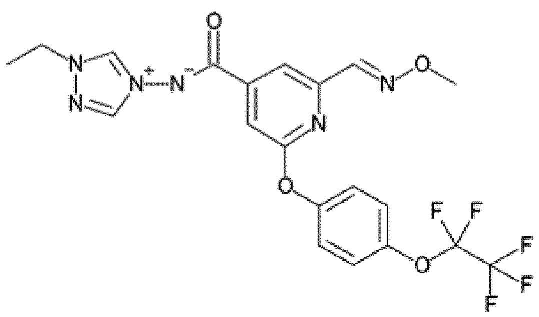</td><td>m. p. 118-120</td></tr>
</table>

[Table 28]

Table 1 (continued)

| No. | Chemical formula | Physical properties |
|---|---|---|
| 107 | 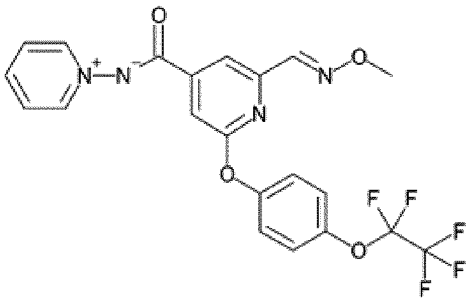 | m.p. 144-146 |
| 108 | 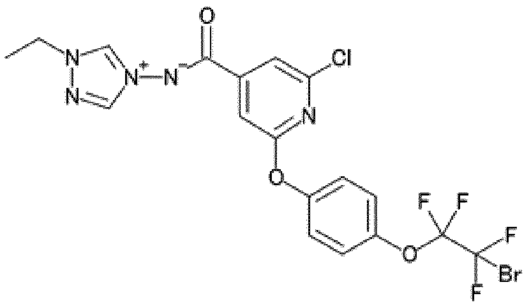 | VISCOUS OIL<br>LCMS retention time 1.74 min. |
| 109 | 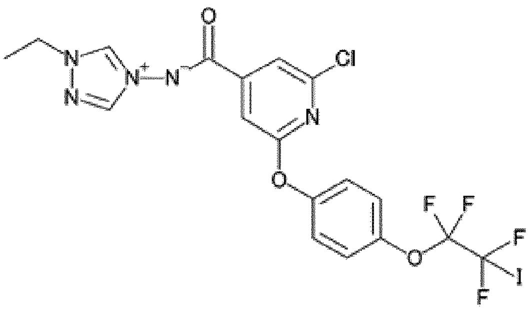 | VISCOUS OIL<br>LCMS retention time 1.74 min. |
| 110 | 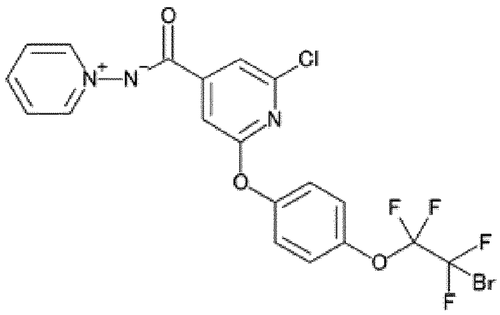 | m.p. 138-140 |

[Table 29]

Table 1 (continued)

| No. | Chemical formula | Physical properties |
|---|---|---|
| 111 | 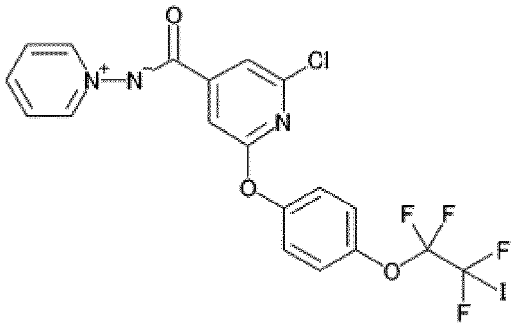 | m. p. 133-135 |
| 112 | 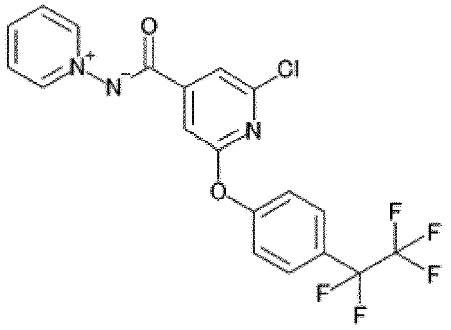 | VISCOUS OIL<br>LCMS retention time 1.66 min. |
| 113 | 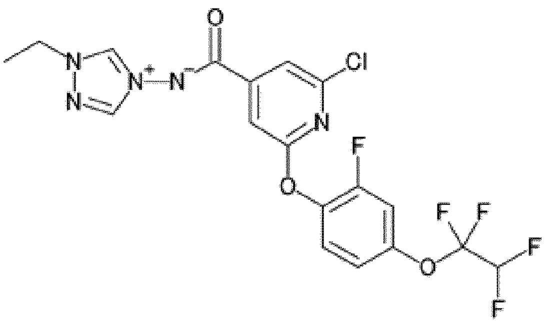 | m. p. 136-138 |
| 114 | 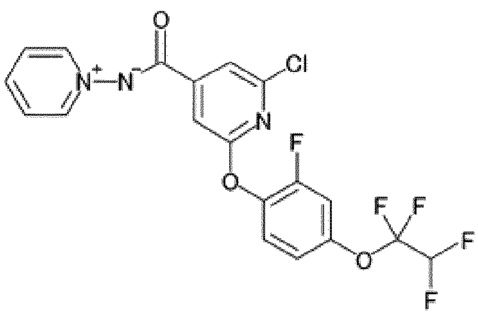 | m. p. 160-162 |

[Table 30]

Table 1 (continued)

| No. | Chemical formula | Physical properties |
|---|---|---|
| 115 | 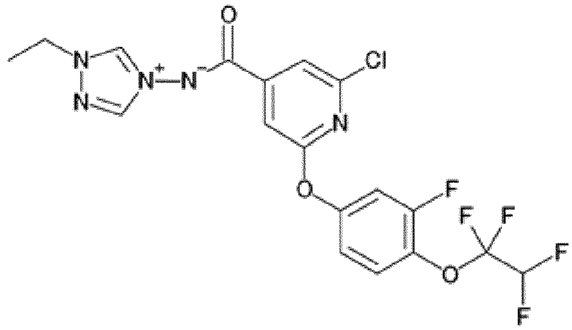 | m.p. 66-68 |
| 116 | 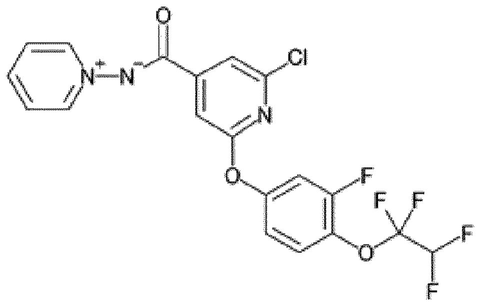 | m.p. 124-126 |
| 117 | 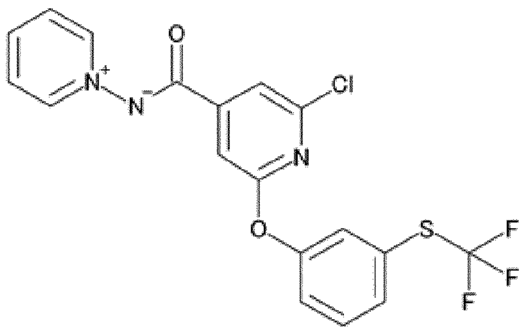 | VISCOUS OIL<br>LCMS retention time 1.48 min. |
| 118 | 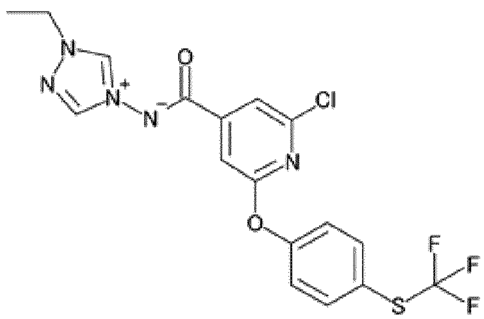 | m.p. 60-63 |

[Table 31]

Table 1 (continued)

| No. | Chemical formula | Physical properties |
|---|---|---|
| 119 | 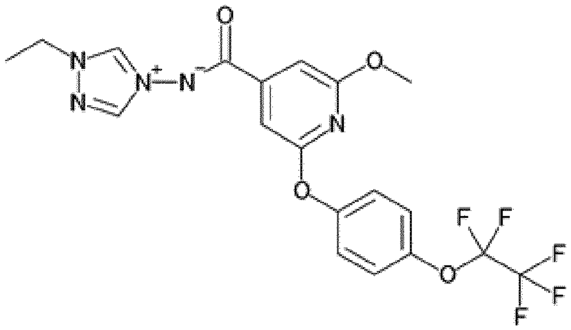 | m. p. 136-138 |
| 120 | 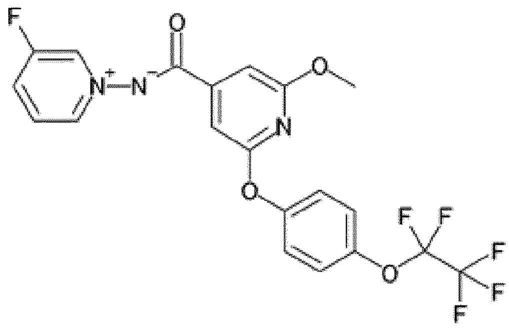 | m. p. 146-148 |
| 121 | 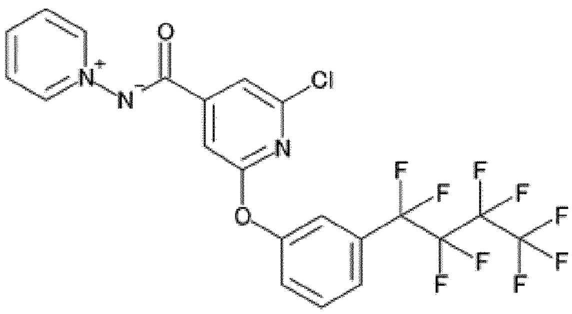 | VISCOUS OIL<br>LCMS retention time 1.81 min. |
| 122 | 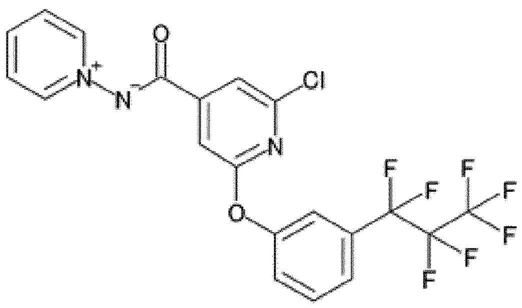 | VISCOUS OIL<br>LCMS retention time 1.70 min. |

[Table 32]

Table 1 (continued)

| No. | Chemical formula | Physical properties |
|---|---|---|
| 123 | 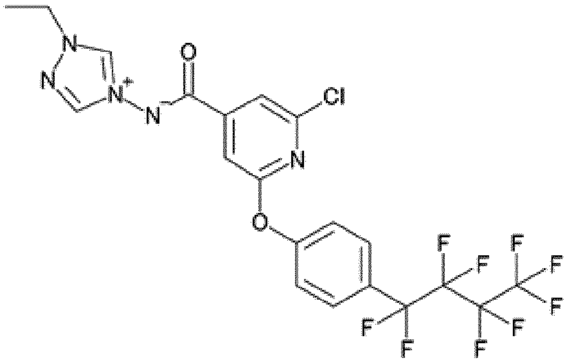 | m. p. 103-104 |
| 124 | 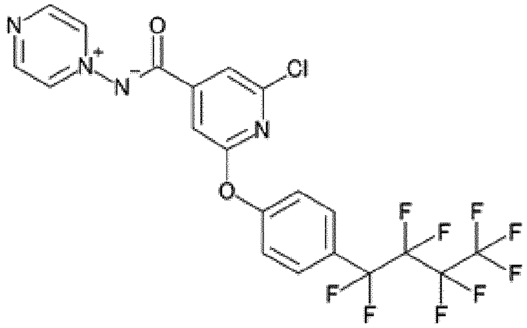 | VISCOUS OIL<br>LCMS retention time 1.92 min. |
| 125 | 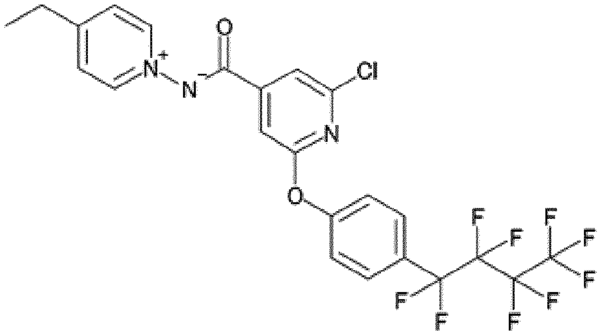 | VISCOUS OIL<br>LCMS retention time 1.93 min. |
| 126 | 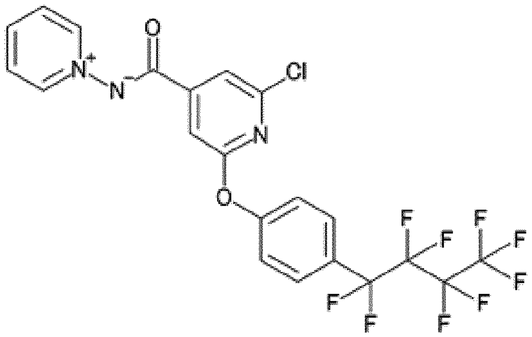 | VISCOUS OIL<br>LCMS retention time 1.86 min. |

[Table 33]

Table 1 (continued)

| No. | Chemical formula | Physical properties |
|---|---|---|
| 127 | 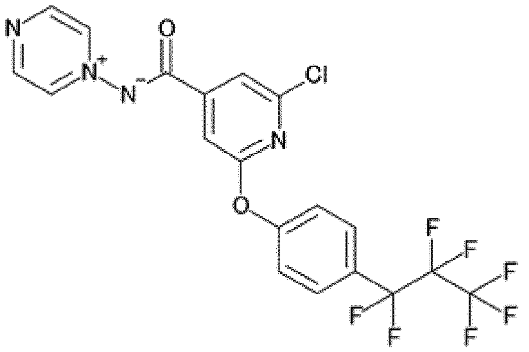 | VISCOUS OIL<br>LCMS retention time 1.81 min. |
| 128 | 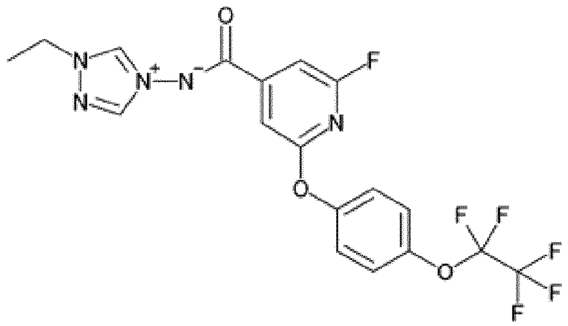 | m. p. 160-162 |
| 129 | 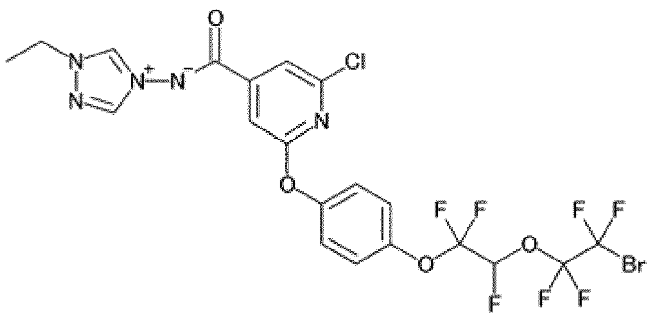 | m. p. 83-85 |
| 130 | 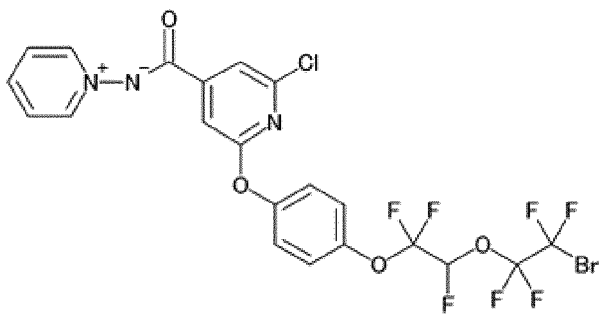 | m. p. 116-118 |

[Table 34]

Table 1 (continued)

| No. | Chemical formula | Physical properties |
|---|---|---|
| 131 | 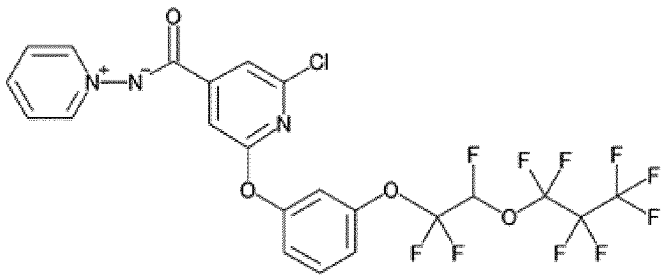 | VISCOUS OIL<br>LCMS retention time 1.84 min. |
| 132 | 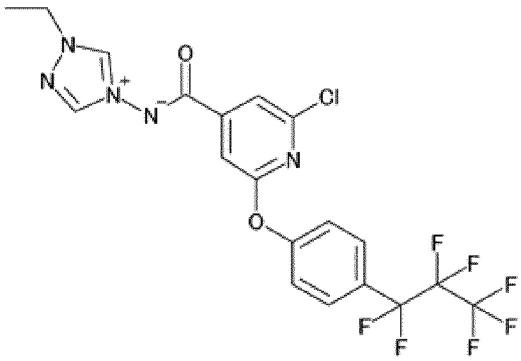 | m. p. 107-110 |
| 133 | 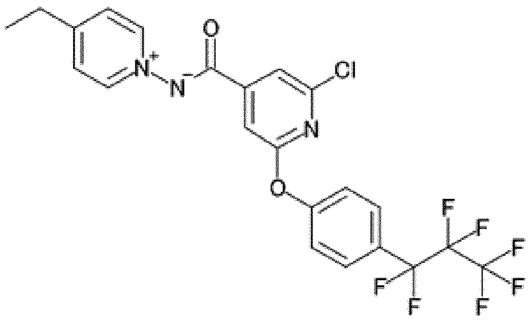 | VISCOUS OIL<br>LCMS retention time 1.83 min. |
| 134 | 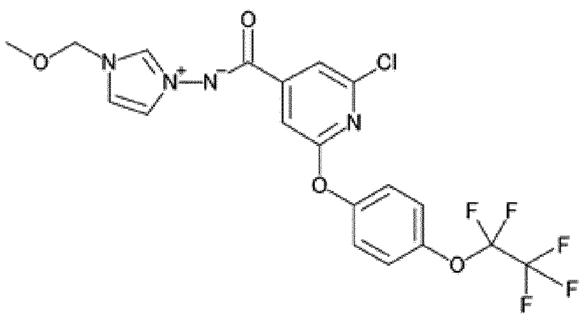 | m. p. 141-143 |

[Table 35]

Table 1 (continued)

| No. | Chemical formula | Physical properties |
|---|---|---|
| 135 | 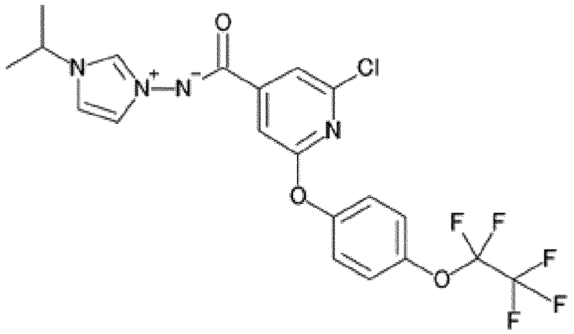 | VISCOUS OIL<br>LCMS retention time 1.64 min. |
| 136 | 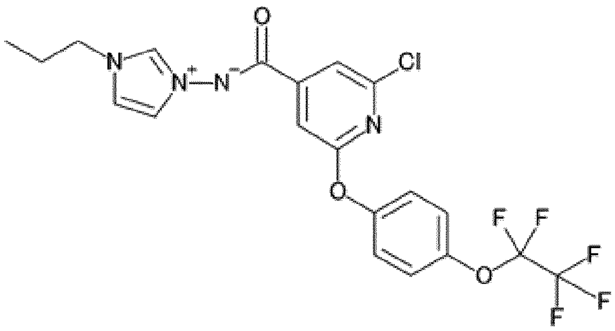 | VISCOUS OIL<br>LCMS retention time 1.67 min. |
| 137 | 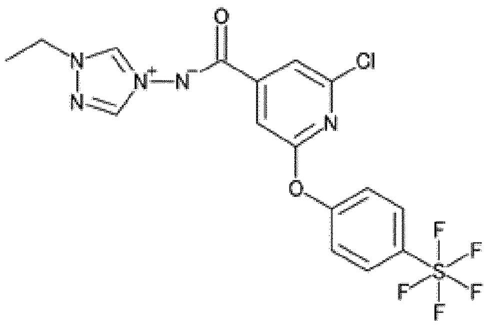 | m.p. 90-92 |
| 138 | 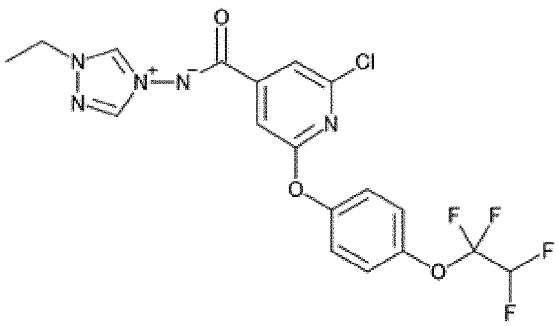 | m.p. 120-122 |

[Table 36]

Table 1 (continued)

| No. | Chemical formula | Physical properties |
|---|---|---|
| 139 | 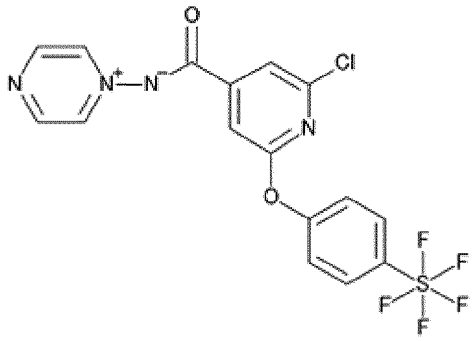 | m. p. 117-119 |
| 140 | 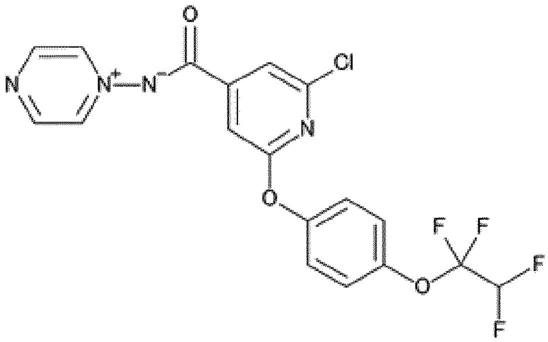 | m. p. 130-132 |
| 141 | 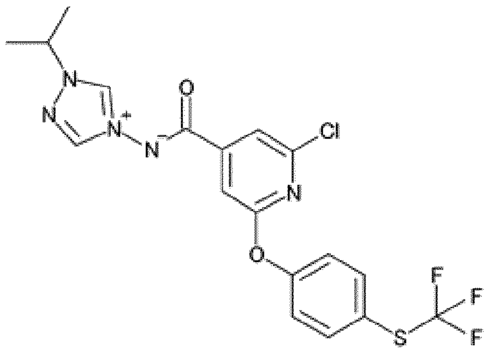 | m. p. 101-104 |
| 142 | 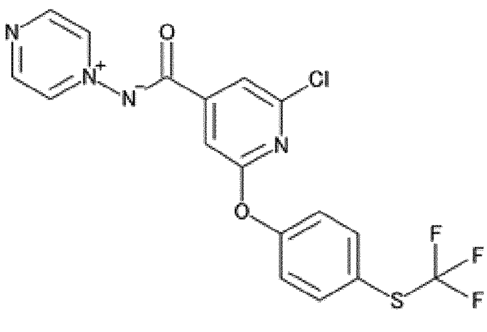 | VISCOUS OIL<br>LCMS retention time 1.67 min. |

[Table 37]

Table 1 (continued)

| No. | Chemical formula | Physical properties |
|---|---|---|
| 143 | 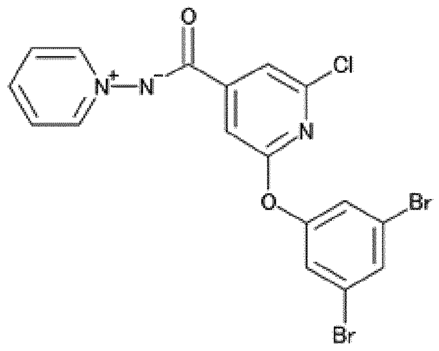 | m. p. 190-192 |
| 144 | 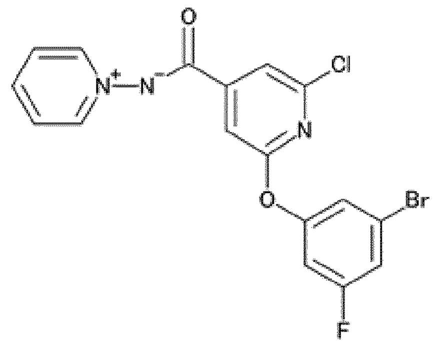 | m. p. 165-167 |
| 145 | 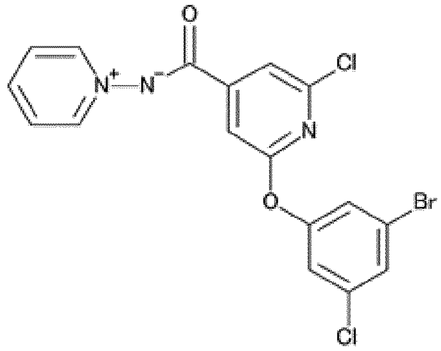 | m. p. 197-199 |
| 146 | 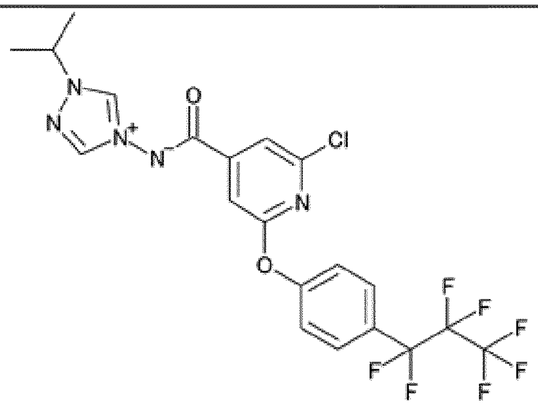 | VISCOUS OIL<br>LCMS retention time 1.80 min. |

[Table 38]

Table 1 (continued)

| No. | Chemical formula | Physical properties |
|---|---|---|
| 147 | 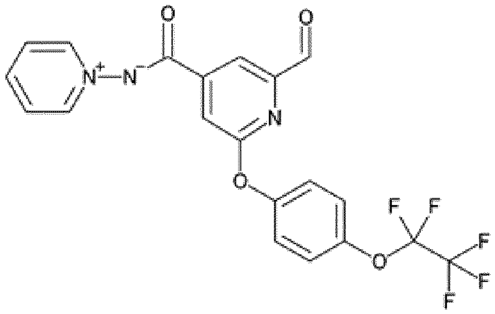 | m. p. 163-165 |
| 148 | 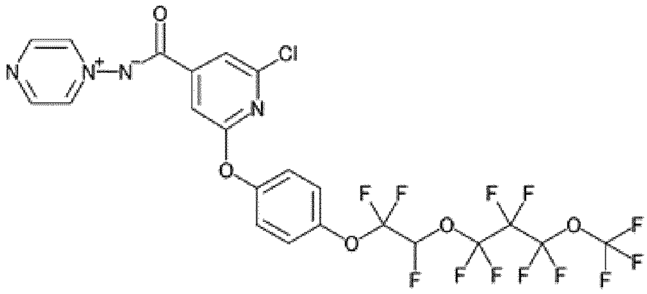 | AMORPHOUS<br>LCMS retention time 1.96 min. |
| 149 | 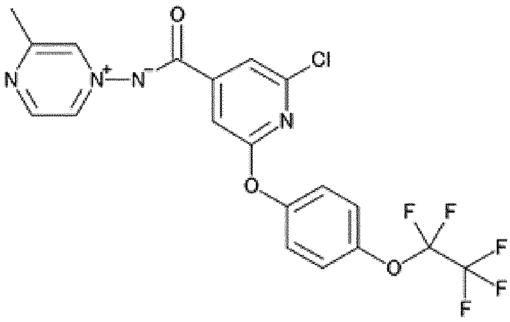 | m. p. 120-122 |
| 150 | 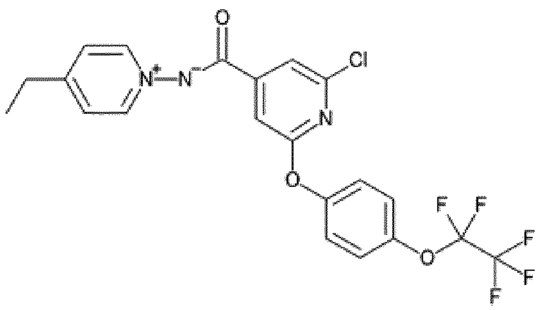 | m. p. 130-132 |

[Table 39]

Table 1 (continued)

| No. | Chemical formula | Physical properties |
|---|---|---|
| 151 | 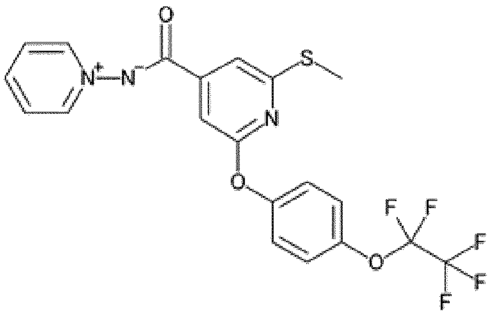 | m. p. 115-117 |
| 152 | 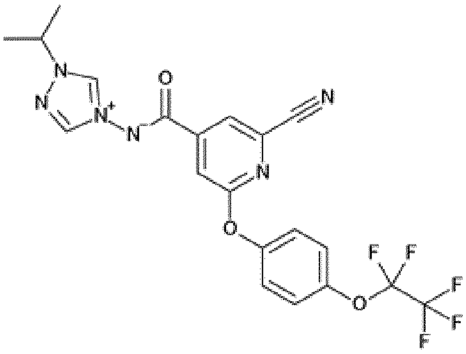 | m. p. 176-178 |
| 153 | 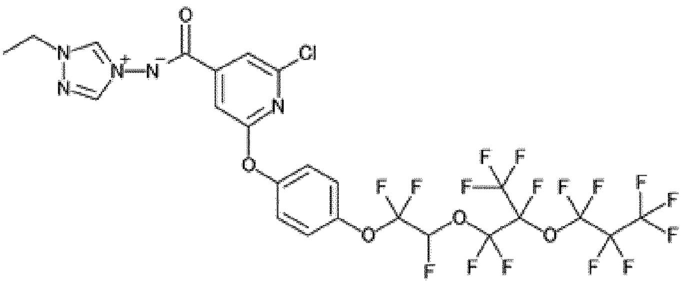 | m. p. 104-106 |
| 154 | 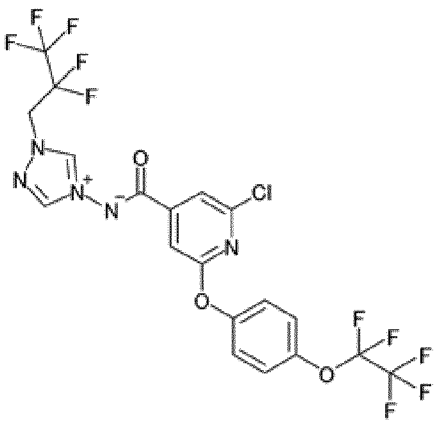 | m. p. 160-162 |

[Table 40]

Table 1 (continued)

| No. | Chemical formula | Physical properties |
|---|---|---|
| 155 | 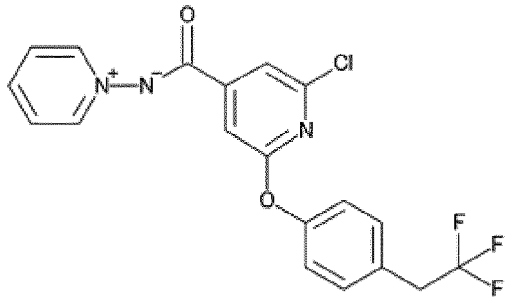 | m. p. 146-148 |
| 156 | 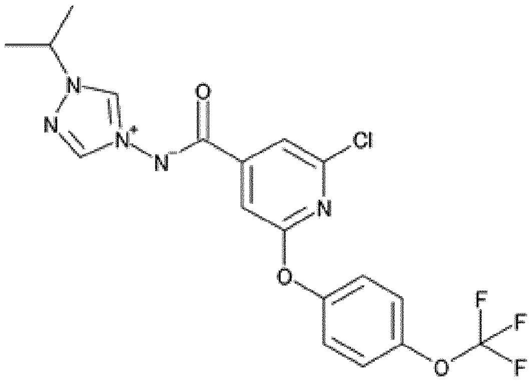 | m. p. 122-124 |
| 157 | 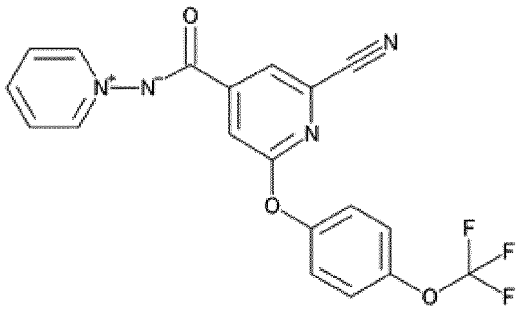 | m. p. 85-87 |
| 158 | 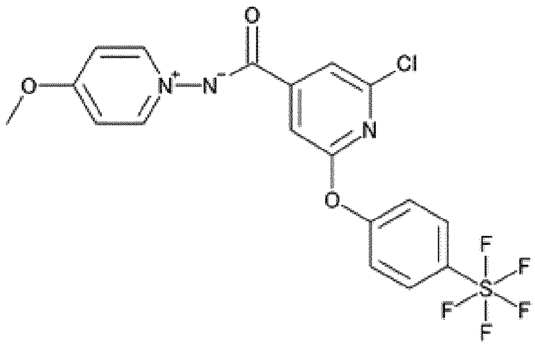 | m. p. 174-176 |

[Table 41]

Table 1 (continued)

| No. | Chemical formula | Physical properties |
|---|---|---|
| 159 | 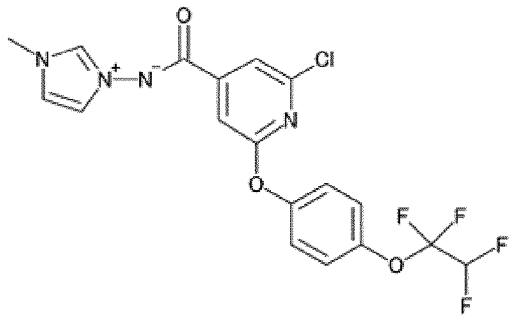 | m. p. 173-175 |
| 160 | 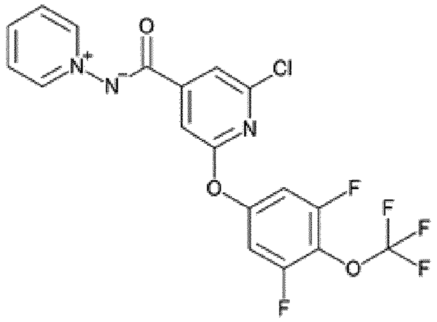 | m. p. 147-150 |
| 161 | 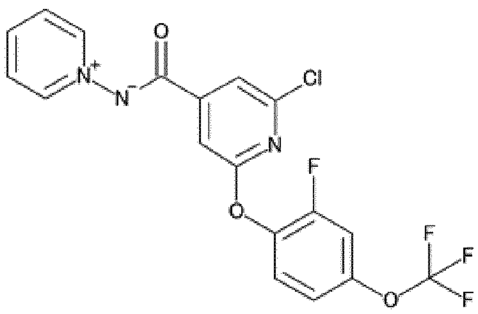 | m. p. 118-120 |
| 162 | 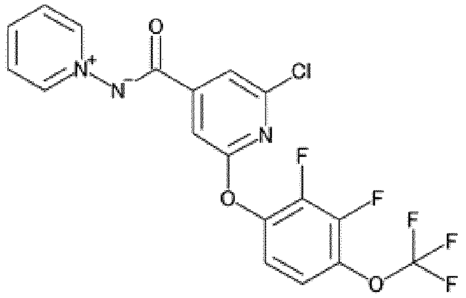 | m. p. 156-159 |

[Table42]

Table 1 (continued)

| No. | Chemical formula | Physical properties |
|---|---|---|
| 163 | 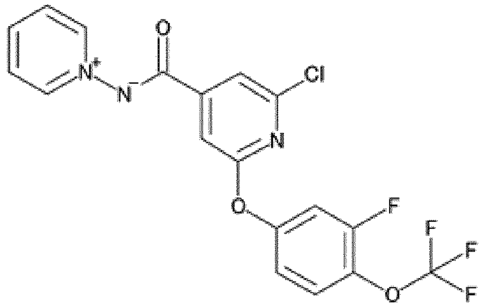 | m. p. 110-112 |
| 164 | 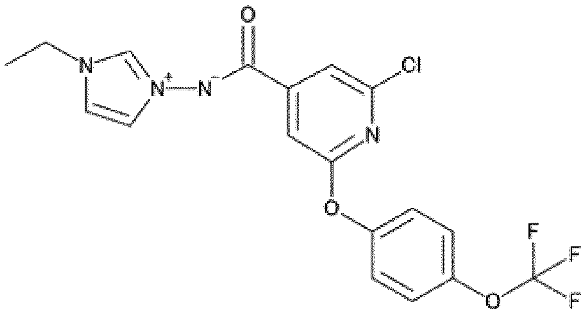 | m. p. 162-164 |
| 165 | 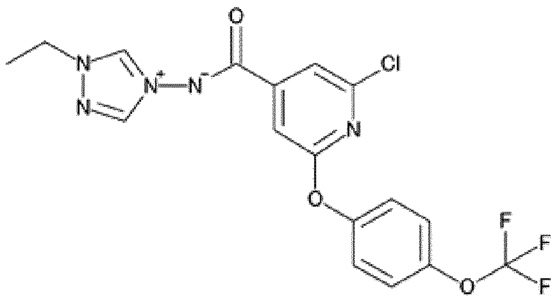 | m. p. 150-152 |
| 166 | 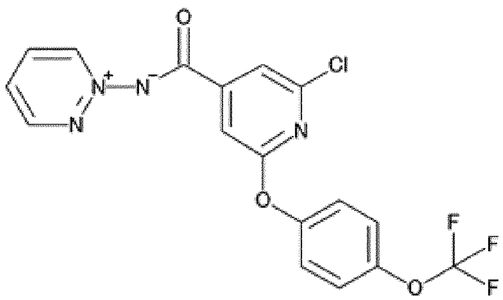 | m. p. 150-152 |

[Table 43]

Table 1 (continued)

| No. | Chemical formula | Physical properties |
|---|---|---|
| 167 | 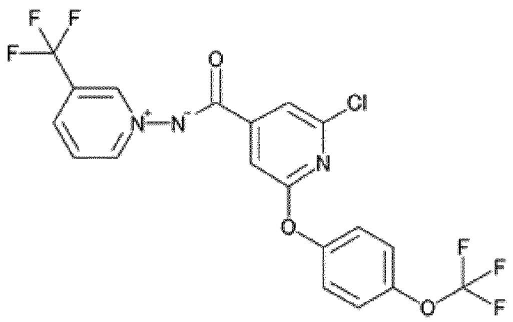 | m. p. 165-167 |
| 168 | 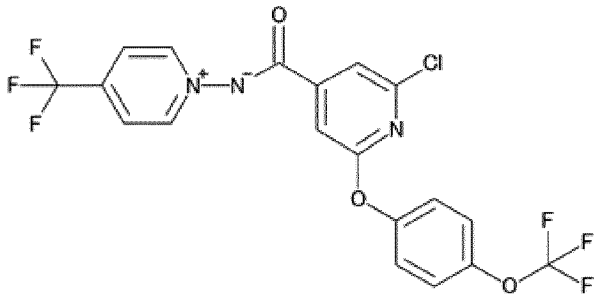 | m. p. 145-147 |
| 169 | 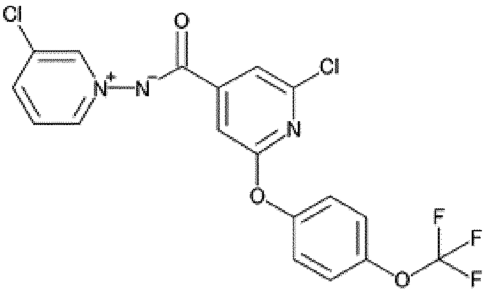 | m. p. 138-140 |
| 170 | 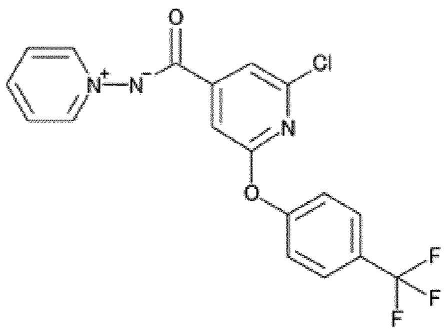 | m. p. 162-164 |

[Table 44]

Table 1 (continued)

| No. | Chemical formula | Physical properties |
|---|---|---|
| 171 | 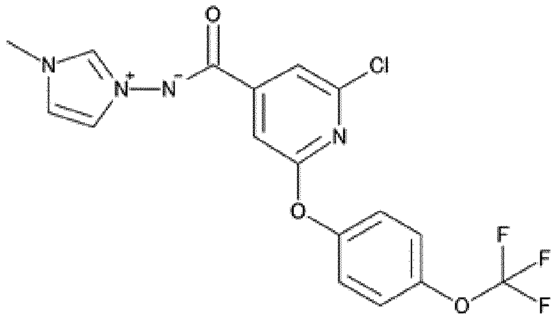 | m. p. 114-116 |
| 172 | 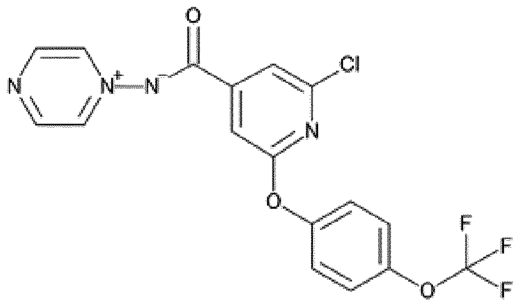 | m. p. 156-158 |
| 173 | 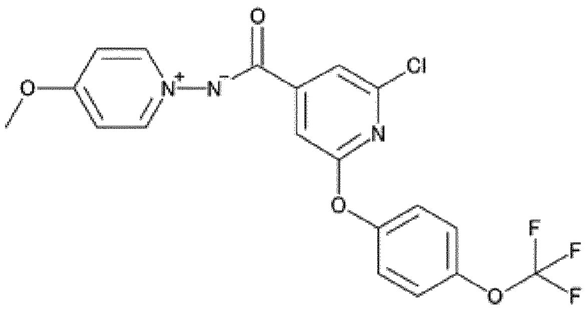 | m. p. 139-141 |
| 174 | 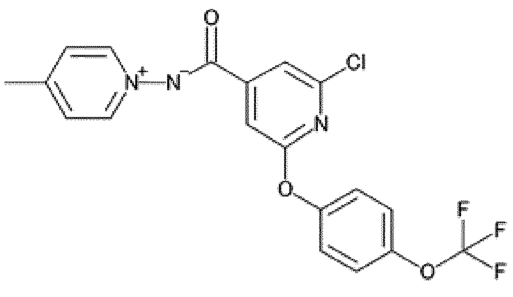 | m. p. 153-155 |

[Table 45]

Table 1 (continued)

| No. | Chemical formula | Physical properties |
|---|---|---|
| 175 | 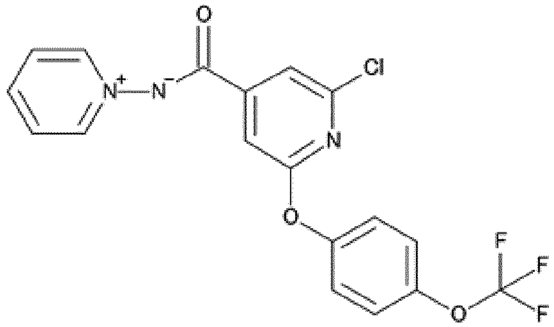 | m. p. 156-158 |
| 176 | 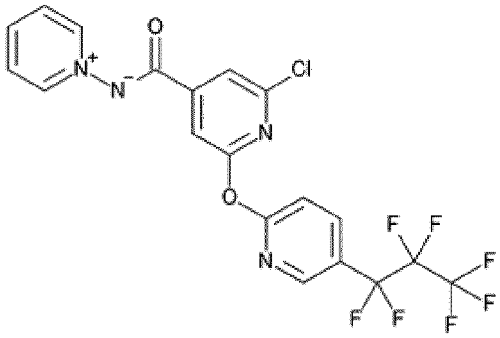 | m. p. 150-152 |
| 177 | 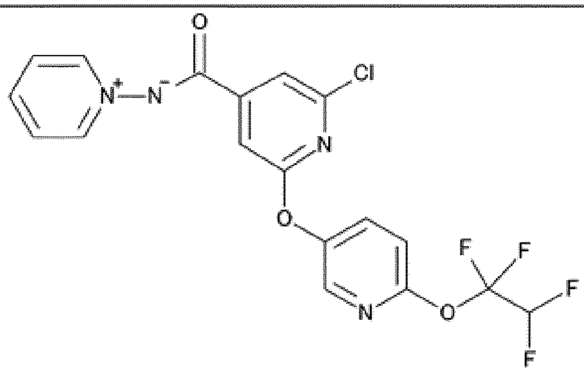 | m. p. 158-160 |
| 178 | 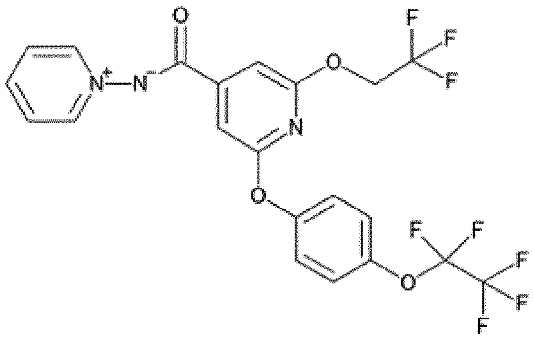 | m. p. 112-114 |

[Table 46]

Table 1 (continued)

| No. | Chemical formula | Physical properties |
|---|---|---|
| 179 | 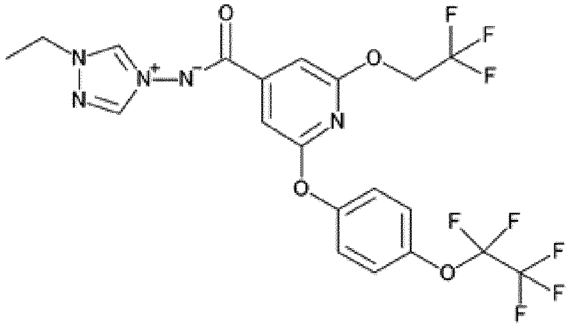 | m. p. 130-132 |
| 180 | 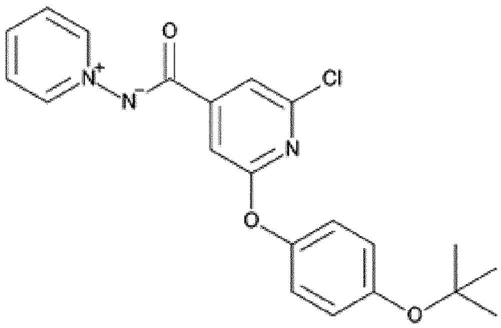 | m. p. 160-162 |
| 181 | 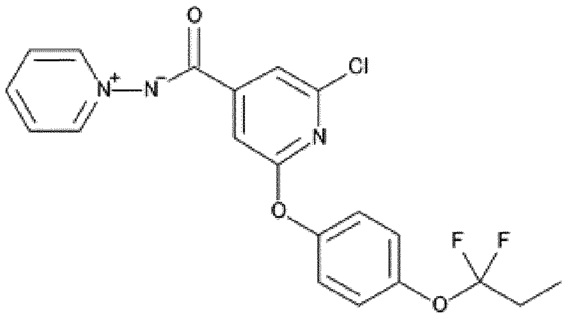 | m. p. 98-100 |
| 182 | 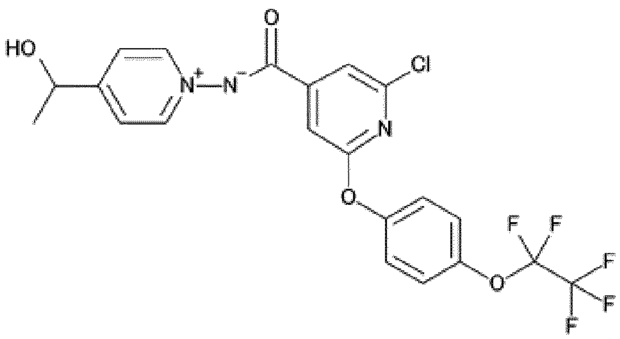 | m. p. 124-126 |

[Table 47]

Table 1 (continued)

| No. | Chemical formula | Physical properties |
|---|---|---|
| 183 | 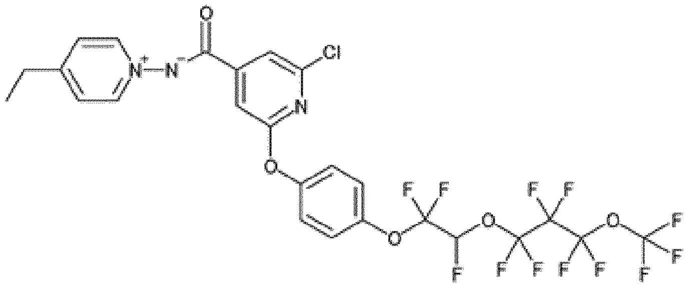 | AMORPHOUS<br>LCMS retention time 1.97 min. |
| 184 | 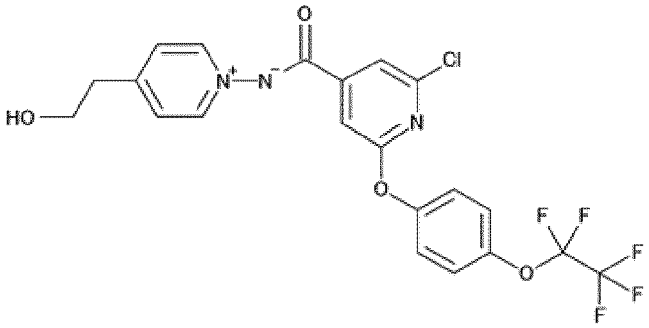 | m. p. 172-174 |
| 185 | 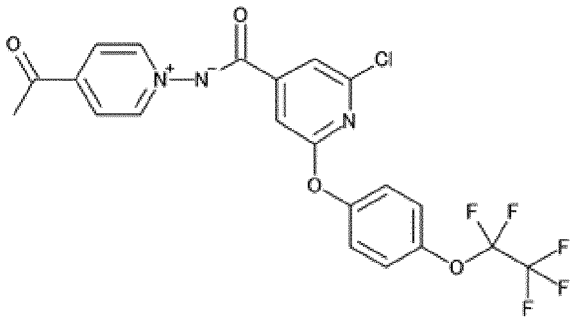 | m. p. 132-134 |
| 186 | 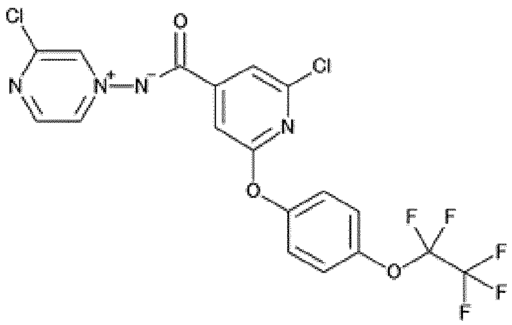 | VISCOUS OIL<br>LCMS retention time 1.89 min. |

[Table 48]

Table 1 (continued)

| No. | Chemical formula | Physical properties |
|---|---|---|
| 187 | 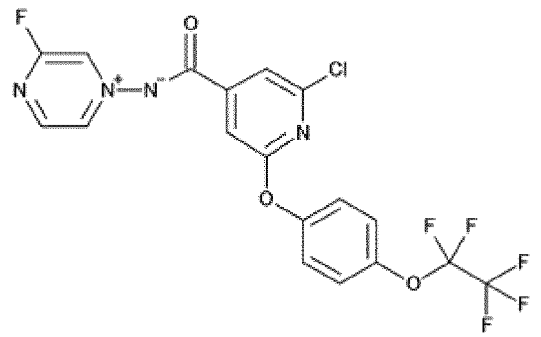 | m. p. 97-99 |
| 188 | 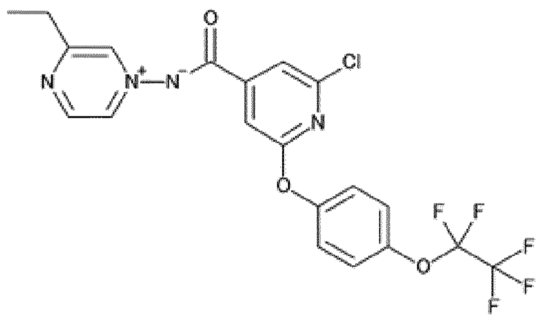 | m. p. 110-112 |
| 189 | 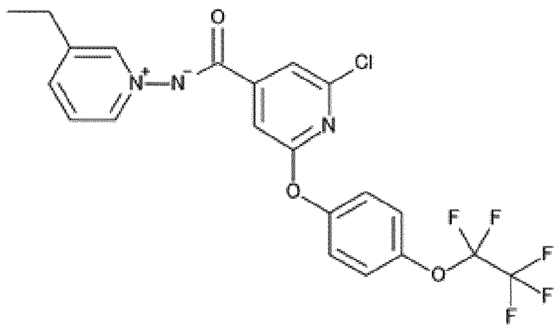 | m. p. 90-92 |
| 190 | 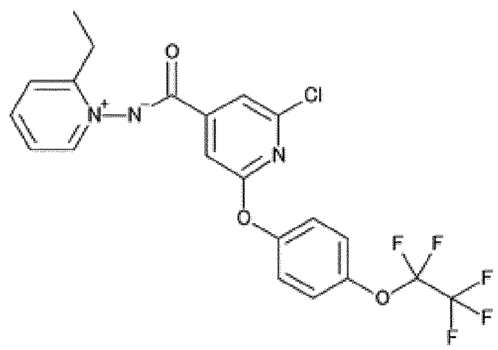 | m. p. 124-126 |

[Table 49]

Table 1 (continued)

| No. | Chemical formula | Physical properties |
|---|---|---|
| 191 | 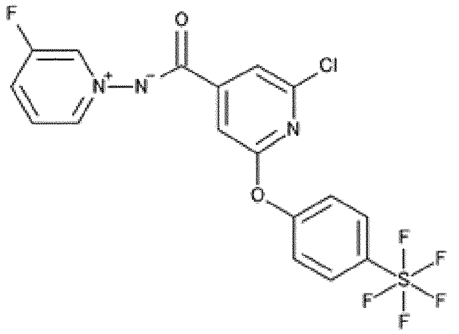 | m. p. 159-161 |
| 192 | 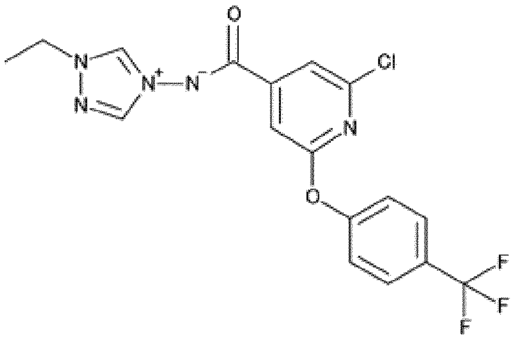 | m. p. 131-133 |
| 193 | 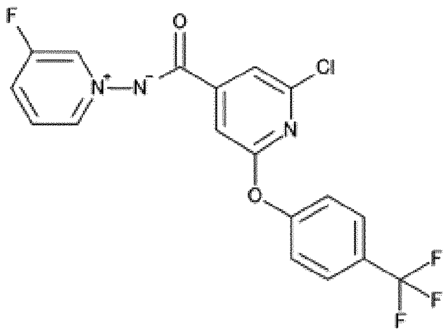 | m. p. 192-194 |
| 194 | 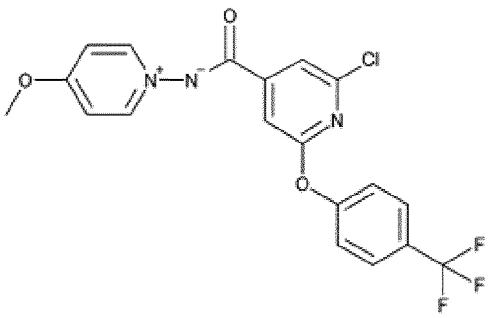 | m. p. 144-146 |

[Table 50]

Table 1 (continued)

| No. | Chemical formula | Physical properties |
|---|---|---|
| 195 | 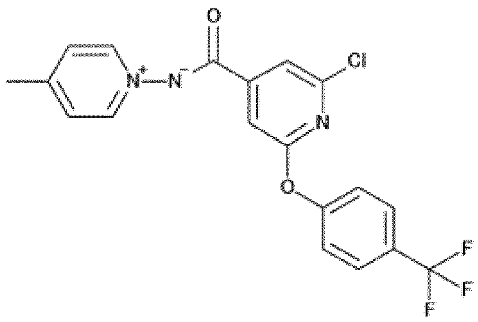 | m. p. 153-155 |
| 196 | 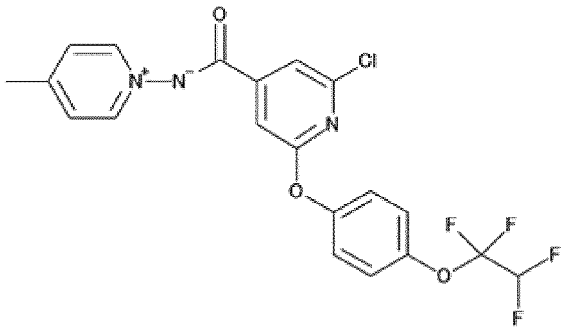 | m. p. 152-154 |
| 197 | 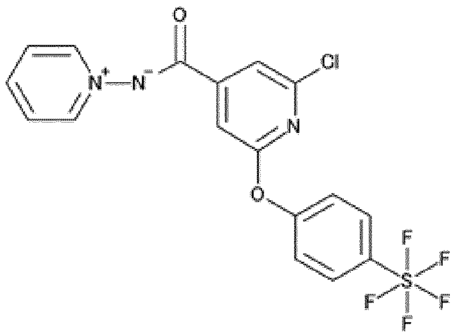 | m. p. 146-148 |
| 198 | 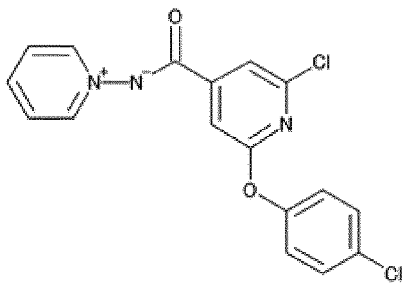 | m. p. 158-160 |

[Table 51]

Table 1 (continued)

| No. | Chemical formula | Physical properties |
|---|---|---|
| 199 | 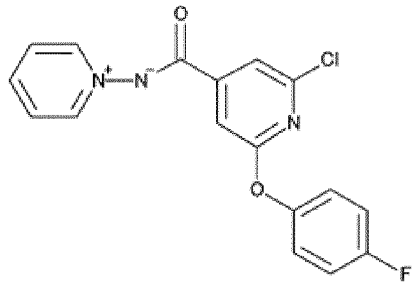 | m. p. 170-172 |
| 200 | 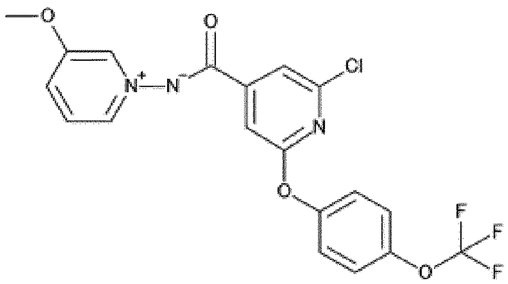 | m. p. 110-112 |
| 201 | 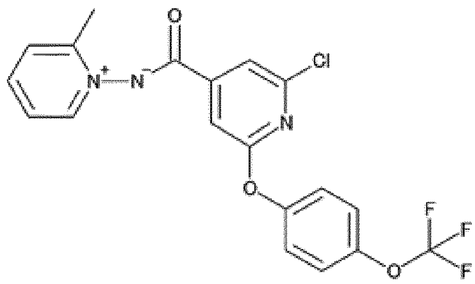 | m. p. 105-107 |
| 202 | 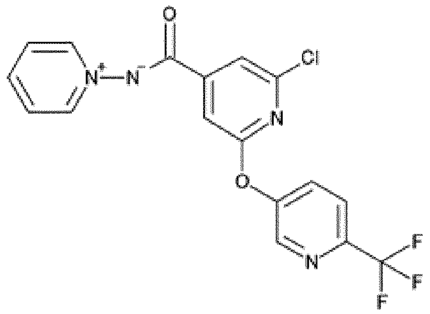 | m. p. 186-188 |

[Table 52]

Table 1 (continued)

| No. | Chemical formula | Physical properties |
|---|---|---|
| 203 | 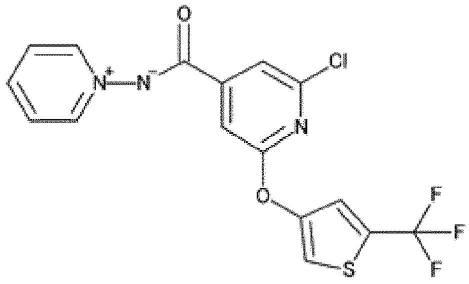 | m. p. 170-172 |
| 204 | 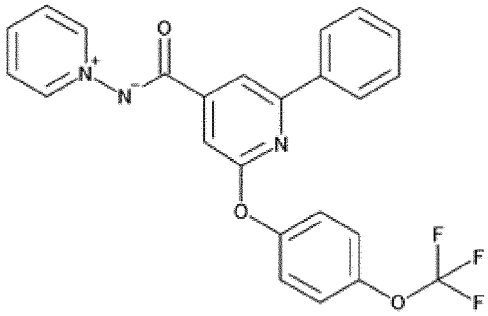 | m. p. 170-173 |
| 205 | 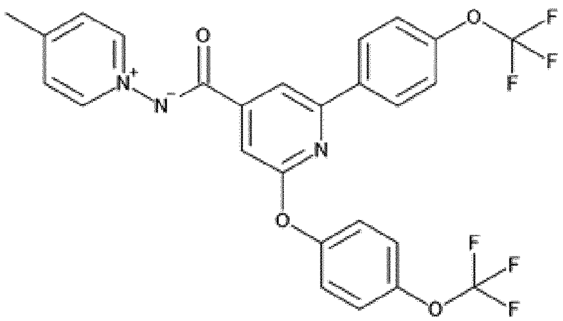 | m. p. 115-116 |
| 206 | 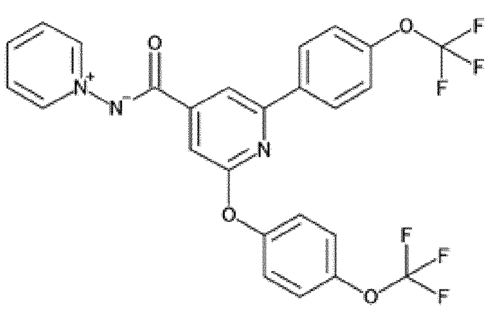 | m. p. 85-87 |

[Table 53]

Table 1 (continued)

| No. | Chemical formula | Physical properties |
|---|---|---|
| 207 | 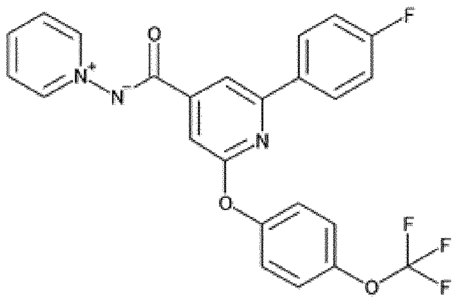 | m. p. 132-133 |
| 208 | 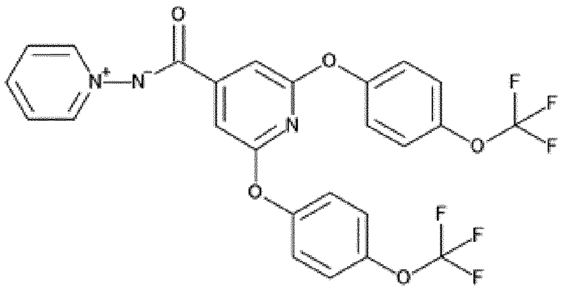 | m. p. 164-166 |
| 209 | 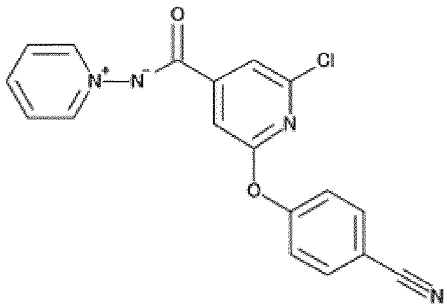 | m. p. 220-222 |
| 210 | 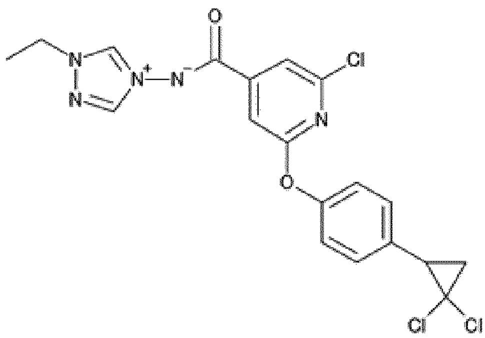 | VISCOUS OIL<br>LCMS retention time 1.63 min. |

[Table 54]

Table 1 (continued)

| No. | Chemical formula | Physical properties |
|---|---|---|
| 211 | 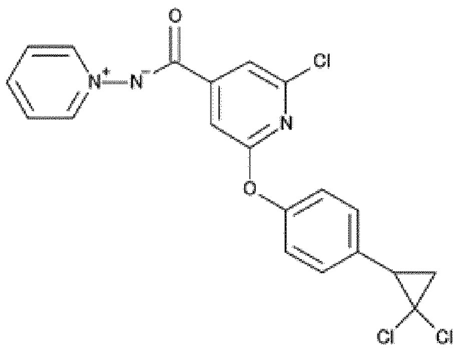 | VISCOUS OIL<br>LCMS retention time: 1.61 min. |
| 212 | 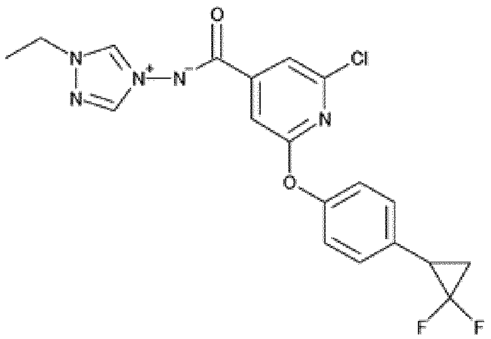 | VISCOUS OIL<br>LCMS retention time: 1.47 min. |
| 213 | 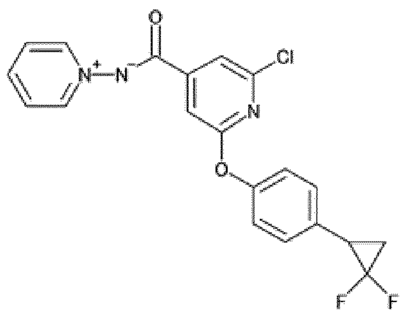 | m. p. 140-142 |
| 214 | 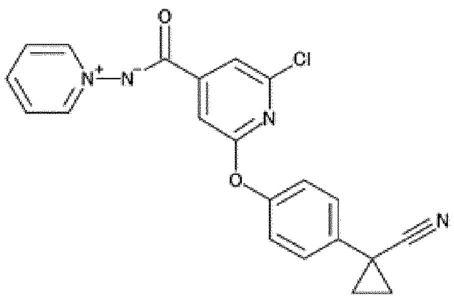 | m. p. 153-155 |

[Table 55]

Table 1 (continued)

| No. | Chemical formula | Physical properties |
|---|---|---|
| 215 | 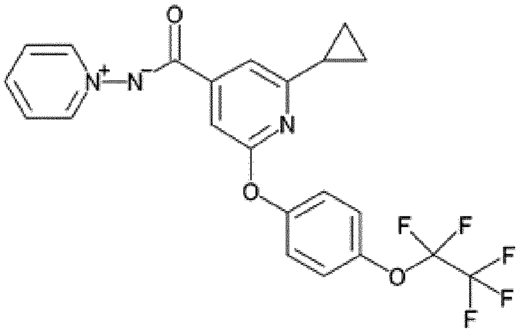 | m.p. 117-119 |
| 216 | 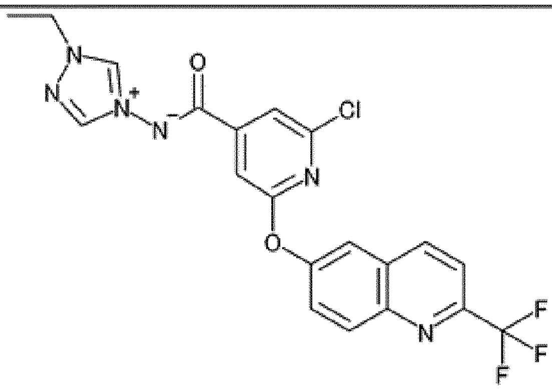 | m.p. 177-179 |
| 217 | 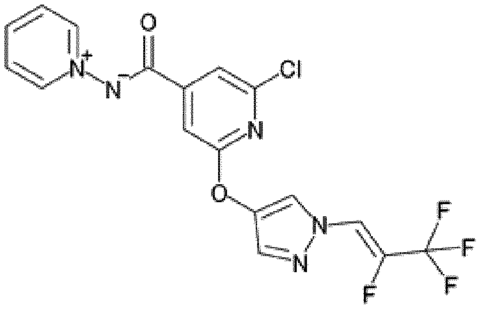 | m.p. 167-169 |
| 218 | 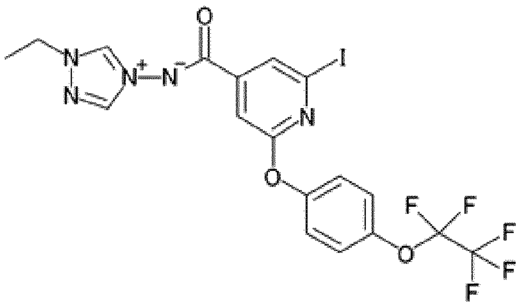 | m.p. 85-87 |

[Table 56]

Table 1 (continued)

| No. | Chemical formula | Physical properties |
|---|---|---|
| 219 | 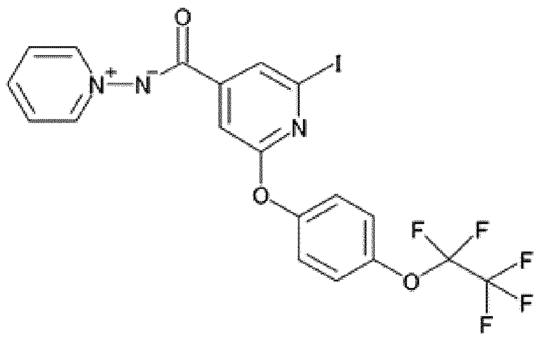 | m. p. 118-120 |
| 220 | 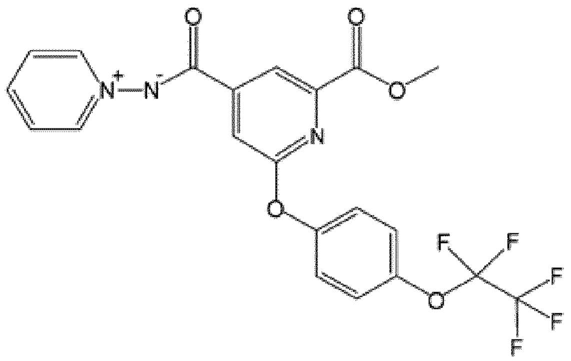 | m. p. 180-182 |
| 221 | 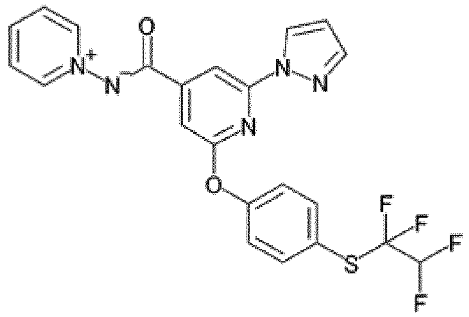 | m. p. 197-198 |

(Biological test)

The following test examples show that the compound of the present invention is useful as an active ingredient of a pest control agent. The term "part" is based on the mass.

(Preparation of test emulsion)

5 parts by mass of a compound of the present invention, 93.6 parts by mass of dimethylformamide, and 1.4 parts by mass of polyoxyethylene alkyl aryl ether were mixed and dissolved to obtain an emulsion (I) containing 5% by mass of the active ingredient.

(Preparation of control emulsion)

93.6 parts by mass of dimethylformamide, and 1.4 parts by mass of polyoxyethylene alkyl aryl ether were mixed and dissolved to obtain an emulsion (II).

(1) Efficacy Test against Mythimna separata

0.8 g of commercially-available artificial feed (Insect LFS, manufactured by Nosan Corporation) and 1 μl of the emulsion (I) were mixed well, and then 0.2 g of the mixture per treated area was packed into plastic test containers (each having a capacity of 1.4 ml) as test feeds.

Two second-instar larvae of Mythimna separata were left per treated area, and then the test containers were sealed with plastic covers. The test containers were placed in a thermostatic chamber at 25°C, and, after five days had passed therefrom, the mortality and the feed intake were measured. The test was repeated twice.

In addition, the test was carried out under the same conditions as described above, except that the emulsion (II) was used instead of the emulsion (I), and evaluated as a solvent control area.

The compounds of No. 1, 2, 3, 4, 37, 38, 39, 47, 48, 52, 53, 54, 55, 57, 58, 59, 63, 64, 66, 67, 68, 70, 71, 72, 73, 75, 76, 77, 78, 79, 80, 81, 82, 84, 85, 86, 88, 89, 90, 91, 92, 93, 100, 101, 102, 103, 104, 105, 106, 107, 110, 111, 113, 114, 115, 116, 119, 120, 121, 122, 123, 124, 125, 126, 127, 128, 129, 130, 131, 132, 133, 134, 135, 136, 137, 138, 139, 140, 141, 143, 144, 145, 148, 150, 151, 154, 156, 158, 159, 161, 162, 163, 164, 165, 166, 167, 168, 169, 170, 171, 172, 173, 174, 175, 177, 183, 184, 185, 186, 188, 189, 190, 191, 193, 194, 195, 196, 197, 198, 199, 200, 201, 202, 204, 205, 206, 207, 209, 210, 211, 212, 213, 215 and 216 were subjected to an efficacy test against Mythimna separata.
All of the compounds caused 100% mortality of Mythimna separata or 10% or less of the feed intake, relative to the feed intake at the solvent control area, and thus the efficacy thereof against Mythimna separata was confirmed. The mortality was calculated in accordance with the following equation.

$$\text{Mortality (\%)} = (\text{Died insect number} / \text{Tested insect number}) \times 100$$

(2) Efficacy Test against Spodoptera litura

The emulsion (I) was diluted with water such that the concentration of the compound of the present invention became 125 ppm by mass. A cabbage leaf was immersed in the diluted solution for 30 seconds. The cabbage leaf was air-dried and then put in a petri dish, followed by leaving five second-instar larvae of Spodoptera litura therein. The petri dish was placed in a thermostatic chamber at a temperature of 25°C and a humidity of 60%. After six days had passed from leaving the larvae, life or death was assessed and the mortality was calculated. The test was repeated twice.

The compounds of No. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 73, 80, 81, 96, 97, 98, 99, 100, 101, 102, 103, 104, 105, 106, 107, 108, 109, 110, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 142, 147, 148, 149, 150, 151, 152, 153, 154, 155, 156, 157, 158, 159, 160, 163, 164, 165, 167, 168, 169, 170, 171, 172, 173, 174, 175, 176, 177, 178, 192, 205, 206, 207, 208, 215, 217, 218, 219, and 221 were subjected to an efficacy test against Spodoptera litura. All of the compounds caused 80% or more mortality of Spodoptera litura.

(3) Efficacy Test against Plutella xylostella

The emulsion (I) was diluted with water such that the concentration of the compound of the present invention became 125 ppm by mass, thereby obtaining a diluted solution (I). The emulsion (II) was diluted with water at the same dilution ratio as the diluted solution (I), thereby obtaining a diluted solution (II). A cabbage leaf was immersed in each of the diluted solutions for 30 seconds. Each cabbage leaf after immersion was air-dried and then put in a petri dish, followed by leaving five second-instar larvae of Plutella xylostella therein. The petri dishes were placed in a thermostatic chamber at a temperature of 25°C and a humidity of 60%. After three days had passed from leaving the larvae, life or death was assessed and the mortality was calculated. The test was repeated twice.

The compounds of No. 1, 3, 4, 5, 6, 7, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 102, 104, 105, 107, 108, 109, 110, 111, 112, 113, 114, 115, 116, 117, 118, 119, 120, 121, 122, 123, 124, 126, 127, 128, 129, 130, 131, 132, 133, 134, 135, 136, 137, 138, 139, 140, 141, 143, 144, 145, 146, 147, 148, 149, 150, 152, 153, 154, 156, 157, 158, 160, 161, 163, 164, 165, 167, 168, 169, 170, 171, 172, 173, 174, 175, 176, 177, 178, 180, 181, 182, 183, 185, 188, 189, 190, 191, 192, 193, 194, 195, 196, 197, 200, 201, 202, 204, 205, 206, 207, 208, 215, 218, 219, 220

and 221 were subjected to an efficacy test against Plutella xylostella. All of the compounds caused 80% or more mortality. The diluted solution (II) caused 0% mortality.

(4) Efficacy Test against Phyllotreta striolata

The emulsion (I) was diluted with water such that the concentration of the compound of the present invention became 125 ppm by mass. The diluted solution was sprayed to a bok-choy seedling (at the $7^{th}$ leafe stage) planted in a 3-sun pot. After air-dried, the bok-choy seedling was placed in a plastic cup, and ten adults of Phyllotreta striolata were left therein. The plastic cup was placed in a thermostatic chamber at a temperature of 25°C and a humidity of 65%. After seven days had passed from leaving the adults, life or death was assessed and the mortality was calculated. The test was repeated twice.

The compounds of No. 1, 3, 10, 19, 20, 21, 22, 23, 24, 25, 26, 27, 28, 30, 31, 32, 33, 34, 35, 57, 59, 64, 66, 67, 79, 81, 82, 98, 99, 110, 111, 112, 113, 114, 116, 125, 126, 130, 134, 135, 136, 137, 138, 148, 149, 150, 163, 172, 173, 175, 176, 184, 185, 188, 189, 190, 197, 211, 218 and 219 were subjected to an efficacy test against Phyllotreta striolata. All of the compounds caused 80% or more mortality aginst the adults of Phyllotreta striolata.

(5) Efficacy Test against Thrips palmi

Eight adults of Thrips palmi were inoculated to each of cucumber seedlings. The emulsion (I) was diluted with water such that the concentration of the compound of the present invention became 125 ppm by mass, thereby obtaining a diluted solution (I). The emulsion (II) was diluted with water at the same dilution ratio as the diluted solution (I), thereby obtaining a diluted solution (II). Each of the diluted solution (I) and the diluted solution (II) was sprayed to each of the cucumber seedlings, and then air-dried. After seven days had passed from spraying, the number of living larvae was counted.

The compounds of No. 1, 3, 7, 8, 12, 13, 15, 16, 20, 25, 30, 31, 35, 76, 82, 95, 97, 98, 110, 111, 148, 150, 161, 163, 170, 172, 173, 175, 176, 178, 197, and 215 were subjected to an efficacy test against Thrips palmi. All of the compounds caused 80% or more control ratio. The diluted solution (II) caused 0% control ratio. The control ratio was calculated in accordance with the following equation.

$$\text{Control ratio (\%)} = [1 - (Nt)/(Nc)] \times 100$$

Nt: Living number at the treated area
Nc: Living number at the untreated area.

(6) Efficacy Test against Ctenocephalides felis

The compound of the present invention was dissolved in isopropanol to prepare a drug solution having a concentration of 16 ppm by mass. 100 μL of the drug solution was coated on the inner surface of the bottom of a glass vial container (φ 330 mm), and then isopropanol was vaporized by air-drying to form a thin film of the compound of the present invention.

In the vial container, four adults (a mixture of males and females) of cat flea (Ctenocephalides felis) were left. The lid was put on the vial container, and the vial container was placed in a thermostatic chamber at 25°C. After four days had passed from leaving the adults, life or death of Ctenocephalides felis was assessed and the mortality was calculated.

The compounds of No. 1, 7, 10, and 175 were subjected to an efficacy test against Ctenocephalides felis. All of the compounds caused 80% or more mortality aginst Ctenocephalides felis.

(7) Efficacy Test against Haemophysalis longicornis

The compound of the present invention was dissolved in isopropanol to prepare a drug solution having a concentration of 16 ppm by mass. 100 μL of the drug solution was coated on the inner surface of the bottom of a glass test tube (φ 12 mm), and then isopropanol was vaporized by air-drying to form a thin film of the compound of the present invention.

In the test tube, four nymphs of Haemophysalis longicornis were left. The lid was put on the test tube, and the test tube was placed in a thermostatic chamber at 25°C. After four days had passed from leaving the nymphs, life or death of Haemophysalis longicornis was assessed and the mortality was calculated.

The compound of No. 175 was subjected to an efficacy test against Haemophysalis longicornis. All of the compounds caused 80% or more mortality aginst Haemophysalis longicornis.

Since the compounds randomly selected from the aromacyclic quaternary ammonium compounds of the present invention exhibited the above-described effects, it is understood that the aromacyclic quaternary ammonium compound of the present invention, involving aspects of the compound that are not shown in the above examples, is a compound that exhibits pest control effects, acaricidal effects, and particularly insecticidal effects.

INDUSTRIAL APPLICABILITY

The aromacyclic quaternary ammonium compound of the present invention exhibits excellent control activity against pests that cause problems to agricultural crops or in the health field. The control agent containing the aromacyclic quaternary ammonium compound of the present invention can effectively control pests, particularly agricultural insect pests and acarians, at lower doses, and can further effectively control ectoparasites and endoparasites that may harm humans and animals.

## Claims

**1.** A compound of formula (I) or formula (II):

[Chemical Formula 1]

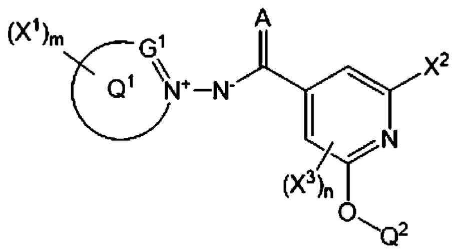

(I)

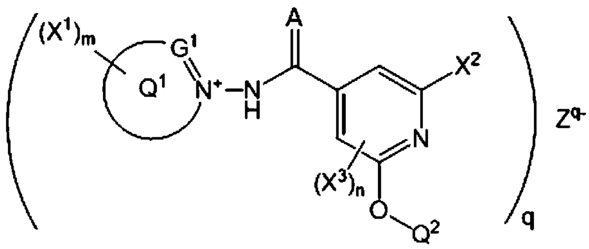

(II)

(in formulae (I) and (II),

$Q^1$ is a pyrazole ring, an imidazole ring, a 1,2,4-triazole ring, a pyridine ring, a pyrazine ring, a pyrimidine ring, or a pyridazine ring,
$G^1$ is a nitrogen atom or a carbon atom,
$X^1$ is a halogeno group, a substituted or unsubstituted C1-6 alkyl group, a substituted or unsubstituted C2-6 alkenyl group, a substituted or unsubstituted C2-6 alkynyl group, a substituted or unsubstituted C1-6 alkoxy group, a substituted or unsubstituted C1-6 alkylthio group, a substituted or unsubstituted C1-6 alkylsulfinyl group, a substituted or unsubstituted C1-6 alkylsulfonyl group, a substituted or unsubstituted C3-8 cycloalkyl group, a substituted or unsubstituted C1-6 alkylcarbonyl group, a substituted or unsubstituted C6-10 aryl group, a substituted or unsubstituted 5- or 6-membered heteroaryl group, a pentafluorosulfanyl group, a nitro group, or a cyano group,
m indicates the number of $X^1$, and m is an integer of 1 to 3 when $Q^1$ is a 1,2,4-triazole ring, is an integer of 1 to 4 when $Q^1$ is a pyrazole ring or an imidazole ring, is an integer of 0 to 4 when $Q^1$ is a pyrazine ring, a pyrimidine ring or a pyridazine ring, or is an integer of 0 to 5 when $Q^1$ is a pyridine ring,
A is an oxygen atom or a sulfur atom,
$Q^2$ is a substituted or unsubstituted phenyl group, a substituted or unsubstituted 5- or 6-membered heteroaryl group, or a substituted or unsubstituted 9- or 10-membered heteroaryl group,
$X^2$ is a halogeno group, a substituted or unsubstituted C1-6 alkyl group, a substituted or unsubstituted C2-6

alkynyl group, a substituted or unsubstituted C1-6 alkoxy group, a substituted or unsubstituted C1-6 alkoxycarbonyl group, a substituted or unsubstituted C1-6 alkylthio group, a substituted or unsubstituted C3-8 cycloalkyl group, a substituted or unsubstituted C6-10 aryl group, a substituted or unsubstituted C6-10 aryloxy group, a substituted or unsubstituted pyrazolyl group, an aldehyde group, a cyano group, or $-C=N-OCH_3$,
$X^3$ is a halogeno group, a substituted or unsubstituted C1-6 alkyl group, a substituted or unsubstituted C1-6 alkoxy group, or a cyano group,
n indicates a number of $X^3$, and is an integer of 0 to 2,
$Z^{q-}$ is a counter ion, and
q indicates a valence of the counter ion and is 1 or 2.)

**2.** A pest control agent comprising at least one selected from compounds of claim 1 as an active ingredient thereof.

**3.** An insecticide or acaricide comprising at least one selected from compounds of claim 1 as an active ingredient thereof.

**4.** An ectoparasite control agent comprising at least one selected from compounds of claim 1 as an active ingredient thereof.

**5.** An endoparasite control agent or expellant comprising at least one selected from compounds of claim 1 as an active ingredient thereof.

# INTERNATIONAL SEARCH REPORT

International application No.
**PCT/JP2023/029530**

**A. CLASSIFICATION OF SUBJECT MATTER**

***C07D 401/12***(2006.01)i; ***A01N 43/40***(2006.01)i; ***A01N 43/50***(2006.01)i; ***A01N 43/653***(2006.01)i; ***A01N 47/02***(2006.01)i; ***A01P 7/02***(2006.01)i; ***A01P 7/04***(2006.01)i; ***A61K 31/4439***(2006.01)i; ***A61K 31/444***(2006.01)i; ***A61K 31/695***(2006.01)i; ***A61P 33/00***(2006.01)i; ***C07F 7/12***(2006.01)i

FI: C07D401/12 CSP; A01N43/40 101L; A01N43/50 M; A01N43/653 M; A01N43/653 N; A01N47/02; A01P7/02; A01P7/04; A61K31/4439; A61K31/444; A61K31/695; A61P33/00; C07F7/12 T

According to International Patent Classification (IPC) or to both national classification and IPC

**B. FIELDS SEARCHED**

Minimum documentation searched (classification system followed by classification symbols)

C07D401/12; A01N43/40; A01N43/50; A01N43/653; A01N47/02; A01P7/02; A01P7/04; A61K31/4439; A61K31/444; A61K31/695; A61P33/00; C07F7/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2021/049522 A1 (NIPPON SODA CO., LTD.) 18 March 2021 (2021-03-18) entire text | 1-5 |
| A | WO 2020/241632 A1 (NIPPON SODA CO., LTD.) 03 December 2020 (2020-12-03) entire text | 1-5 |
| A | JP 8-510462 A (SHELL INTERNATIONALE RESEARCH MAATSCHAPPIJ B.V.) 05 November 1996 (1996-11-05) entire text | 1-5 |
| A | JP 60-104068 A (FBC LTD.) 08 June 1985 (1985-06-08) entire text | 1-5 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 October 2023** | **07 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/029530**

| Patent document cited in search report | Publication date (day/month/year) | Patent family member(s) | Publication date (day/month/year) |
|---|---|---|---|
| WO 2021/049522 A1 | 18 March 2021 | US 2022/0332686 A1 | |
| | | EP 4029855 A1 | |
| | | KR 10-2022-0062269 A | |
| WO 2020/241632 A1 | 03 December 2020 | US 2022/0267272 A1 | |
| | | KR 10-2022-0015388 A | |
| | | TW 202110803 A | |
| JP 8-510462 A | 05 November 1996 | US 5759956 A | |
| | | WO 1994/027974 A1 | |
| | | EP 700391 A1 | |
| JP 60-104068 A | 08 June 1985 | US 4602944 A | |
| | | EP 142248 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022131222 A
- WO 2021049522 A


### Non-patent literature cited in the description


- **COLBY. S. R.** Calculating Synergistic and Antagonistic Responses of Herbicide Combinations. *Weeds*, 1967, vol. 15, 20-22